# EUROPEAN PATENT SPECIFICATION

(11) **EP 1 558 284 B1**
(45) Date of publication and mention of the grant of the patent: **11.09.2013**
(21) Application number: 02769028.8
(22) Date of filing: 11.10.2002
(51) Int. Cl.: A61K 39/395, A61P 35/00

(54) **COMBINATION THERAPY WITH NAKED CLASS III ANTI-CEA MONOCLONAL ANTIBODIES AND THERAPEUTIC AGENTS**
KOMBINATIONSTHERAPIE MIT NAKTEN KLASSE III ANTI-CEA MONOKLONALEN ANTIKÖRPERN UND THERAPEUTISCHEN WIRKSTOFFEN
THERAPIE ASSOCIANT DES ANTICORPS NUS MONOCLONAUX ANTI-CEA DE CLASSE III ET DES AGENTS THERAPEUTIQUES

(30) Priority: 08.10.2002 US 416531 P
(43) Date of publication of application: 03.08.2005
(73) Proprietor: Immunomedics, Inc., Morris Plains, NJ 07950 (US)
(72) Inventor: GOLDENBERG, David, M., Morris Plains NJ 07950 (US); HANSEN, Hans, J., Morris Plains NJ 07950 (US)
(74) Representative: Bohmann, Armin K.
(86) International application number: PCT/US2002/032307
(87) International publication number: WO 2004/032962

(56) References cited:
- US-A- 4 970 071
- US-A- 5 874 540
- US-A- 6 096 289
- STEIN R ET AL: "COMBINING RADIOIMMUNOTHERAPY AND CHEMOTHERAPY FOR TREATMENT OF MEDULLARY THYROID CARCINOMA EFFECTIVENESS OF DACARBAZINE" CANCER, AMERICAN CANCER SOCIETY, PHILADELPHIA, PA, US, vol. 94, no. 1, 1 January 2002 (2002-01-01), pages 51-61, XP008055316 ISSN: 0008-543X
- LONBERG N ET AL: "HUMAN ANTIBODIES FROM TRANSGENIC MICE" INTERNATIONAL REVIEWS OF IMMUNOLOGY, HARWOOD ACADEMIC PUBLISHERS, LONDON, GB, vol. 13, 1 January 1995 (1995-01-01), pages 65-93, XP000944265 ISSN: 0883-0185
- STEIN R ET AL: "A humanized monoclonal antibody to carcinoembryonic antigen, labetuzumab, inhibits tumor growth and sensitizes human medullary thyroid cancer xenografts to dacarbazine chemotherapy" MOLECULAR CANCER THERAPEUTICS, AMERICAN ASSOCIATION OF CANCER RESEARCH, US, vol. 3, no. 12, 1 December 2004 (2004-12-01), pages 1559-1564, XP002996191 ISSN: 1535-7163
- 'Chemotherapy', [Online] pages 1 - 12 Retrieved from the Internet: <URL:http://en.wikipedia.org/wiki/Chemother apy> [retrieved on 2012-08-29]
- 'Cancer Treatment', [Online] pages 1 - 2 Retrieved from the Internet: <URL:http://www.knowcancer.com/cancer-treat ment/> [retrieved on 2012-08-29]

## Description

### BACKGROUND OF THE INVENTION

### A. Field of the Invention

The invention relates to a composition comprising at least one naked Class III anti-CEA monoclonal antibody (MAb) or fragment thereof, a naked Class III anti-CEA monoclonal antibody or fragment thereof for use in a method for treating CEA expressing non-medullary thyroid carcinoma, and a naked Class III anti-CEA monoclonal antibody or fragment thereof for use in a treating medullary thyroid carcinoma.

The cancers that express carcinoembryonic antigen ("CEA") comprise medullary thyroid cancer (MTC), non- medullary thyroid cancers (non-MTC), colorectal cancers, hepatocellular carcinoma, gastric cancer, lung cancer, breast cancer and other cancers The antibody is administered in combination with at least one other therapeutic agent, such as a chemotherapeutic agent, a radioactive agent, an immunomodulator, an immunoconjugate or a combination thereof. The MAb is administered prior to, with or after administering the therapeutic agent Preferably, the MAb has the binding affinity characteristics and specificities of corresponding murine Class III anti-CEA MAb. More preferably, the MAb is a humanized, chimeric or human MAbs, that possesses more of the antigenic and effector properties of a human antibody. Particularly useful MAbs are humanized MAbs in which the complementarity-determining regions ("CDRs") of an anti-CEA murine MAb are grafted into the framework regions of a human antibody.

### B. Background

CEA is an oncofetal antigen commonly expressed in a number of epithelial cancers, most commonly those arising in the colon but also in the breast, lung, pancreas, thyroid (medullary type) and ovary *(*Goldenberg et al., J. Natl. Cancer Inst.57:11-22 (1976), Shively, et al., Crit. Rev. Oncol. Hematol. 2:355-399 (1985)). CEA was originally thought to be a tumor-specific antigen of colorectal cancer (Gold et al., J. Exper. Med., 122:467 (1965)). However, it was later found to be present in a diverse number of carcinomas, benign tumors, and diseased tissues, as well as in normal human colon (Shively et al., Crit. Rev. Oncol. Hematol., 2:355 (1985); von Kleist et al., Proc. Natl. Acad. Sci. U.S.A., 69:2492 (1972)). CEA has been shown to mediate cell-cell adhesion through homotypic and heterotypic interactions, which in turn have implicated a role for CEA in various aspects of tumorigenesis.

Medulary thyroid cancer (MTC) confined to the thyroid gland is potentially curable by total thyroidectomy and central lymph node dissection. However, disease recurs in approximately 50% of these patients. In addition, the prognosis of patients with unresectable disease or distant metastases is poor, less than 30% survive 10 years (Rossi et al., Amer. J. Surgery, 139:554 (1980); Samaan et al., J. Clin. Endocrinol. Metab., 67:801 (1988); Schroder et al., Cancer, 61:806 (1988). These patients are left with few therapeutic choices (Principles and Practice of Oncology, DeVita, Hellman and Rosenberg (eds.), New York: JB Lippincott Co. 1333-1435 (1989); Cance et al., Current Problems Surgery, 22:1 (1985)). Chemotherapy has been of little value and radiation therapy may only be used to control local disease (Cance *et al*.; Tubiana et al., Cancer, 55:2062 (1985)). Thus, new therapeutic modalities are needed to control this disease.

A useful approach to cancer therapy and diagnosis involves the use of targeting antibodies to deliver therapeutic and diagnostic agents directly to the site of a malignancy. Over the past decade, a wide variety of tumor-specific antibodies and antibody fragments have been developed, as have methods to conjugate the antibodies to therapeutic agents, such as drugs, toxins, radionuclides, immunomodulators, such as cytokines or other agents, and to administer the conjugates to patients that target the tumor. However, patients treated with drugs or radionuclides complexed with murine monoclonal antibodies (which have been the most commonly used targeting antibodies for humans) develop circulating human anti-mouse antibodies (HAMAs) and sometimes a generalized immediate type-III hypersensitivity reaction to the antibody moiety of the conjugate. But these problems have been minimized by making these murine antibodies less immunogenic by a number of different methods, which include making humanized, chimeric or human antibodies, by chemically modifying the targeting antibody, such as by conjugating to polyethylene glycol to the targeting antibody (PEGylation), or by characterizing the *situs* of antigenicity in an antibody and then removing it; e.g., Fab', F(ab)₂ and other antibody fragments have been used in place of whole IgG. In addition, attempts have been made to reduce the adverse effects of HAMA by plasmaphoretically removing HAMA from blood. Immunosuppressive techniques also have been used to ameliorate the adverse effect of the foreign antibody sufficiently to permit multiple treatments with the targeting agent.

Regardless of these treatment advances, there still exists a need to provide more affective methods of treating CEA-expressing cancers. The present invention provides an effective therapy utilizing a Class III anti-CEA MAb, the murine MN-14 MAb as defined in U.S. Patent No. 5,874,540 and Hansen et al., Cancer, 71:3478 (1993), and a Class III anti-CEA MAb, the chimeric and humanized MN-14 MAb as defined in U.S. Patent No. 5,874,540, and the NP-4 as defined in U.S. Patent No. 4,818,709 by Primus et al.*,* for example. Preferably, the Class III anti-CEA MAb is humanized, and used in combination with a therapeutic agent, particularly a chemotherapeutic agent, to yield an effective therapeutic treatment for CEA expressing cancers with minimal toxicity. Further, the separate administration of these two components provides enhanced results and the versatility and the flexibility to tailor individual treatment methods.

Stein et al. (Stein R et al. Cancer, January 2002, Vol. 94(1), pp. 51-61) teach combining radioimmunotherapy and chemotherapy for the treatment of medullary thyroid carcinoma.

Lonberg and Huszar (Lonberg N and Huszar D, Intern. Rev. Immunol., 1995, Vol. 13, pp. 65-93) teach the generation of human antibodies from transgenic mice.

US patent 6,096,289 is related to methods for close-range intraoperative, endoscopic and intravascular deflection and treatment of lesions, including tumors and non-malignant lesions, using antibody fragments or subfragments labeled with isotopic or non-isotopic agents.

### SUMMARY OF THE INVENTION

The problem underlying the present invention is solved by the subject matter of the attached independent claims. Preferred embodiments may be taken from the attached dependent claims.

More specifically, the present invention is related in a first aspect to a composition comprising at least one naked Class III anti-CEA monoclonal antibody (MAb) or fragment thereof and at least one therapeutic agent; in a second aspect to a naked Class III anti-CEA monoclonal antibody or fragment thereof for use in a method for treating CEA expressing non-medullary thyroid carcinoma comprising administering to a subject, either concurrently or sequentially, a therapeutically effective amount of the Class III anti-CEA monoclonal antibody or fragment thereof and at least one therapeutic agent, and optionally formulated in a pharmaceutically acceptable vehicle; and in a third aspect to a naked Class III anti-CEA monoclonal antibody or fragment thereof for use in treating medullary thyroid carcinoma comprising administering to a subject, either concurrently or sequentially, a therapeutically effective amount of the Class III anti-CEA monoclonal antibody or fragment thereof and at least one therapeutic agent, and optionally formulated in a pharmaceutically acceptable vehicle.

According to the first aspect of the present invention the composition comprises at least one Class III anti-CEA monoclonal antibody (MAb) or fragment thereof and at least one therapeutic agent. Preferably, the antibody fragment is selected from the group consisting of F(ab')2, Fab', Fab, Fv and scFv. Also preferred, the Class III anti-CEA MAb or fragment thereof is humanized, and wherein the humanized MAb retains substantially the Class III anti-CEA binding specificity of a murine Class III anti-CEA MAb. Also preferred, the Class III anti-CEA MAb or fragment thereof is a chimeric MAb, and wherein the chimeric MAb retains substantially the Class III anti-CEA binding specificity of murine Class III anti-CEA MAb. Still preferred, the Class III anti-CEA MAb or fragment thereof is a fully human MAb, and wherein said fully human MAb retains substantially the Class III anti-CEA binding specificity of murine Class III anti-CEA MAb.

In an embodiment of the present invention, the Class III anti-CEA monoclonal antibody or fragment thereof is preferably a MN-14 antibody or fragment thereof. More preferably, the MN-14 monoclonal antibody or fragment thereof comprises the complementarity-determining regions (CDRs) of a murine MN-14 monoclonal antibody, wherein the CDRs of the light chain variable region of the MN-14 antibody comprises CDR1 comprising the amino acid sequence KASQDVGTSVA; CDR2 comprising the amino acid sequence WTSTRHT; and CDR3 comprising the amino acid sequence QQYSLYRS; and the CDRs of the heavy chain variable region of the Class III anti-CEA antibody comprises CDR1 comprising TYWMS; CDR2 comprising EIHPDSSTINYAPSLKD; and CDR3 comprising LYFGFPWFAY. Also preferred, the MN-14 monoclonal antibody reacts with CEA and is unreactive with normal cross-reactive antigen (NCA) and meconium antigen (MA). Most preferably, the MN-14 monoclonal antibody or fragment thereof is a humanized, chimerized or fully human MN-14 antibody or fragment thereof.

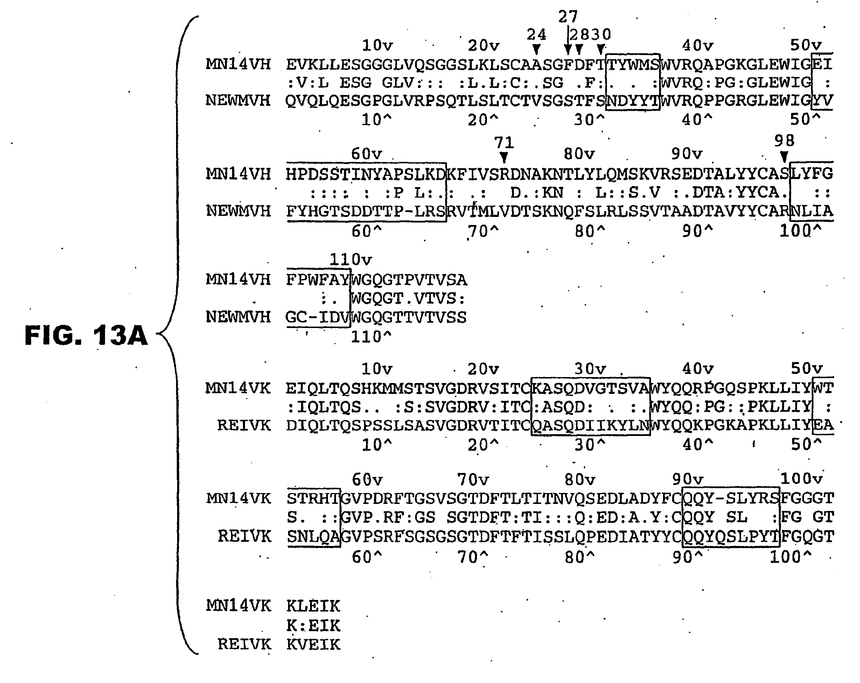

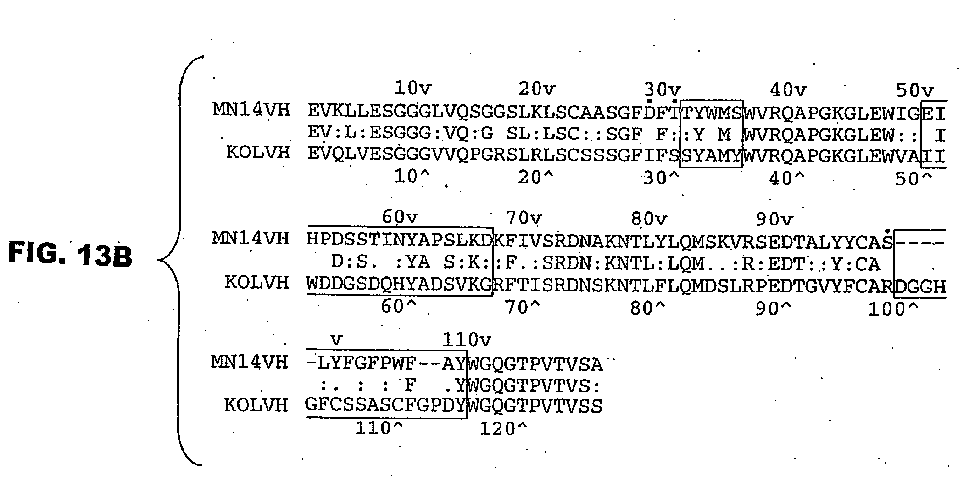

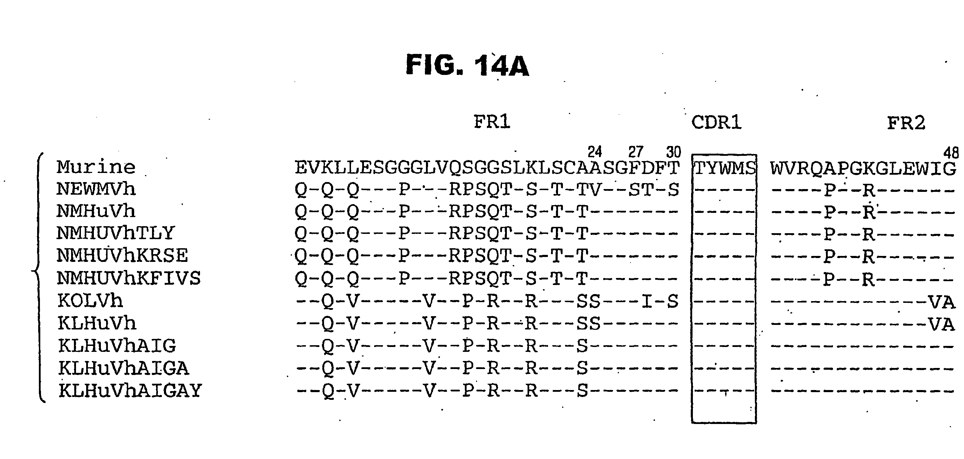

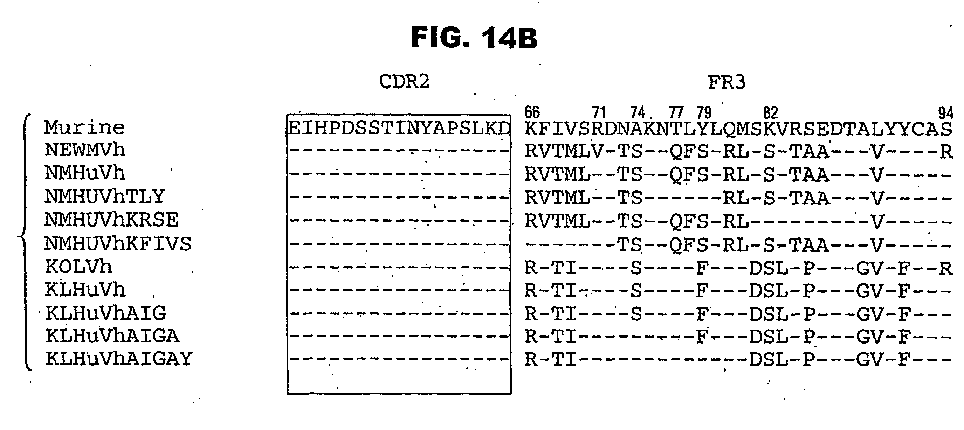

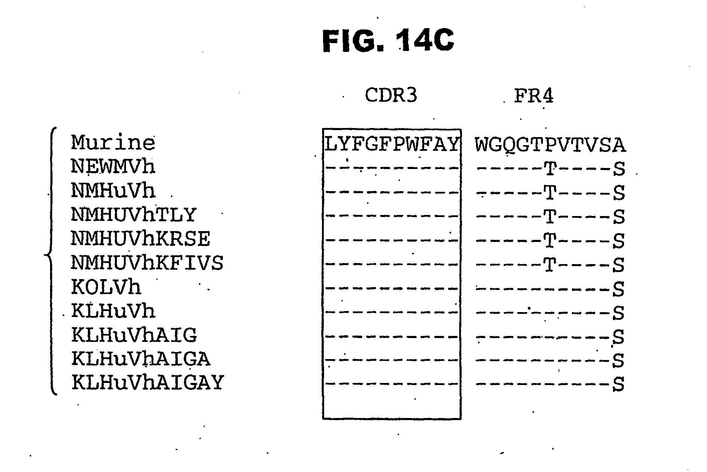

In a preferred embodiment, the framework regions (FRs) of the light and heavy chain variable regions of the humanized MN-14 antibody or fragment thereof comprise at least one amino acid substituted from the corresponding FRs of a murine MN-14 monoclonal antibody. Specifically, the humanized MN-14 antibody or fragment thereof preferably comprises at least one amino acid from the corresponding FR of the murine MN-14 antibody is selected from the group consisting of amino acid residue 24 (A), 28 (D), 30 (T), 48 (I), 49 (G), 74 (A) and 94 (S) of the murine heavy chain variable region (KLHuVhAIGA) of Figure 14A-C. Likewise, the humanized MN-14 antibody or fragment thereof may also comprise at least one amino acid from said corresponding FR of the murine MN-14 light chain variable region. Still preferred, the humanized MN-14 antibody or fragment thereof comprises the light chain variable region as set forth in Figure 13A, which is the middle sequence between the MN14VK and REIVK that is composed of a combination of sequences from both Vks of these antibodies, and the heavy chain variable region set forth in Figure 14A-C designated as KLHuVhAIGA.

In an embodiment of the present invention, the therapeutic agent is selected from the group consisting of a naked antibody, a cytotoxic agent, a drug, a radionuclide, an immunomodulator, a photoactive therapeutic agent, an immunoconjugate, a hormone, or a combination thereof, optionally formulated in a pharmaceutically acceptable vehicle. It is also contemplated herein that the therapeutic agent is not dacarbazine (DTIC).

In connection with the second and third aspect the method is a method for treating medullary as well as non-medullary thyroid carcinoma comprising administering to a subject, either concurrently or sequentially, a therapeutically effective amount a Class III anti-CEA monoclonal antibody or fragment thereof and at least one therapeutic agent, and optionally formulated in a pharmaceutically acceptable vehicle. Preferably, the antibody fragment is selected from the group consisting of F(ab')2, Fab', Fab, Fv and scFv. Also preferred, the Class III anti-CEA MAb or fragment thereof is humanized, wherein said humanized MAb retains substantially the Class III anti-CEA binding specificity of a murine Class III anti-CEA MAb. It is also contemplated that the Class III anti-CEA MAb or fragment thereof is a chimeric MAb, and wherein said chimeric MAb retains substantially the Class III anti-CEA binding specificity of murine Class III anti-CEA MAb.

In a preferred embodiment, the Class III anti-CEA monoclonal antibody or fragment thereof is a MN-14 antibody or fragment thereof Preferably, the MN-14 monoclonal antibody or fragment thereof comprises the complementanty-determining regions (CDRs) of a murine MN-14 monoclonal antibody, wherein the CDRs of the light chain variable region of said MN-14 antibody comprises CDR1 comprising the amino acid sequence KASQDVGTSVA; CDR2 comprising the amino acid sequence WTSTRHT; and CDR3 comprising the amino acid sequence QQYSLYRS; and the CDRs of the heavy chain variable region of said Class in anti-CEA antibody comprises CDR1 comprising TYWMS; CDR2 comprising EIHPDSSTINYAPSLKD; and CDR3 comprising LYFGFPWFAY. Also preferred, the MN-14 monoclonal antibody is humanized, chimerized or fully human, and reacts with CEA and is unreactive with normal cross-reactive antigen (NCA) and meconium antigen. Also preferred, the MN-14 antibody or fragment thereof is administered in a dosage of 100 to 600 milligrams protein per dose per injection. Most preferably, the MN-14 antibody or fragment thereof is administered in a dosage of 300 milligrams protein per dose per injection.

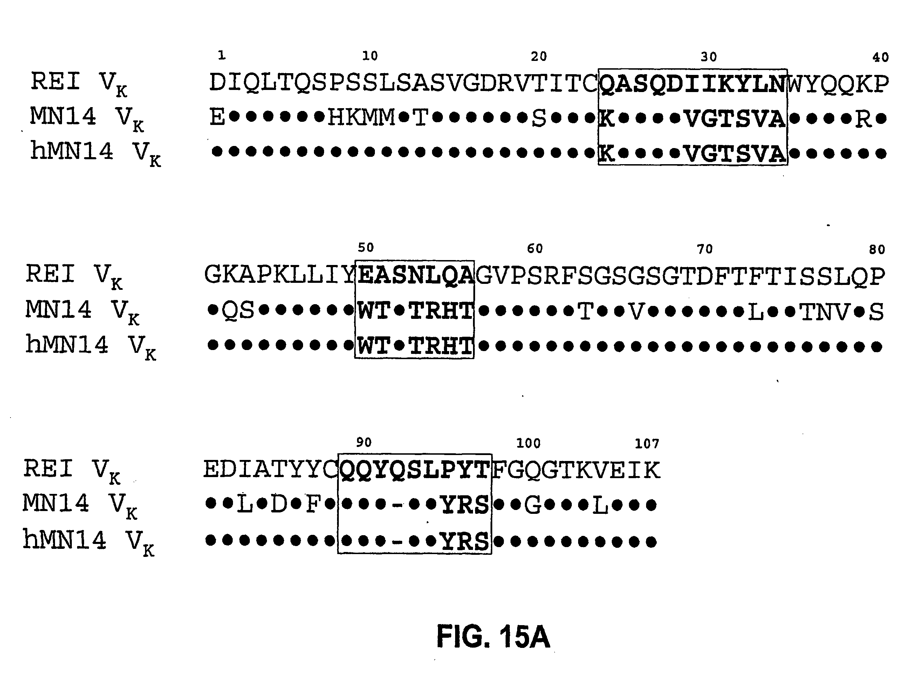

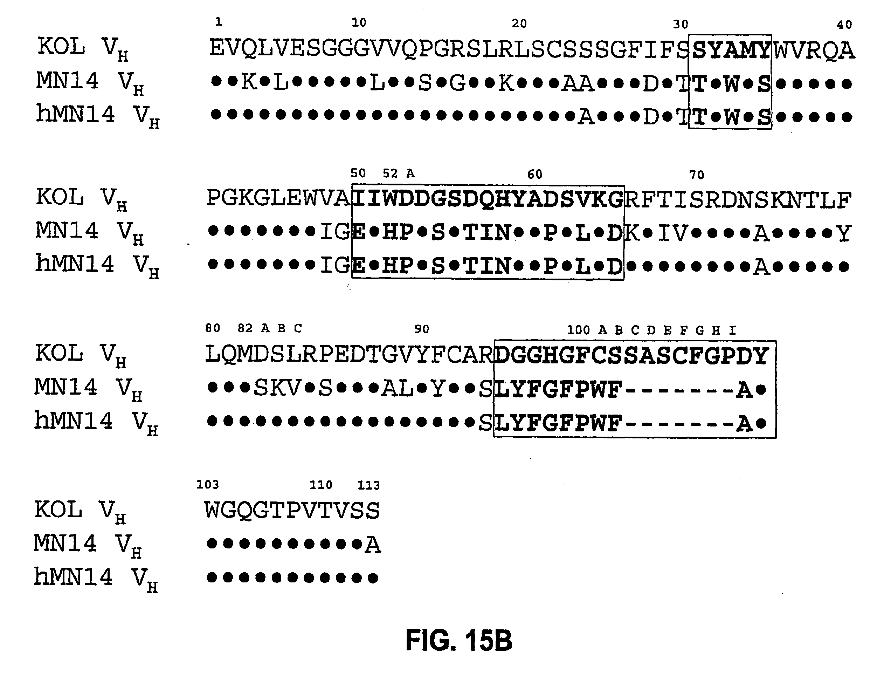

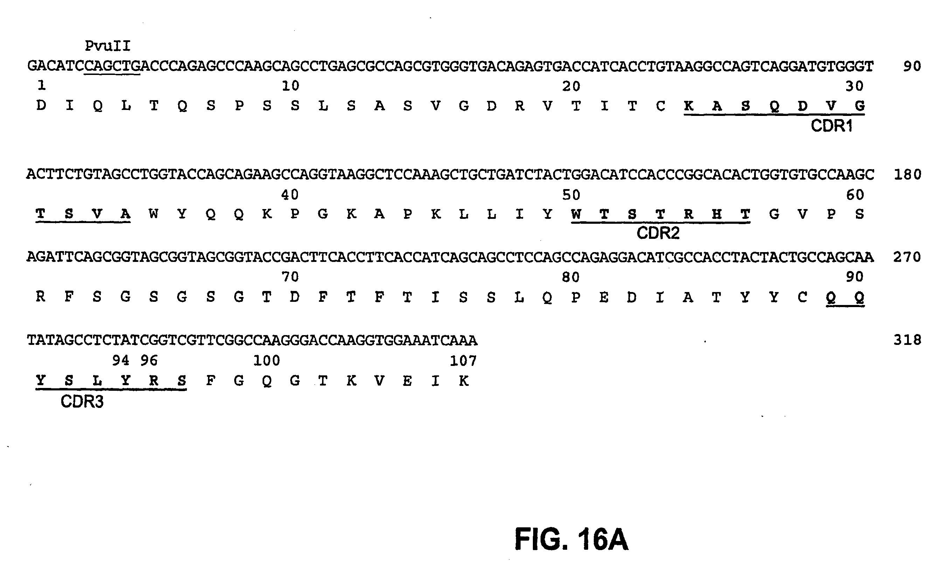

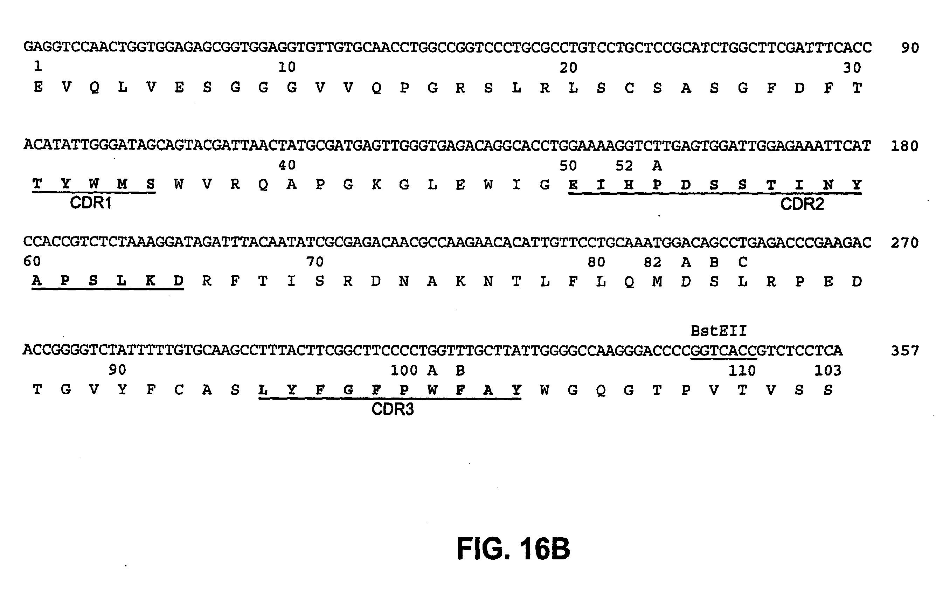

In the anti-CEA MAb of the instant invention, the framework regions (FRs) of the light and heavy chain variable regions of said humanized MN-14 antibody or fragment thereof preferably comprise at least one amino acid substituted from the corresponding FRs of a murine MN-14 monoclonal antibody. More preferred, the humanized MN-14 antibody or fragment thereof comprising at least one amino acid from said corresponding FR of said murine MN-14 antibody is selected from the group consisting of amino acid residue 24, 28, 30, 48, 49, 74 and 94 of the murine heavy chain variable region of Figure 14A-C, as noted above. Also preferred, the humanized MN-14 antibody or fragment thereof comprising at least one amino acid from said corresponding FR of said murine MN-14 light chain variable region. Most preferably, the humanized MN-14 antibody or fragment thereof comprises the light chain variable region as set forth in Figure 13A (middle sequence) or Figure 15A (hMN-14) or Figure 16A and the heavy chain variable region set forth in Figure 14A-C designated as KLHuVhAIGA or Figure 15B (hMn-14) or Figure 16B.

The methods of the instant invention also contemplate a therapeutic agent selected from the group consisting of a humanized, chimeric, human or murine monoclonal antibody or fragment thereof reactive with EGP-1, EGP-2 (e.g., 17-1A), MUC-1, MUC-2, MUC-3, MUC-4, PAM-4, KC4, TAG-72, EGFR, EGP-2, HER2/neu, BrE3, Le-Y, A3, A33, Ep-CAM, AFP, Tn, Thomson-Friedenreich antigens, VEGF, Ga 733, or a combination thereof. Similarly, the methods may comprise administering to a subject, either concurrently or sequentially, a therapeutically effective amount of a second humanized, chimeric, human or murine monoclonal antibody or fragment thereof selected from the group consisting of a Class II monoclonal antibody or fragment thereof. Preferably, the second antibody or fragment thereof is either naked or conjugated to a therapeutic agent

In a preferred embodiment, the therapeutic agent is selected from the group consisting of a naked antibody, cytotoxic agent, a drug, a radionuclide, an immunomodulator, a photoactive therapeutic agent, an immunoconjugate of a CEA or non-CEA antibody, a hormone, or a combination thereof, optionally formulated in a pharmaceutically acceptable vehicle. It is also contemplated that the therapeutic agent is not dacarbazine (DTIC).

Preferably, the therapeutic agent is a cytotoxic agent selected from the group consisting of a drug or a toxin. For example, it is contemplated that the drug possesses the pharmaceutical property selected from the group consisting of antimitotic, alkylating, antimetabolite, autiangiogenic, apoptotic, alkaloid, COX-2, and antibiotic agents and combinations thereof. Preferably, the drug is selected from the group consisting of nitrogen mustards, ethylenimine derivatives, alkyl sulfonates, nitrosoureas, triazenes, folic acid analogs, anthracyclines, taxanes, COX-2 inhibitors, pyrimidine analogs, purine analogs, antimetabolites, antibiotics, enzymes, epipodophyllotoxins, platinum coordination complexes, vinca alkaloids, substituted ureas, methyl hydrazine derivatives, adrenocortical suppressants, antagonists, endostatin, taxols, camptothecins, doxorubicins and their analogs, and a combination thereof.

When the therapeutic agent is a microbial, plant or animal toxin, the agent can be selected from the group consisting of ricin, abrin, alpha toxin, saporin, ribonuclease (RNase), DNase I, *Staphylococcal* enterotoxin-A, pokeweed antiviral protein, gelonin, diphtherin toxin, *Pseudomonas* exotoxin, and *Pseudomonas* endotoxin.

It is also contemplated in connection with the instant invention that the therapeutic agent is an immunomodulator is selected from the group consisting of a cytokine, a stem cell growth factor, a lymphotoxin, a hematopoietic factor, a colony stimulating factor (CSF), an interferon (IFN), a stem cell growth factor, erythropoietin, thrombopoietin and a combination thereof. Preferably, the lymphotoxin is tumor necrosis factor (TNF), said hematopoietic factor is an interleukin (IL), said colony stimulating factor is granulocyte-colony stimulating factor (G-CSF) or granulocyte macrophage-colony stimulating factor (GM-CSF)), said interferon is interferons-α, -β or -γ, and said stem cell growth factor is designated "S1 factor". Also preferred, the immunomodulator comprises IL-1, IL-2, IL-3, IL-6, IL-10, IL-12, IL-18, interferon-γ, TNF-α or a combination thereof. Also preferred the therapeutic agent is a photoactive therapeutic agent that is a chromogen or dye or an alkylating agent that is dacarbazine.

Also preferred, the therapeutic agent is a radionuclide that has an energy between 20 and 10,000 keV. Preferably, the radionuclide is selected from the group consisting of ¹²⁵I, ¹³¹I, ⁹⁰Y, ⁸⁸Y, ²²⁵Ac, ¹⁷⁷Lu, ¹⁸⁸Re, ¹⁸⁶Re, and combinations thereof.

### BRIEF DESCRIPTION OF THE DRAWINGS

**Figure 1**. Graphs comparing tumor volume after treatment with hMN-14 alone, DTIC alone or the combination of hMN-14 and DTIC. Figure 1A shows DTIC administered alone at 25 and 100 µg/dose or with 250 µg hMN-14 antibody, and Figure 1B shows DTIC administered alone at 50 and 75 µg/dose or with 100 µg hMN-14 antibody.
**Figure 2****.** Graph comparing tumor volume after radioimmunotherapy (RAIT) with ¹³¹I and ⁹⁰Y-MN-14.
**Figure 3****.** Graph comparing the therapeutic efficacy of several chemotherapeutic drugs on tumor volume in TT bearing mice
**Figure 4****.** Graph comparing the therapeutic efficacy of combination therapy of RAIT with ⁹⁰Y-labeled anti CEA MAb MN-14 and a 4-drug combination initiated 24 hours after RAIT on tumor volume in mice.
**Figure 5****.** Graph comparing the efficace of RAIT plus DTIC and RAIT plus doxorubicin and DTIC in TT bearing mice.
**Figure 6****.** Graph comparing effectiveness of naked hMN-14 treatment regimens in mice bearing TT xenografts with. Animals were given s.c. injections of TT cells, and either left untreated (A) or given an i.v. injection of 0.5 mg hMN-14 1 day (B) or 11 days (C) later.
**Figure 7****.** Graph comparing the effectiveness of humanized and murine MN-14 antibodies in treating medullary thyroid carcinoma. Animals were given s.c. injections of TT cells, and either left untreated or given an i.v. injection of MAb (0.5 mg) 1 day later.
**Figure 8**. Graph comparing the effectiveness of different hMN-14 doses in treating medullary thyroid carcinoma. Animals were given i.v. injections of increasing doses of hMN-14 1 day after s.c. injection of TT cells.
**Figure 9**. Graph comparing the effectiveness of different treatment times in TT bearing nude mice.
**Figure 10**. Graph comparing treatment of TT bearing nude mice with hMN-14 plus DTIC, DTIC alone, hMN-14 alone, and untreated mice.

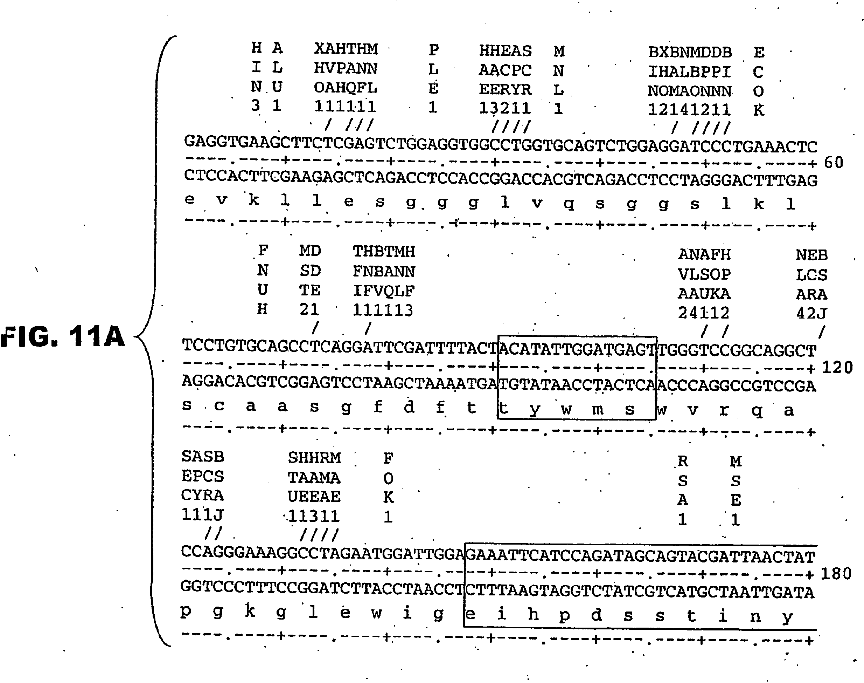

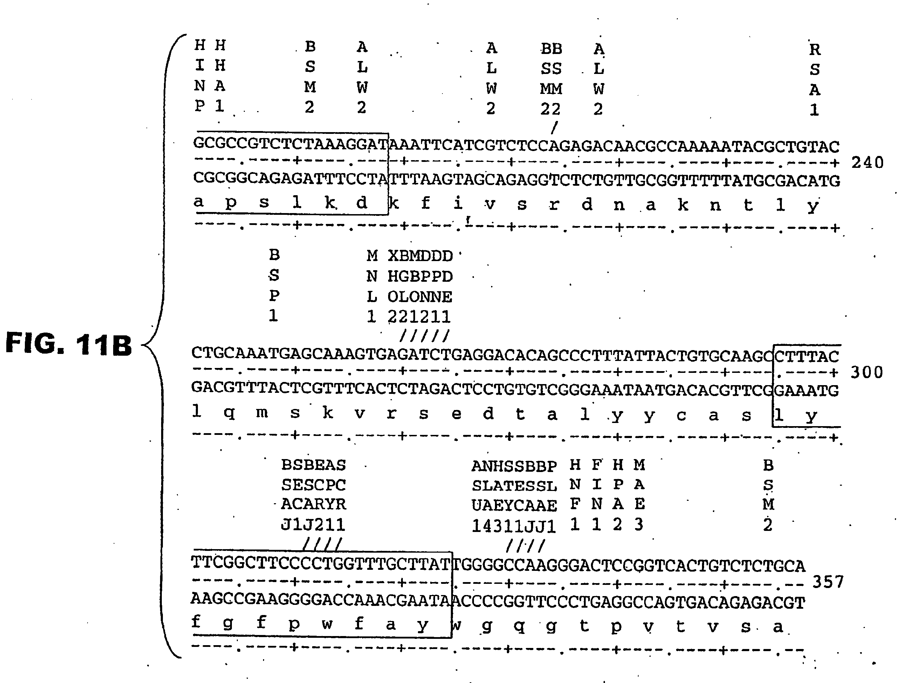

**Figure 11**. Figures 11A and 11B show the consensus DNA sequence of the murine MN-14 variable region heavy chain (VH) and the amino acid sequence encoded by the DNA sequence. The CDRs are enclosed in boxes.

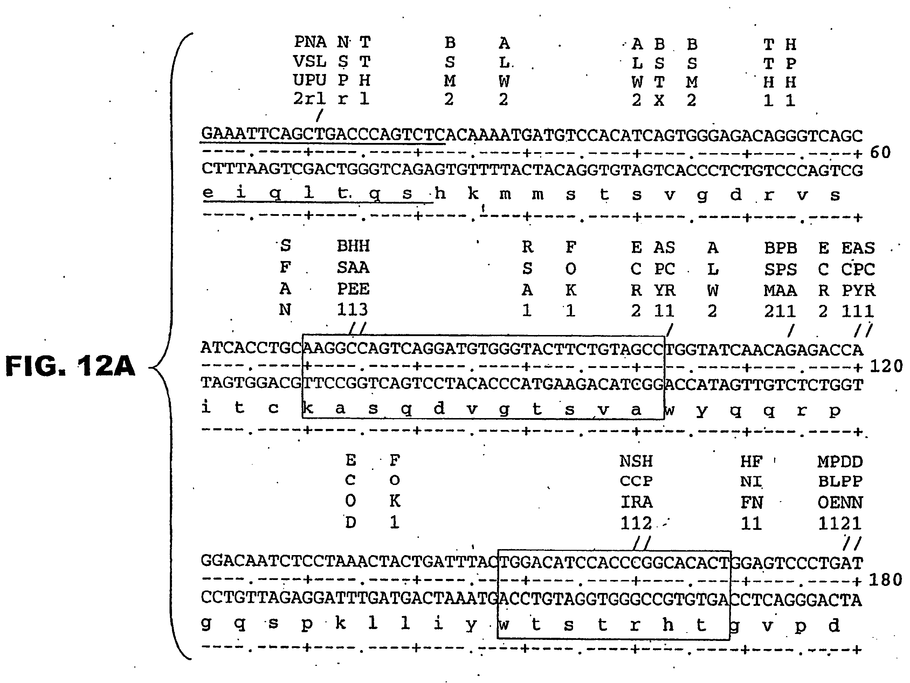

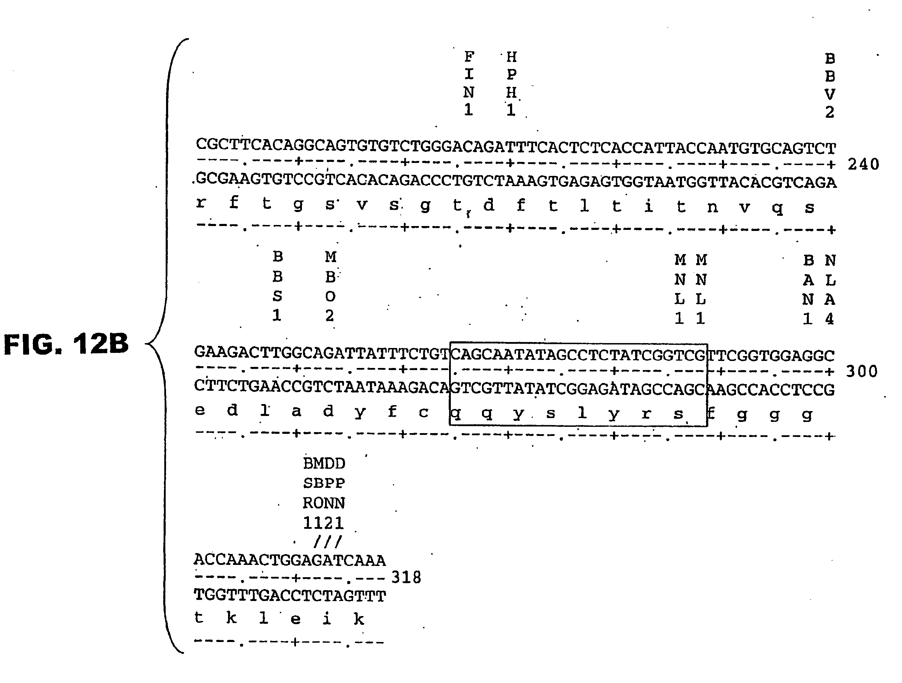

**Figure 12**. Figures 12A and 12B show the consensus DNA sequence of the murine MN-14 variable region light chain (VK) and the amino acid sequence encoded by the DNA sequence.
The CDRs are enclosed in boxes.
**Figure 13**. Figures 13A and 13B show the alignment of the murine MN-14 variable region of the with the human variable regions NEWM VH and REI VK (Figure 13A). and with the human KOL VH region (Figure 13B). CDRs are boxed, and the murine VH FRs, which are incorporated into the humanized VH, are marked with their positions according to the numbering system of Kabat et al. SEQUENCES OF PROTEINS OF IMMUNOLOGICAL INTEREST, U.S. Government Printing Office, Washington, D.C., 1987. Murine residues outside the CDRs that were included in the KLHuVH are indicated by a filled circle.
**Figure 14**. Figures 14A-14C show a comparison of the amino acid sequence between murine and humanized MN-14 VH framework residues (FR). Only human FR residues different from the mouse are shown. CDRs for NEWM and KOL are also not shown. The areas of amino acid substitutions in the respective FRs are highlighted in bold, and the position of the substitution indicated according to the Kabat et al. numbering system. The 3 CDRs are boxed.
**Figure 15**. Figures 15A and 15B show the comparison of the human, murine and humanized sequences of the Vk and VH regions of the human REI and KOL antibodies, respectively with murine and humanized MN-14. The human sequences of the REI Vk in Figure 15A are compared with the murine and humanized MN-14 Vk sequences. The closed circles indicate sequences retained from the humanREI Vk sequences. The CDRs are boxed. The human sequences of the KOL VH in Figure 15B are compared with the murine and humanized MN-14 VH sequences. The closed circles indicate sequences retained from the human KOL VH sequences. The CDRs are boxed.
**Figure 16**. Figures 16A and 16B show the Vk, the variable light chain, and the VH, the variable heavy chain sequences of hMN-14, a humanized Class III anti-CEA antibody. The CDR region sequences are shown in bold and underlined. The amino acid residues and the nucleotides are numbered sequentially. The light chain variable region is shown in Fig. 16A and the heavy chain variable region is shown in Fig. 16B

### DETAILED DESCRIPTION OF THE PREFERRED EMBODIMENTS

### 1. Overview

The present invention provides a composition comprising at least one naked Class III anti-CEA monoclonal antibody or a fragment thereof, and at least one therapeutic agent; as well as a naked anti-CEA monoclonal antibody or fragment thereof for use in methods of treatment in which the naked Class III anti-CEA antibody or fragment thereof and at least one therapeutic agent are administered either sequentially or concurrently over a treatment period. The MAb is particularly useful for treating medullary thyroid carcinoma but is surprisingly useful for treating non-medullary thyroid cancers, colorectal cancers, hepatocellular carcinoma, pancreatic, breast, lung, head-and-neck, bladder, uterine and ovarian cancers, and even cancers that do not express CEA at very high levels. For example, treatment is contemplated in cancers that express CEA at levels of at least 100 ng/g of tissue.The present method further provides compositions comprising the Class III anti-CEA antibody or antibody fragment in which the antibody and the therapeutic agent are not conjugated or linked to each other. As used herein, the phrase "Class III anti-CEA" antibody or antibody fragment means an antibody or fragment that binds the CEA antigen (or CD66e) and is unreactive with normal cross-reactive antigen (NCA), meconium antigen (MA), granulocytes and CD66a-d (*see,* Primus et al., U.S. Patent No. 4,818,709). The naked Class III anti-CEA antibody or fragment thereof may be a humanized, chimeric, human or murine antibody. In a preferred embodiment, the naked Class III anti-CEA antibody or fragment thereof is a humanized MN-14 antibody or fragment thereof.

Surprisingly, the compositions and methods described herein are also useful for treating CEA expressing non-medullary thyroid carcinoma, including colorectal cancer, pancreatic cancer, breast cancer, hepatocellular carcinoma, and ovarian cancer. Because such forms of cancer express less CEA than medullary thyroid cancers, it was unexpected that a naked Class III anti-CEA antibody, in combination with a therapeutic agent, would be useful for treating non-medullary thyroid carcinomas.

The mechanism of tumor cell killing by the naked Class II anti-CEA antibody is not known with certainty and is likely involves several mechanisms. It is hypothesized that the naked antibody alone or in combination with the therapeutic agent may affect tumor growth by blocking biological activities of their respective antigens or by stimulating natural immunological functions, such as antibody-dependent cell-mediated cytotoxicity (ADCC) or complement-mediated lysis. Additionally, the naked antibody alone or in combination with the therapeutic agent may treat and control the cancer by inhibiting cell growth and cell cycle progression, inducing apoptosis, inhibiting angiogenesis, inhibiting metastatic activity, and/or affecting tumor cell adhesion. In fact, the anti-CEA antibody or fragment thereof of the present invention may be more effective in treating metastases than primary cancers, since the metastases may be more susceptible to antagonists of tumor cell adhesion. The present treatment method provides a treatment plan that may be optimized to provide the maximum anti-tumor activity for individual patients by allowing the titration of the antibody and one or more different therapeutic agents to provide an effective treatment regimen.

In one aspect of the present invention, the naked Class III anti-CEA antibody or fragment thereof and therapeutic agent may be supplemented with at least one additional therapeutic agent, such as a naked or conjugated humanized, murine, chimeric or human antibody, fusion protein, or fragment thereof. For example, another class III CEA antibody or antibody fragment that is non-blocking and does not bind granulocytes or CD66a-d; a Class II anti-CEA antibody or antibody fragment that is non-blocking and does not bind granulocytes or CD66a-d; or an antibody against a different carcinoma-associated epitope or antigen, may be used as the therapeutic agent for combination therapy with the preferred humanized MN-14 antibody. Such an additional antibody, fusion protein or fragment thereof may bind CEA or another cancer or tumor-associated antigen, as described in more detail below.

### 2. Definitions

In the description that follows, a number of terms are used and the following definitions are provided to facilitate understanding of the present invention.

An antibody as described herein, refers to a full-length (i.e., naturally occurring or formed by normal immunoglobulin gene fragment recombinatorial processes) immunoglobulin molecule (*e.g*., an IgG antibody) or an immunologically active (i.e., specifically binding) portion of an immunoglobulin molecule, like an antibody fragment.

An antibody fragment is a portion of an antibody such as F(ab')₂, F(ab)₂, Fab', Fab, Fv, scFv (single chain Fv) and the like. Regardless of structure, an antibody fragment binds with the same antigen that is recognized by the intact antibody.

The term "antibody fragment" also includes any synthetic or genetically engineered protein that acts like an antibody by binding to a specific antigen to form a complex. For example, antibody fragments include isolated fragments consisting of the variable regions, such as the "Fv" fragments consisting of the variable regions of the heavy and light chains, recombinant single chain polypeptide molecules in which light and heavy variable regions are connected by a peptide linker ("scFv proteins"), and minimal recognition units consisting of the amino acid residues that mimic the hypervariable region. The Fv fragments may be constructed in different ways as to yield multivalent and/or multispecific binding forms. In the former case of multivalent, they react with more than one binding site against the CEA epitope, whereas with multispecific forms, more than one epitope (either of CEA or even against CEA and a different antigen) is bound.

As used herein, the term antibody component includes both an entire antibody, a fusion protein, and fragments thereof.

A naked antibody is generally an entire antibody which is not conjugated to a therapeutic agent. This is so because the Fc portion of the antibody molecule provides effector or immunological functions, such as complement fixation and ADCC (antibody dependent cell cytotoxicity), which set mechanisms into action that may result in cell lysis. However, the Fc portion may not be required for therapeutic function of the antibody, but rather other mechanisms, such as apoptosis, anti-angiogenesis, anti-metastatic activity, anti-adhesion activity, such as inhibition of heterotypic or homotypic adhesion, and interference in signaling pathways, may come into play and interfere with the disease progression. Naked antibodies include both polyclonal and monoclonal antibodies, and fragments thereof, that include murine antibodies, as well as certain recombinant antibodies, such as chimeric, humanized or human antibodies and fragments thereof. As defined in the present invention, "naked" is synonymous with "unconjugated," and means not linked or conjugated to the therapeutic agent with which it administered.

A chimeric antibody is a recombinant protein that contains the variable domains of both the heavy and light antibody chains, including the complementarity determining regions (CDRs) of an antibody derived from one species, preferably a rodent antibody, while the constant domains of the antibody molecule are derived from those of a human antibody. For veterinary applications, the constant domains of the chimeric antibody may be derived from that of other species, such as a cat or dog.

A humanized antibody is a recombinant protein in which the CDRs from an antibody from one species; e.g., a rodent antibody, is transferred from the heavy and light variable chains of the rodent antibody into human heavy and light variable domains. The constant domains of the antibody molecule is derived from those of a human antibody.

A human antibody is an antibody obtained from transgenic mice that have been "engineered" to produce specific human antibodies in response to antigenic challenge. In this technique, elements of the human heavy and light chain locus are introduced into strains of mice derived from embryonic stem cell lines that contain targeted disruptions of the endogenous heavy chain and light chain loci. The transgenic mice can synthesize human antibodies specific for human antigens, and the mice can be used to produce human antibody-secreting hybridomas. Methods for obtaining human antibodies from transgenic mice are described by Green et al., Nature Genet. 7:13 (1994), Lonberg et al., Nature 368:856 (1994), and Taylor et al., Int. Immun. 6:579 (1994). A fully human antibody also can be constructed by genetic or chromosomal transfection methods, as well as phage display technology, all of which are known in the art. See for example, McCafferty et al., Nature 348:552-553 (1990) for the production of human antibodies and fragments thereof *in vitro,* from immunoglobulin variable domain gene repertoires from unimmunized donors. In this technique, antibody variable domain genes are cloned in-frame into either a major or minor coat protein gene of a filamentous bacteriophage, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. In this way, the phage mimics some of the properties of the B cell. Phage display can be performed in a variety of formats, for their review, see e.g. Johnson and Chiswell, Current Opiniion in Structural Biology 3:5564-571 (1993).

Human antibodies may also be generated by *in vitro* activated B cells. See U.S. Patent Nos. 5,567,610 and 5,229,275.

A therapeutic agent is a molecule or atom which is administered separately, concurrently or sequentially with an antibody component, i.e., an antibody or antibody fragment, or a subfragment thereof, and is useful in the treatment of a disease. Examples of therapeutic agents include antibodies, antibody fragments, immunoconjugates, drugs, cytotoxic agents, toxins, nucleases, hormones, immunomodulators, chelators, boron compounds, photoactive agents or dyes, radioisotopes or radionuclides, immunoconjugates or combinations thereof.

An immunoconjugate is an antibody component conjugated to a therapeutic agent. Suitable therapeutic agents are described above.

As used herein, the term antibody fusion protein is a recombinantly-produced antigen-binding molecule in which two or more of the same or different natural antibody, single-chain antibody or antibody fragment segments with the same or different specificities are linked. A Class III anti-CEA fusion protein comprises at least one CEA binding site. Preferably, the Class III anti-CEA fusion protein is a MN-14 fusion protein.

Valency of the fusion protein indicates the total number of binding arms or sites the fusion protein has to antigen(s) or epitope(s); i.e., monovalent, bivalent, trivalent or mutlivalent. The multivalency of the antibody fusion protein means that it can take advantage of multiple interactions in binding to an antigen, thus increasing the avidity of binding to the antigen, or to different antigens. Specificity indicates how many different types of antigen or epitope an antibody fusion protein is able to bind; i.e., monospecific, bispecific, trispecific, multispecific. Using these definitions, a natural antibody, e.g., an IgG, is bivalent because it has two binding arms but is monospecific because it binds to one type of antigen or epitope. A monospecific, multivalent fusion protein has more than one binding site for the same antigen or epitope. For example, a monospecific diabody is a fusion protein with two binding sites reactive with the same antigen. The fusion protein may comprise a multivalent or multispecific combination of different antibody components or multiple copies of the same antibody component. The fusion protein may additionally comprise a therapeutic agent.

An immunomodulator is a therapeutic agent as defined in the present invention that when present, alters, suppresses or stimulates the body's immune system. Typically, the immunomodulator useful in the present invention stimulates immune cells to proliferate or become activated in an immune response cascade, such as macrophages, B-cells, and/or T-cells. An example of an immunomodulator as described herein is a cytokine, which is a soluble small protein of approximately 5-20 kDs that are released by one cell population (e.g., primed T-lymphocytes) on contact with specific antigens, and which act as intercellular mediators between cells. As the skilled artisan will understand, examples of cytokines include lymphokines, monokines, interleukins, and several related signalling molecules, such as tumor necrosis factor (TNF) and interferons. Chemokines are a subset of cytokines. Certain interleukins and interferons are examples of cytokines that stimulate T cell or other immune cell proliferation.

### Preparation of Monoclonal Antibodies, Including Chimeric, Humanized and Human Antibodies

Monoclonal antibodies (MAbs) are a homogeneous population of antibodies to a particular antigen and the antibody comprises only one type of antigen binding site and binds to only one epitope on an antigenic determinant. Rodent monoclonal antibodies to specific antigens may be obtained by methods known to those skilled in the art. *See*, for example, Kohler and Milstein, Nature 256: 495 (1975), and Coligan et al. (eds.), CURRENT PROTOCOLS IN IMMUNOLOGY, VOL. 1, pages 2.5.1-2.6.7 (John Wiley & Sons 1991) [hereinafter "Coligan"]. Briefly, monoclonal antibodies can be obtained by injecting mice with a composition comprising an antigen, verifying the presence of antibody production by removing a serum sample, removing the spleen to obtain B-lymphocytes, fusing the B-lymphocytes with myeloma cells to produce hybridomas, cloning the hybridomas, selecting positive clones which produce antibodies to the antigen, culturing the clones that produce antibodies to the antigen, and isolating the antibodies from the hybridoma cultures.

MAbs can be isolated and purified from hybridoma cultures by a variety of well-established techniques. Such isolation techniques include affinity chromatography with Protein-A Sepharose, size-exclusion chromatography, and ion-exchange chromatography. See, for example, Coligan at pages 2.7.1-2.7.12 and pages 2.9.1-2.9.3. Also, see Baines et al., "Purification of Immunoglobulin G (IgG)," in METHODS IN MOLECULAR BIOLOGY, VOL. 10, pages 79-104 (The Humana Press, Inc. 1992).

Abs to peptide backbones are generated by well-known methods for Ab production. For example, injection of an immunogen, such as (peptide)ₙ-KLH, wherein KLH is keyhole limpet hemocyanin, and n=1-30, in complete Freund's adjuvant, followed by two subsequent injections of the same immunogen suspended in incomplete Freund's adjuvant into immunocompetent animals. The animals are given a final i.v. boost of antigen, followed by spleen cell harvesting three days later. Harvested spleen cells are then fused with Sp2/0-Ag14 myeloma cells and culture supernatants of the resulting clones analyzed for anti-peptide reactivity using a direct-binding ELISA. Fine specificity of generated Abs can be analyzed for by using peptide fragments of the original immunogen. These fragments can be prepared readily using an automated peptide synthesizer. For Ab production, enzyme-deficient hybridomas are isolated to enable selection of fused cell lines. This technique also can be used to raise antibodies to one or more of the chelates comprising the linker, e.g., In(III)-DTPA chelates. Monoclonal mouse antibodies to an In(III)-di-DTPA are known (U.S. Patent No. 5,256,395 to Barbet).

Another method for producing antibodies is by production in the milk of transgenic livestock. See, e.g., Colman, A., Biochem. Soc. Symp., 63: 141-147, 1998; U.S. Patent 5,827,690. Two DNA constructs are prepared which contain, respectively, DNA segments encoding paired immunoglobulin heavy and light chains. The DNA segments are cloned into expression vectors that contain a promoter sequence that is preferentially expressed in mammary epithelial cells. Examples include, but are not limited to, promoters from rabbit, cow and sheep casein genes, the cow α-lactoglobulin gene, the sheep β-lactoglobulin gene and the mouse whey acid protein gene. Preferably, the inserted fragment is flanked on its 3' side by cognate genomic sequences from a mammary-specific gene. This provides a polyadenylation site and transcript-stabilizing sequences. The expression cassettes are coinjected into the pronuclei of fertilized, mammalian eggs, which are then implanted into the uterus of a recipient female and allowed to gestate. After birth, the progeny are screened for the presence of both transgenes by Southern analysis. In order for the antibody to be present, both heavy and light chain genes must be expressed concurrently in the same cell. Milk from transgenic females is analyzed for the presence and functionality of the antibody or antibody fragment using standard immunological methods known in the art. The antibody can be purified from the milk using standard methods known in the art.

After the initial raising of antibodies to the immunogen, the variable genes of the monoclonal antibodies can be cloned from the hybridoma cells, sequenced and subsequently prepared by recombinant techniques. General techniques for cloning murine immunoglobulin variable domains are described, for example, by the publication of Orlandi et al., Proc. Nat'l Acad. Sci. USA 86: 3833 (1989). Humanization and chimerization of murine antibodies and antibody fragments are well known to those skilled in the art. A chimeric antibody is a recombinant protein that contains the variable domains including the CDRs derived from one species of animal, such as a rodent antibody, while the remainder of the antibody molecule; i.e., the constant domains, is derived from a human antibody. The use of antibody components derived from humanized and chimerized monoclonal antibodies alleviates potential problems associated with the immunogenicity of murine constant regions. Techniques for constructing chimeric antibodies are well known to those of skill in the art. As an example, Leung et al., Hybridoma 13:469 (1994), describe how they produced an LL2 chimera by combining DNA sequences encoding the V_{κ} and V_{H} domains of LL2 monoclonal antibody, an anti-CD22 antibody, with respective human κ and IgG₁ constant region domains.

A chimeric monoclonal antibody (MAb) can also be humanized by replacing the sequences of the murine FR in the variable domains of the chimeric MAb with one or more different human FR. Specifically, humanized monoclonal antibodies are produced by transferring mouse complementary determining regions from heavy and light variable chains of the mouse immunoglobulin into a human variable domain, and then, substituting human residues in the framework regions of the murine counterparts. As simply transferring mouse CDRs into human FRs often results in a reduction or even loss of antibody affinity, additional modification might be required in order to restore the original affinity of the murine antibody. This can be accomplished by the replacement of one or more human residues in the FR regions with their murine counterparts to obtain an antibody that possesses good binding affinity to its epitope. See, for example, Tempest et al., Biotechnology 9:266 (1991) and Verhoeyen et al., Science 239:1534 (1988).

In a preferred embodiment, some human residues in the framework regions of the humanized anti-CEA antibody or fragments thereof are replaced by their murine counterparts. Additionally, knowing that chimeric anti-CEA exhibits a binding affinity comparable to that of its murine counterpart, defective designs, if any, in the original version of the humanized anti-CEA MAb can be identified by mixing and matching the light and heavy chains of the chimeric anti-CEA to those of the humanized version. Preferably, the humanized anti-CEA antibody is a humanized MN-14 antibody, and it preparation and sequences are disclosed in U.S. 5,874,540. Although the two human antibodies are REI and NEWM are the preferred antibodies for preparing both humanized and chimeric MN-14 antibodies, a combination of framework sequences from 2 or more different human antibodies can be used for V_{H} and V_{K}. The production of humanized MAbs are described, for example, by Jones et al., Nature 321: 522 (1986), Riechmann et al., Nature 332: 323 (1988), Verhoeyen et al., Science 239: 1534 (1988), Carter et al., Proc. Nat'l Acad. Sci. USA 89: 4285 (1992), Sandhu, Crit. Rev. Biotech. 12: 437 (1992), and Singer et al., J. Immun. 150: 2844 (1993). Further, the affinity of humanized, chimeric and human MAbs to a specific epitope can be increased by mutagenesis of the CDRs, so that a lower dose of antibody may be as effective as a higher dose of a lower affinity MAb prior to mutagenesis. See for example, WO0029584A1.

In another embodiment, an antibody of the present invention is a human Class III anti-CEA monoclonal antibody. The anti-CEA MAb, or another human antibody, can be obtained from a transgenic non-human animal. See, e.g., Mendez et al., Nature Genetics, 15: 146-156 (1997) and U.S. Patent No. 5,633,425. For example, a human antibody can be recovered from a transgenic mouse possessing human immunoglobulin loci. Preferably, the anti-CEA antibody is an MN-14 antibody. The mouse humoral immune system is humanized by inactivating the endogenous immunoglobulin genes and introducing human immunoglobulin loci. The human immunoglobulin loci are exceedingly complex and comprise a large number of discrete segments which together occupy almost 0.2% of the human genome. To ensure that transgenic mice are capable of producing adequate repertoires of antibodies, large portions of human heavy- and light-chain loci must be introduced into the mouse genome. This is accomplished in a stepwise process beginning with the formation of yeast artificial chromosomes (YACs) containing either human heavy- or light-chain immunoglobulin loci in germline configuration. Since each insert is approximately 1 Mb in size, YAC construction requires homologous recombination of overlapping fragments of the immunoglobulin loci. The two YACs, one containing the heavy-chain loci and one containing the light-chain loci, are introduced separately into mice via fusion of YAC-containing yeast spheroblasts with mouse embryonic stem cells. Embryonic stem cell clones are then microinjected into mouse blastocysts. Resulting chimeric males are screened for their ability to transmit the YAC through their germline and are bred with mice deficient in murine antibody production. Breeding the two transgenic strains, one containing the human heavy-chain loci and the other containing the human light-chain loci, creates progeny which produce human antibodies in response to immunization.

Unrearranged human immunoglobulin genes also can be introduced into mouse embryonic stem cells via microcell-mediated chromosome transfer (MMCT). See, e.g., Tomizuka et al., Nature Genetics, 16: 133 (1997). In this methodology microcells containing human chromosomes are fused with mouse embryonic stem cells. Transferred chromosomes are stably retained, and adult chimeras exhibit proper tissue-specific expression.

As an alternative, an antibody or antibody fragment of the present invention may be derived from human antibody fragments isolated from a combinatorial immunoglobulin library. See, e.g., Barbas et al., METHODS: A Companion to Methods in Enzymology 2: 119 (1991), and Winter et al., Ann. Rev. Immunol. 12: 433 (1994). Many of the difficulties associated with generating monoclonal antibodies by B-cell immortalization can be overcome by engineering and expressing antibody fragments in *E. coli,* using phage display. To ensure the recovery of high affinity, monoclonal antibodies a combinatorial immunoglobulin library must contain a large repertoire size. A typical strategy utilizes mRNA obtained from lymphocytes or spleen cells of immunized mice to synthesize cDNA using reverse transcriptase. The heavy- and light-chain genes are amplified separately by PCR and ligated into phage cloning vectors. Two different libraries are produced, one containing the heavy-chain genes and one containing the light-chain genes. Phage DNA is islolated from each library, and the heavy-and light-chain sequences are ligated together and packaged to form a combinatorial library. Each phage contains a random pair of heavy- and light-chain cDNAs and upon infection of *E. coli* directs the expression of the antibody chains in infected cells. To identify an antibody that recognizes the antigen of interest, the phage library is plated, and the antibody molecules present in the plaques are transferred to filters. The filters are incubated with radioactively labeled antigen and then washed to remove excess unbound ligand. A radioactive spot on the autoradiogram identifies a plaque that contains an antibody that binds the antigen. Cloning and expression vectors that are useful for producing a human immunoglobulin phage library can be obtained, for example, from STRATAGENE Cloning Systems (La Jolla, CA).

In one embodiment, the antibodies of the present invention are produced as described in Hansen et al., U.S. Patent No. 5,874,540; Hansen et al., Cancer, 71:3478 (1993); Primus et al., U.S. Patent No. 4,818,709, and Shively et al., U.S. Patent No. 5,081,235.

### Production of Antibody Fragments

The present invention contemplates the use of fragments of a Class III anti-CEA antibody, preferably a MN-14 antibody. The Class III anti-CEA antibody or fragment thereof of the present invention does not bind granulocytes or CD66a-d. Antibody fragments which recognize specific epitopes can be generated by known techniques. For example, antibody fragments can be prepared by proteolytic hydrolysis of an antibody or by expression in *E. coli* of the DNA coding for the fragment. The antibody fragments are antigen binding portions of an antibody, such as F(ab')₂, Fab', Fab, Fv, scFv and the like, and can be obtained by pepsin or papain digestion of whole antibodies by conventional methods.

For example, an antibody fragment can be produced by enzymatic cleavage of antibodies with pepsin to provide a 100 Kd fragment denoted F(ab')₂. This fragment can be further cleaved using a thiol reducing agent, and optionally a blocking group for the sulfhydryl groups resulting from cleavage of disulfide linkages, to produce 50 Kd Fab' monovalent fragments. Alternatively, an enzymatic cleavage using papain produces two monovalent Fab fragments and an Fc fragment directly. These methods are described, for example, by Goldenberg, U.S. Patent Nos. 4,036,945 and 4,331,647 and references contained therein. Also, see Nisonoff et al., Arch Biochem. Biophys. 89: 230 (1960); Porter, Biochem. J. 73:119 (1959), Edelman et al., in METHODS IN ENZYMOLOGY VOL. 1, page 422 (Academic Press 1967), and Coligan at pages 2.8.1-2.8.10 and 2.10. 2.10.4.

Other methods of cleaving antibodies, such as separation of heavy chains to form monovalent light-heavy chain fragments, further cleavage of fragments, or other enzymatic, chemical or genetic techniques may also be used, so long as the fragments bind to the antigen that is recognized by the intact antibody.

For example, Fv fragments comprise an association of V_{H} and V_{L} chains. This association can be noncovalent, as described in Inbar et al., Proc. Nat'l. Acad. Sci. U.S.A. 69:2659 (1972). Alternatively, the variable chains can be linked by an intermolecular disulfide bond or cross-linked by chemicals such as glutyraldehyde. See, for example, Sandhu, Crit. Rev. Biotech. 12:437 (1992).

Preferably, the Fv fragments comprise V_{H} and V_{L} chains which are connected by a peptide linker. These single-chain antigen binding proteins (sFv) are prepared by constructing a structural gene comprising DNA sequences encoding the V_{H} and V_{L} domains which are connected by an oligonucleotide. The structural gene is inserted into an expression vector that is subsequently introduced into a host cell, such as *E*. *coli.* The recombinant host cells synthesize a single polypeptide chain with a linker peptide bridging the two V domains. Methods for producing sFvs are described, for example, by Whitlow et al., Methods: A Companion to Methods in Enzymology, 2:97 (1991). Also see Bird et al., Science 242:423 (1988), Ladner et al., U.S. Patent No. 4,946,778; Pack et al., Bio Technology 11:1271 (1993) and Sandhu, *supra*.

Another form of an antibody fragment is a peptide coding for a single complementarity-determining region (CDR). A CDR is a segment of the variable region of an antibody that is complementary in structure to the epitope to which the antibody binds and is more variable than the rest of the variable region. Accordingly, a CDR is sometimes referred to as hypervariable region. A variable region comprises three CDRs. CDR peptides can be obtained by constructing genes encoding the CDR of an antibody of interest. Such genes are prepared, for example, by using the polymerase chain reaction to synthesize the variable region from RNA of antibody-producing cells. See, for example, Larrick et al., Methods: A Companion to Methods in Enzymology 2: 106 (1991); Courtenay-Luck, "Genetic Manipulation of Monoclonal Antibodies," in MONOCLONAL ANTIBODIES: PRODUCTION, ENGINEERING AND CLINICAL APPLICATION, Ritter et al. (eds.), pages 166-179 (Cambridge University Press 1995); and Ward et al., "Genetic Manipulation and Expression of Antibodies," in MONOCLONAL ANTIBODIES: PRINCIPLES AND APPLICATIONS, Birch et al., (eds.), pages 137-185 (Wiley-Liss, Inc. 1995).

Other methods of cleaving antibodies, such as separation of heavy chains to form monovalent light-heavy chain fragments, further cleavage of fragments, or other enzymatic, chemical or genetic techniques may also be used, so long as the fragments bind to the antigen that is recognized by the intact antibody.

### Humanized, Chimeric and Human anti-CEA Antibodies for Treatment

Described in the present invention are compositions and methods using murine, chimeric, humanized and human Class III anti-CEA antibodies and fragments thereof for treatment. Preferably, the Class III anti-CEA antibody or fragment thereof is a MN-14 antibody or fragment thereof. The antibodies of the present invention can be used to treat medullary thyroid carcinoma (MTC), as well as non-MTC CEA expressing carcinomas. Exemplary non-MTC CEA expressing carcinomas include colorectal cancer, pancreatic cancer, hepatocellular carcinoma, gastric cancer, lung cancer, head-and neck cancers, urinary bladder cancer, uterine cancer, breast cancer, and ovarian cancer.

### Compositions

Contemplated herein is a composition comprising at least one Class III anti-CEA monoclonal antibody (MAb) or fragment thereof and at least one therapeutic agent, which are not conjugated to each other, and thus are present in the composition as unconjugated forms of each of the components. In compositions comprising more than one antibody or antibody fragments, such as a second Class III anti-CEA antibody, the second antibody is non-blocking (*i.e*., does not block binding of the first Class III anti-CEA antibody or antibody fragment).

In one embodiment, the Class III anti-CEA monoclonal antibody or fragment thereof is humanized, chimeric, or fully human, wherein the humanized, chimeric, or fully human MAb retains substantially the Class III anti-CEA binding specificity of a murine Class III anti-CEA MAb.

In a preferred embodiment, the Class III anti-CEA monoclonal antibody or fragment thereof is a MN-14 antibody or fragment thereof. Preferably, the MN-14 monoclonal antibody or fragment thereof comprises the complementarity-determining regions (CDRs) of a murine MN-14 monoclonal antibody, wherein the CDRs of the light chain variable region of said MN-14 antibody comprises CDR1 comprising the amino acid sequence KASQDVGTSVA; CDR2 comprising the amino acid sequence WTSTRHT; and CDR3 comprising the amino acid sequence QQYSLYRS; and the CDRs of the heavy chain variable region of said Class III anti-CEA antibody comprises CDR1 comprising TYWMS; CDR2 comprising EIENDSSTINYAPSLKD; and CDR3 comprising LYFGFPWFAY. Also preferred, the MN-14 monoclonal antibody reacts with CEA and is unreactive with normal cross-reactive antigen (NCA) and meconium antigen (MA). However, antibodies against these cross-reactive determinants may be used in combination therapy with CEA-specific antibodies, such as combined with the MN-14 monoclonal antibody.

In another embodiment of the present invention, the MN-14 monoclonal antibody or fragment thereof is a humanized or fully human MN-14 antibody or fragment thereof. The framework regions (FRs) of the light and heavy chain variable regions of the humanized MN-14 antibody or fragment thereof preferably comprise at least one amino acid substituted from the corresponding FRs of a murine MN-14 monoclonal antibody. Still preferred, the humanized MN-14 antibody or fragment thereof comprises at least one amino acid from the corresponding FR of the murine MN-14 antibody selected from the group consisting of amino acid residue 24, 28, 30, 48, 49, 74 and 94 of the murine heavy chain variable region (KLHuVhAIGA) of Figure 14A-C as noted above or of Figure 15B or Figure 16B. The amino acid sequence of a preferred humanized heavy chain variable region is also set forth in Hansen et al., U.S. Patent No. 5,874,540. Also preferred, the humanized heavy chain variable region comprises the amino acid sequence set forth in Figures 14A-C, designated as KLHuVhAIG and KLHuVhAIGAY. In another embodiment, the humanized MN-14 antibody or fragment thereof comprises at least one amino acid from the corresponding FR of the murine Man- 14 light chain variable region. Most preferably, the humanized MN-14 antibody or fragment thereof comprises the light chain variable region of Figure 13A or Figure 15A or Figure 16A. Another embodiment of the present invention is a composition comprising a chimeric MN-14 monoclonal antibody or fragment thereof and at least one therapeutic agent, which are not conjugated to each other, and thus are present in the composition as unconjugated forms of each of the components. Preferably, the chimeric MN-14 antibody or fragment thereof comprises the CDRs of the murine MN14 light chain variable region set forth in Figure 13A or Figure 15A or Figure 16A and the CDRs of the murine MN14 heavy chain variable region as set forth in Figures 14A-C or Figure 15B or Figure 16B.

Also described herein is a composition comprising a naked murine, humanized, chimeric or human Class III anti- CEA antibody or fragment thereof and a therapeutic agent, and a second naked or conjugated Class III anti- CEA antibody or antibody fragment thereof, that is non-blocking, *i*.*e*., does not block binding of the first Class III anti-CEA antibody or fragment thereof, and formulated in a pharmaceutically acceptable vehicle. In other words, both Class III anti-CEA antibodies or fragments thereof are non-blocldng to each other, thus, allowing both antibodies or fragments thereof to bind to CEA (CD66e). Additionally, the Class III CEA antibody or antibody fragment of the present invention, as well as those for use in combination therapy, do not bind granulocytes or CD66a-d. Other Class III antibodies suitable for combination therapy as a naked antibody or as a component of an immunoconjugate, with the naked Class III anti-CEA antibody of antibody fragment of the present invention include the non-blocking antibodies or fragments thereof described in Kuroki et al., JP J. Can. Res., 78(4):386 (1987) and Hammarstrom (Cancer Res. 52(8):2329 (1992)), that also do not bind granulocytes or CD66a-d.

Additionally, other anti-CEA antibodies, such as Class II antibodies, can be used in combination with the Class III anti-CEA antibody of the present invention, in either a naked or conjugated form. Such Class II antibodies or antibody fragments that can be used for combination therapy are non-blocking and do not bind granulocytes or CD66a-d. For example, one or more chimeric or humanized Class II anti-CEA antibody or fragment thereof, such as MN-6 or NP-3, may be combined with a Class III anti-CEA antibody or fragment thereof of the present invention. These two antibodies do not react with CD66a-d or with granulocytes. A number of publications disclose MAbs that recognize CEA and different members of the CEA gene family, such as Thompson et al., J. Clin. Lab. Anal. 5:344 (1991); Kuroki et al., J. Biol. Chem. 266:11810 (1991); Nagel et al., Eur. J. Biochem. 214:27 (1993); Skubitz et al., J. Immunol. 155:5382 (1995); Skubitz et al., J. Leukoc. Biol. 60:106 (1996); and Chen et al., Proc. Natl. Acad Sci. 93:14851 (1996).

Moreover, the second antibody or antibody fragment is either unconjugated (naked) or conjugated to at least one therapeutic agent (immunoconjugate). Immunoconjugates can be prepared by indirectly conjugating a therapeutic agent to an antibody component. General techniques are described in Shih et al., Int. J. Cancer, 41:832 (1988); Shih et al., Int. J. Cancer, 46:1101 (1990); and Shih et al., U.S. Patent No. 5,057,313. The general method involves reacting an antibody component having an oxidized carbohydrate portion with a carrier polymer that has at least one free amine function and that is loaded with a plurality of drug, toxin, chelator, boron addends, or other therapeutic agent. This reaction results in an initial Schiffbase (imine) linkage, which can be stabilized by reduction to a secondary amine to form the final conjugate. Preferably, the anti-CEA antibody or fragment thereof in the composition for treatment is a MN-14 antibody or fragment thereof. More preferred, the MN-14 antibody or fragment thereof is humanized.

Also contemplated in the present invention is a composition comprising a naked humanized, chimeric, murine or human Class III anti- CEA antibody or fragment thereof and a therapeutic agent, and a second therapeutic agent such as a conjugated or unconjugated antibody or antibody fragment thereof that is not a Class II or Class III anti- CEA antibody. In one embodiment, the second therapeutic agent (or second antibody or fragment thereof) is unconjugated (naked) or conjugated to at least one therapeutic agent. Non Class II or Class III anti-CEA antibodies and fragments thereof that are suitable for combination therapy include, but are not limited to, carcinoma-associated antibodies and fragments thereof. Examples of carcinoma associated antibodies and antibody fragments bind EGP-1, EGP-2 (e.g., 17-1A), MUC-1, MUC-2, MUC-3, MUC-4, PAM-4, KC4, TAG-72, EGFR, HER2/neu, BrE3, I,e-Y, A3, A33, Ep-CAM, AFP, Tn, Thomson-Friedenreich antigens, tumor necrosis antigens, VEGF or other tumor angiogenesis antigens, Ga 733, or a combination thereof. As discussed *supra,* non-blocking Class II and Class III anti-CEA MAbs that do not bind CD66a-d or granulocytes may also be used in combination with Class III CEA antibodies. Other antibodies and antibody fragments suitable for combination therapy also include those targeted against oncogene markers or products, or antibodies against tumor-vasculature markers, such as the angiogenesis factor, Placental Growth Factor (P1GF), and antibodies against certain immune response modulators, such as antibodies to CD40.

### Methods

Also described in the present invention is a naked Class III anti-CEA MAb for use in methods for treating medullary thyroid carcinoma and non-medullary thyroid carcinomas. Non-medullary thyroid carcinomas include colorectal cancer and any other CEA expressing tumor, such as pancreatic cancer, breast cancer, hepatocellular carcinoma, ovarian cancer, certain kinds of lung, head-and-neck, endometrial, bladder, and liver cancers that express variable quantities of CEA. The CEA levels in these types of cancers are much lower than present in medullary thyroid carcinomas but all that is necessary is that the CEA levels be sufficiently high so that the Class III anti-CEA therapy provides an effective treatment. Normal colon mucosa has about 500 ng/gram but carcinomas expressing CEA at levels of 100 ng/gram of tissue are suitable for treatment with the methods described in the instant invention.

For example, contemplated herein is a method for treating medullary thyroid carcinoma or non-medullary thyroid carcinoma comprising administering to a subject, either concurrently or sequentially, a therapeutically effective amount of a Class III anti-CEA monoclonal antibody or fragment thereof and at least one therapeutic agent, and optionally formulated in a pharmaceutically acceptable vehicle. Preferably, the Class in anti-CEA monoclonal antibody or fragment thereof is chimeric, murine, humanized or human, wherein the chimeric, humanized, murine, or human Class III anti-CEA MAb retains substantially the Class III anti-CEA binding specificity of the murine MAb. More preferably, the Class III anti-CEA antibody is humanized, and most preferably, the humanized MN-14 monoclonal antibody, as described herein and in U.S. Patent 5,874,540. Preferably the therapeutic agent is a cytotoxic agent, more preferably an alkylating agent, and most preferably, dacarbazine (DTIC). Other classes of anti-cancer cytostatic and cytotoxic agents, such as CPT-11, can also be used in combinations with these antibodies. But in another embodiment, the therapeutic agent may also not be DTIC.

Also contemplated herein is a method for treating medullary thyroid carcinoma and non-medullary thyroid carcinoma comprising administering to a subject, either concurrently or sequentially, a therapeutically effective amount of a first Class III anti-CEA monoclonal antibody or fragment thereof and at least one therapeutic agent, and a naked or conjugated second humanized, chimeric, human or murine monoclonal antibody or fragment thereof, and optionally formulated in a pharmaceutically acceptable vehicle. Preferably, the first Class III anti-CEA MAb is a humanized MN-14 antibody or fragment thereof. In one embodiment, the second antibody or fragment thereof (i.e., the second therapeutic agent) is a carcinoma-associated antibody or fragment thereof selected from the group consisting of a monoclonal antibody or fragment thereof reactive with TAG-72, EGFR, HER2/*neu*, MUC1, MUC2, MUC3, MUC4, EGP-1, EGP-2, AFP, Tn, or another such tumor-associated antigen, as described above. In another embodiment, the second antibody or fragment thereof can be a different Class III anti-CEA antibody or fragment thereof that is non-blocking and does not bind granulocytes or CD66a-d.

In another embodiment, the second anti-CEA antibody is a Class II antibody or fragment thereof, such as those described in Hammarstrom and Kuroki, provided that they do not bind granulocytes or CD66a-d. The antibodies and fragments thereof may be administered either concurrently or sequentially with each other or the therapeutic agent. In one embodiment, the second antibody or fragment thereof is either naked or conjugated to a therapeutic agent.

Accordingly, the present invention contemplates the administration of naked murine, humanized, chimeric and human Class III anti-CEA antibodies and fragments thereof sequentially or concurrently with a therapeutic agent, or administered as a multimodal therapy. Multimodal therapies of the present invention include immunotherapy with a Class III anti-CEA antibody or fragment thereof, and a therapeutic agent, supplemented with administration of an unconjugated or conjugated antibody, unconjugated or conjugated fusion protein, or fragment thereof. For example, an unconjugated humanized, chimeric, murine or human MN-14 MAb or fragment thereof may be combined with another naked humanized, murine, chimeric or human Class III anti-CEA antibody (such as an antibody against a different epitope on CEA and also does not bind granulocytes or CD66a-d), or a humanized, chimeric, murine or human Class III anti-CEA antibody immunoconjugate conjugated to a radioisotope, chemotherapeutic agent, cytokine, enzyme, enzyme-inhibitor, hormone or hormone antagonist, metal, toxin, or a combination thereof. A naked Class III anti-CEA antibody or fragment thereof may also be combined with a conjugated or unconjugated fusion protein of a murine, humanized, chimeric or human Class III anti-CEA antibody. However, the Class III anti-CEA antibodies for combination therapy are non-blocking to each other and unable to bind granulocytes or CD66a-d. Preferably, the naked Class III anti-CEA antibody is administered sequentially or concurrently with the second naked or conjugated antibody, fusion protein, or fragment thereof. Also preferred, one of the antibodies or antibody fragments for use in combination therapy is a naked humanized MN-14 antibody or fragment thereof. Additionally, the second antibody used as a naked or conjugated antibody, fusion protein, or fragment thereof, may be a human, humanized, chimeric or murine Class II CEA antibody or fragment thereof that is non-blocking and does not bind granulocytes or CD66a-d.

In the methods described herein, subjects receive at least one naked Class III anti-CEA antibody or fragment thereof, administered before, after or in conjunction with a therapeutic agent. Preferably, the therapeutic agent is a drug used in standard cancer chemotherapy, such as taxane or platinum drugs in ovarian cancer, fluorouracil, CPT-11, and oxaloplatin drugs in colorectal cancer, gemcitabine in pancreatic and other cancers, or taxane derivatives in breast cancers. COX-2 inhibitors represent still another class of agents that show activity in combination with typical cytotoxic agents in cancer chemotherapy, and can be used in this invention in the same way, but combined in addition with CEA antibodies alone and in combination with other cancer-associated antibodies. Optionally, these drugs can be used in combination with radiolabeled antibodies, either CEA antibody conjugates or radioconjugates with other carcinoma-associated antibodies, of the kinds described above. Also preferred, the Class III anti-CEA antibody or fragment thereof is a MN-14 antibody or fragment thereof. Still preferred, the MN-14 antibody or fragment thereof is humanized.

In a preferred embodiment, a naked Class III anti-CEA antibody or fragment thereof is administered sequentially (either prior to or after) or concurrently with dacarbazine (DTIC), doxorubin, cyclophosphamide or vincristine, or any combination of these. For example, DTIC and cylcophosphamide may be administered sequentially or concurrently with a naked Class III anti-CEA antibody or fragment thereof. Preferably, the anti-CEA antibody or fragment thereof is a humanized MN-14 antibody or fragment thereof. Similarly, 5-fluorouracil in combination with folinic acid, alone or in combination irinotecan (CPT-11), is a regimen used to treat colorectal cancer. Other suitable combination chemotherapeutic regimens are well known, such as with oxaliplatin alone, or in combination with these other drugs; to those of skill in the art. Accordingly, combination therapy with any of these chemotherapeutic agents and a naked Class III anti-CEA antibody or fragment thereof can be used to treat MTC or non-MTC, depending on the regimen used. In medullary thyroid carcinoma, still other chemotherapeutic agents may be preferred, such as one of the alkylating agents (e.g., DTIC), as well as gemcitabine and other more recent classes of cytotoxic drugs. The chemotherapeutic drugs and a naked Class III anti-CEA antibody or fragment thereof, can be administered in any order, or together. In other words, the antibody and therapeutic agent may be administered concurrently or sequentially. In a preferred multimodal therapy, both chemotherapeutic drugs and naked Class III anti-CEA antibodies or fragments thereof are administered before, after, or co-administered with a conjugated or unconjugated anti-CEA antibody, fusion protein, or fragment thereof, according to the present invention. Preferably, the Class III anti-CEA antibody or fragment thereof is a humanized MN-14 antibody or fragment thereof.

A preferred treatment schedule of multimodal treatment is administering both hMN-14 and DTIC for 3 days, and administering only hMN-14 on days 7, 14, 21 and then every 21 days for a treatment duration of 12 months. The doses of hMN-14 are 0.5 -15 mg/kg body weight per infusion, more preferably 2-8, and still more preferably 3-5 mg/kg per infusion, and the doses of DTIC are as currently applied at the preferred dose clinically, but could also be given at two-thirds or less of the maximum preferred dose in use, thereby decreasing drug-related adverse events. Repeated drug cycles can be given, such as every 3-6 months, with continuation of the naked antibody therapy, or with different schedules of radiolabeled antibody, drug-conjugated antibody, and inclusion of certain cytokines, such as G-CSF and/or GM-CSF, each dose adjusted so that toxicity to the patient is not enhanced by the therapeutic combination. The application of a cytokine growth factor, such as G-CSF, may enable even higher doses of myelosuppressive agents, such as radiolabeled antibody or cytotoxic drugs, to be administered, and these schedules and doses will be adjusted for the patients individually, depending on their disease status and prior therapy, all influence bone marrow status and tolerability to additional cytotoxic therapies. In a preferred embodiment, the MN-14 antibody or fragment thereof is administered in a dosage of 100-600 milligrams protein per dose per injection. Still preferred, the MN-14 antibody or fragment thereof is administered in a dosage of 300 milligrams of protein per dose per injection,with repeated doses preferred.

### Therapeutic Agents

The therapeutic agents recited here are those agents that also are useful for administration separately with a naked antibody, as described herein. Suitable therapeutic agents can be selected from the group consisting of a cytotoxic agent, a radionuclide, an immunomodulator, a photoactive therapeutic agent (such as a chromagen or dye), an immunoconjugate, another naked antibody, a hormone, or a combination thereof. Therapeutic agents include, for example, chemotherapeutic drugs such as vinca alkaloids and other alkaloids, anthracyclines, epidophyllotoxins, taxanes, antimetabolites, alkylating agents, antibiotics, COX-2 inhibitors, antimitotics, antiangiogenic and apoptotoic agents, particularly doxorubicin, methotrexate, taxol, CPT-11, camptothecans, and others from these and other classes of anticancer agents, and the like. Other useful cancer chemotherapeutic drugs for the preparation of immunoconjugates and antibody fusion proteins include nitrogen mustards, alkyl sulfonates, nitrosoureas, triazenes, folic acid analogs, COX-2 inhibitors, pyrimidine analogs, purine analogs, platinum coordination complexes, hormones, toxins (e.g., RNAse, Psudomonas exotoxin), and the like. Preferred therapeutic agents include DTIC, CPT-11, 5-fluorouracil, taxol, oxaliplatin, doxorubicin, cyclophosphamide and vincristine, or a combination thereof, depending on the malignancy to be treated. Still preferred, doxorubicin, DTIC, cyclophosphamide and vincristine are used singly or in combination in the compositions and methods of the instant invention. Accordingly, the compositions and methods described herein may comprise more than one therapeutic agent. Suitable chemotherapeutic agents are descnbed in REMINGTON'S PHARMACEUTICAL SCIENCES, 19th Ed. (Mack Publishing Co. 1995), and in GOODMAN AND GILMAN'S THE PHARMACOLOGICAL BASIS OF THERAPEUTICS, 7th Ed. (MacMillan Publishing Co. 1985), as well as revised editions of these publications. Other suitable chemotherapeutic agents, such as experimental drugs, are known to those of skill in the art.

A toxin, such as *Pseudomonas* exotoxin, may also be administered with a naked Class III anti-CEA antibody or fragment thereof. Preferably, the Class III anti-CEA antibody or fragment thereof is a humanized MN-14 antibody or fragment thereof. Other suitable microbial, plant or animal toxins to be administered unconjugated to, but before, after, or simultaneously with the naked Class III anti-CEA antibody or fragment thereof include ricin, abrin, ribonuclease (RNase), DNase I, *Staphylococcal* enterotoxin-A, pokeweed antiviral protein, gelonin, diphtherin toxin, *Pseudomonas* exotoxin, and *Pseudomonas* endotoxin. See, for example, Pastan et al., Cell 47:641 (1986), and Goldenberg, CA - A Cancer Journal for Clinicians 44:43 (1994). Additional toxins suitable for use in the present invention are known to those of skill in the art and are disclosed in U.S. Patent No. 6,077,499. These can be derived, for example, from animal, plant and microbial sources, or chemically or recombinantly engineered. The toxin can be a plant, microbial, or animal toxin, or a synthetic variation thereof.
An immunomodulator, such as a cytokine may also be administered unconjugated to the chimeric, murine, humanized or human Class III anti-CEA antibody or fragment thereof of the present invention. As used herein, the term "immunomodulator" includes cytokines, stem cell growth factors, lymphotoxins, such as tumor necrosis factor (TNF), and hematopoietic factors, such as interleukins (e.g., interleukin-1 (IL-1), IL-2, IL-3, IL-6, IL-10, IL-12 and IL-18), colony stimulating factors (e.g., granulocyte-colony stimulating factor (G-CSF) and granulocyte macrophage-colony stimulating factor (GM-CSF)), interferons (*e*.*g*., interferons-α, -β and -γ), the stem cell growth factor designated "S1 factor," erythropoietin, and thrombopoietin. Examples of suitable immunomodulator moieties include IL-2, IL-6, IL-10, IL-12, IL-18, interferon-γ, TNF-α, and the like. Therefore, subjects can receive a naked Class III anti-CEA antibody or fragment thereof and a separately administered cytokine, which can be administered before, concurrently or after administration of the naked Class III anti-CEA antibody or fragment thereof. Since some antigens may also be immunomodulators, CD40 antigen, for example, may also be administered in combination with a naked Class III anti-CEA antibody or fragment thereof either together, before or after the naked antibody or antibody combinations are administered. Additionally, radionuclides suitable for treating a diseased tissue include, but are not limited to, ³²P, ³³P, ⁴⁷Sc, ⁵⁹Fe, ⁶⁴Cu, ⁶⁷Cu, ⁷⁵Se, ⁷⁷As, ⁸⁹Sr, ⁹⁰Y, ⁹⁹Mo, ¹⁰⁵Rh, ¹⁰⁹Pd, ¹¹¹Ag, ¹²⁵I, ¹³¹I, ¹⁴²Pr, ¹⁴³Pr, ¹⁴⁹Pm, ¹⁵³Sm, ¹⁶¹Tb, ¹⁶⁶Ho, ¹⁶⁹Er, ¹⁷⁷Lu, ¹⁸⁶Re, ¹⁸⁸Re, ¹⁸⁹Re, ¹⁹⁴Ir, ¹⁹⁸Au, ¹⁹⁹Au, ²¹¹Pb, ²¹²Pb, and ²¹³Bi, ⁵⁸Co, ⁶⁷Ga, ^{80m}Br, ^{99m}Tc, ^{103m}Rh, ¹⁰⁹Pt, ¹¹¹In, ¹¹⁹Sb, ¹⁶¹Ho, ^{189m}Os, ¹⁹²Ir, ¹⁵²Dy, ²¹¹At, ²¹²Bi, ²²³Ra, ²¹⁹Rn, ²¹⁵Po, ²¹¹Bi, ²²⁵Ac, ²²¹Fr, ²¹⁷At, ²¹³Bi, ⁸⁸Y and ²⁵⁵Fm. Preferred radionuclides are ¹²⁵I, ¹³¹I, ⁹⁰Y, ¹⁷⁷Lu, and ²²⁵Ac. Also preferred, the radionuclide has an energy between 20 and 10,000 keV.

### Pharmaceutically Acceptable Vehicles

The naked murine, humanized, chimeric and human Class III anti-CEA MAbs to be delivered to a subject can comprise one or more pharmaceutically acceptable vehicles, one or more additional ingredients, or some combination of these.

The unconjugated Class III anti-CEA antibodies and fragments thereof of the present invention can be formulated according to known methods to prepare pharmaceutically useful compositions. Preferably, the Class III anti-CEA antibody or fragment thereof is a MN-14 antibody or fragment thereof. Sterile phosphate-buffered saline is one example of a pharmaceutically acceptable vehicle. Other acceptable vehicles are well-known to those in the art. See, for example, Ansel et al., PHARMACEUTICAL DOSAGE FORMS AND DRUG DELIVERY SYSTEMS, 5th Edition (Lea & Febiger 1990), and Gennaro (ed.), REMINGTON'S PHARMACEUTICAL SCIENCES, 18th Edition (Mack Publishing Company 1990), and revised editions thereof.

The unconjugated Class III anti-CEA antibody or fragment thereof of the present invention can be formulated for intravenous administration via, for example, bolus injection or continuous infusion.Preferably, the Class III anti-CEA antibody or fragments is a MN-14 antibody or fragment thereof. Formulations for injection can be presented in unit dosage form, e.g., in ampules or in multi-dose containers, with an added preservative. The compositions can take such forms as suspensions, solutions or emulsions in oily or aqueous vehicles, and can contain formulatory agents such as suspending, stabilizing and/or dispersing agents. Alternatively, the active ingredient can be in powder form for constitution with a suitable vehicle, e.g., sterile pyrogen-free water, before use.

Additional pharmaceutical methods may be employed to control the duration of action of the agent and naked antibody or fragment thereof. Control release preparations can be prepared through the use of polymers to complex or adsorb the naked antibody. For example, biocompatible polymers include matrices of poly(ethylene-co-vinyl acetate) and matrices of a polyanhydride copolymer of a stearic acid dimer and sebacic acid. Sherwood et al., Bio/Technology 10: 1446 (1992). The rate of release of an antibody or fragment thereof from such a matrix depends upon the molecular weight of the immunoconjugate or antibody, the amount of antibody within the matrix, and the size of dispersed particles. Saltzman et al., Biophys. J. 55: 163 (1989); Sherwood *et al., supra.* Other solid dosage forms are described in Ansel et al., PHARMACEUTICAL DOSAGE FORMS AND DRUG DELIVERY SYSTEMS, 5th Edition (Lea & Febiger 1990), and Gennaro (ed.), REMINGTON'S PHARMACEUTICAL SCIENCES, 18th Edition (Mack Publishing Company 1990), and revised editions thereof.

The unconjugated Class III anti-CEA antibody or fragment thereof may also be administered to a mammal subcutaneously or even by other parenteral routes. Moreover, the administration may be by continuous infusion or by single or multiple boluses. In general, the dosage of an administered naked antibody or fragment thereof for humans will vary depending upon such factors as the patient's age, weight, height, sex, general medical condition and previous medical history. Typically, it is desirable to provide the recipient with a dosage of naked antibody or fragment thereof that is in the range of from about 0.5mg/kg to 20 mg/kg as a single intravenous infusion, although a lower or higher dosage also may be administered as circumstances dictate. This dosage may be repeated as needed, for example, once per month for 4-10 months, preferably once per every other week for 16weeks, and more preferably, once per week for 8 weeks. It may also be given less frequently, such as every other week for several months or given more frequently and/or over a longer duration. The dosage may be given through various parenteral routes, with appropriate adjustment of the dose and schedule.

For purposes of therapy, the Class III anti-CEA antibody or fragment thereof is administered to a mammal in a therapeutically effective amount to reduce the size of the tumor as compared to untreated controls. Preferably, the Class III anti-CEA antibody or fragment thereof is a humanized MN-14 antibody or fragment thereof. A suitable subject for the present invention is usually a human, although a non-human mammal or animal subject is also contemplated. An antibody preparation is said to be administered in a "therapeutically effective amount" if the amount administered is physiologically significant. An agent is physiologically significant if its presence results in a detectable change in the physiology of a recipient mammal. In particular, an antibody preparation of the present invention is physiologically significant if its presence invokes an antitumor response. A physiologically significant effect could also be the evocation of a humoral and/or cellular immune response in the recipient mammal.

### Example 1: Materials and Methods

### Monoclonal Antibodies and Cell Lines

TT, a human medullary thyroid cell line, was purchased from the American Type Culture Collection. The cells were grown as monolayers in DMEM (Life Technologies, Gaithersburg, MD) supplemented with 10% fetal bovine serum, penicillin (100 U/ml), streptomycin (100 µg/ml), and L-glutamine (2 mM). The cells were routinely passaged after detachment with trypsin, 0.2% EDTA.

MN-14 is a Class III anti-CEA MAb, reacting with CEA and unreactive with the normal cross reactive antigen, NCA, and meconium antigen (Hansen et al., Cancer, 71:3478(1993)). The construction and characterization of the humanized forms of MN-14 and LL2, the anti-CD22 MAb used here as a negative control, have been previously described. (Sharkey et al., Cancer Res., 55:5935s (1995); Leung et al., Mol. Immunol., 32:1416 (1995)). P3x63Ag8 (MOPC-21) is an irrelevant mouse myeloma IgG₁ obtained from the American Type Culture collection (Rockville, MD). The antibodies were purified by protein A chromatography.

### In vivo Studies

Tumors were propagated in female *nu*/*nu* mice (Taconic Farms, Germantown, NY) at 6-8 weeks of age by s.c. injection of 2 x 10⁸ washed TT cells, which had been propagated in tissue culture. Antibodies were injected i.v., via the lateral tail vein, into the tumor-bearing animals. Details on the quantities of antibodies injected and the time of administration are indicated in the Results section for each study. Results are given as tumor volumes of individual animals as well as the means ± SE. Tumor size was monitored by weekly measurements of the length, width, and depth of the tumor using a caliper. Tumor volume was calculated as the product of the three measurements. Statistical comparisons were made using the Student's T-test to compare tumor volumes and area under the growth curves.

### Example 2. Combination therapy of naked hMN-14 and DTIC delivered 2 days after injection of TT (human medullary thyroid) tumor cells.

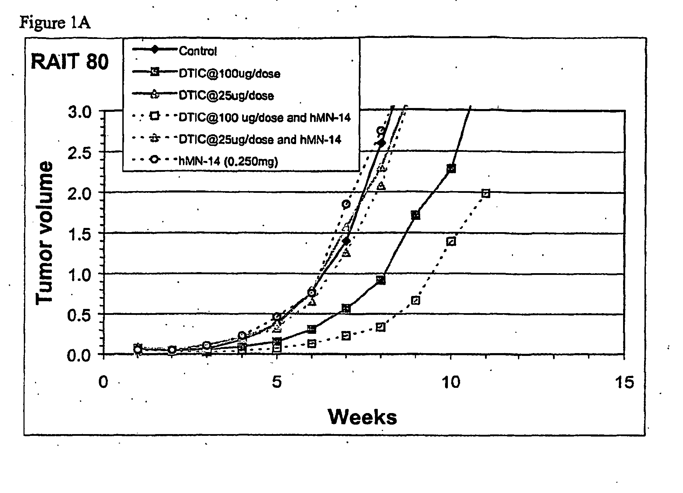

In a previous study, naked hMN-14 and dacarbazine (DTIC) were given in combination to TT 2 days after tumor implantation, using 100 µg and 25 µg doses of DTIC (days 2, 3, and 4) and 250 µg doses of hMN-14 given on day 2, then weekly. The 100 µg DTIC dose combined with hMN-14 was more effective than either treatment alone (Figure 1A). However, the 100 µg DTIC dose yielded too strong a response, while the 25 µg dose was not effective. Surprisingly, the effects of MN-14 alone and DTIC alone were not additive. In other words, given the results of treatment with 250 µg hMN-14 alone and 100 µg DTIC alone, one would not predict that the combination of 250 µg hMN-14 and 100 µg DTIC would have such a pronounced effect. See Figure 1A.

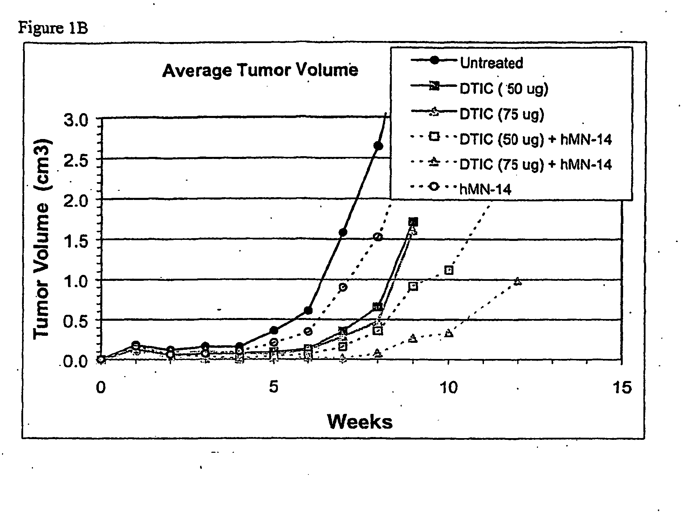

In this study, treatment began 2 days after TT cell injection, as in the previous study. hMN-14 was given at 100 µg/dose on days 2, 3, 4, 5, 7, 8, 9, 10, 11, 15 and 22, then every 7 days until the animal died, the tumor attained a volume of 2.0 cm³ or the study terminated for humane reasons. Doses of DTIC were 50 and 75 µg per dose, which is between the doses given in the previous study. TT cells were injected subcutaneously in 60 nude mice. The day of injection was Monday, day 0. See Figure 1B.

Results demonstrate that significant delays in tumor growth were caused by either MAb therapy alone or chemotherapy alone (Figure 1B). The 75 µg dose of DTIC in combination with this schedule of hMN-14 antibody was significantly more effective than either treatment alone (p< 0.02). Unexpectedly, the results of combined DTIC and MAb therapy were not additive. At 7 weeks, 8/10 mice in the 75 µg DTIC and MAb group had no palpable tumor, compared to 1/10 mice in the 75 µg DTIC only group and 0/10 mice in the untreated and MAb group.

Mean tumor volumes at 7 weeks were 0.018 ± 0.039 cm³ (75 µg DTIC plus MN-14), 0.284 + 0.197 cm³ (75 µg DTIC only), 0.899 ± 0.545 cm³ (hMN-14 only) and 1.578 ± 0.959 cm³ (untreated). Combined thereapy of the naked anti-CEA antibody with DTIC augments the anti-tumor effects of antibody or chemotherapy alone, without increased toxicity. The superiority of the combined modality treatment was surprising.

Dosing Summary: (1) hMN-14 was given daily (i.p.), except Sundays, at 100 µg/dose/mouse on days 2 through 11. The antibody treatment was initiated on the same day as DTIC treatment. (2) DTIC was given on days 2, 3, and 4 at 50 and 75 µg/dose, which corresponded to 5% and 7.5% of the MTD. Only one course of DTIC was given.

Groups: 6 groups of mice, each group containing 10 mice.

Group 1: Untreated.

Group 2. DTIC at 50 µg/dose, days 2, 3, and 4 (Wednesday, Thursday, and Friday).

Group 3. DTIC at 75 µg/dose, days 2, 3, and 4.

Group 4. DTIC at 50 µg/dose, days 2, 3, and 4, plus hMN-14 (100 µg/dose) day 2, 3, 4, 5, 7, 8, 9, 10, 11, 15 and 22, then every 7 dyas until the animal died, the tumor attained a volume of 2.0 cm³, or the study terminated.

Group 5. DTIC at 25 µg/dose, days 2, 3, and 4, plus hMN-14 (100 µg/dose) day 2, 3, 4, 5, 7, 8, 9, 10, 11, 15 and 22, then every 7 dyas until the animal died, the tumor attained a volume of 2.0 cm³, or the study terminated.

Group 6. hMN-14 (confirm 100 µg/dose), days 2, 3, 4, 5, 7, 8, 9,10, 11, 15 and 22, then every 7 days until the animal died, the tumor attained a volume of 2.0 cm³, or the study terminated.

Animals were monitored for survival. Tumor and body weight were measured weekly.

Protocol: On day 2, 200mg/vial DTIC was reconstituted with 19.7 ml sterile water for injection. The resulting solution contained 10 mg/ml of dacarbazine with a pH range of 3.0-4.0. The solution was used as needed for the dilutions described below and the remainder was frozen in 1 ml aliquots for subsequent use.

Groups 2 and 4: 5 ml of 0.5 mg/ml solution was prepared. 100 µl of 0.5 mg/ml/mouse was injected i.v.

Groups 3 and 5: 5 ml of 0.75 mg/ml solution was prepared. 100 µl of 0.75 mg/ml/mouse was injected i.v.

Quantity of hMN-14 was estimated. 100 µl of 1 mg/ml hMN-14 was injected i.p. in mice in Groups 4, 5 and 6.

### Example 3. Radioimmunotherapy studies in a human MTC xenograft model.

Applicants developed a model for experimental radioimmunotherapy of MTC with radiolabeled anti-CEA MAbs using human MTC xenografts of the CEA-and calcitonin producing human MTC cell line designated TT ([Stein, 1999 #82], see Appendix). MTC tumors were established in nude mice by a s.c. inoculation of 2x14⁸ cells and allowed to grow for 2-5 weeks before injection of MAbs. Biodistribution and RAIT studies were then carried out with MN-14, which was shown by flow cytometry to react with TT cells. Both Ag8 and Mu-9 were used as negative control MAbs in these studies. Preliminary studies using smaller tumors of ~ 0.08 g showed that 7 days after the injection of ¹³¹I-MN-14, the percent of injected dose per gram of tumor (%ID/g) was 68.9% compared with only 12.6% ID/g for the co-injected ¹²⁵I-Ag8 control. Using larger tumors, (grown for five weeks in nude mice; mean tumor weight = 0.404 g), the % ID/g of tumor observed at seven days post injection of ¹²⁵I-MN-14 was 12.4%. However, the % ID/g of the co-injected ⁸⁸Y-MN-14 was 50.5%, or 4.1-fold higher than ¹²⁵I-MN-14. The tumor-to-blood, lungs, liver, spleen, and kidneys were also higher with ⁸⁸Y-MN-14 than with ¹²⁵I-MN-14, while the tumor-to-bone ratios were equal with both agents. When ¹²⁵I-MN-14 and ⁸⁸Y-MN-14 biodistribution data were used to predict the tumor dosimetry with ¹³¹I-MN-14 and ⁹⁰Y-MN-14, respectively, the radiation absorbed dose delivered at the MTD of ⁹⁰Y-MN-14 (115 µCi) was 1.75-fold higher than that delivered at the MTD of ¹³¹I-MN-14 (275 µCi) (4900 cGy vs. 2800 cGy).

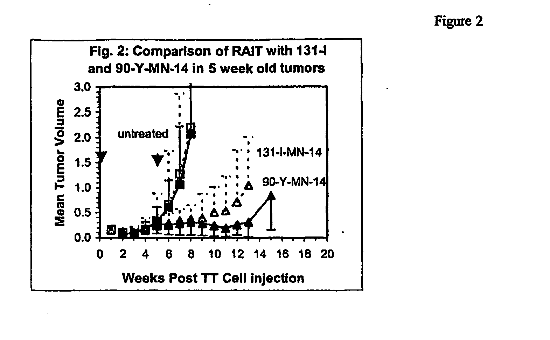

Therapy studies in this model confirmed that ⁹⁰Y-MN-14 is a better therapeutic agent than ¹³¹I-MN-14. In 5-week-old tumors, a 5-week complete inhibition of tumor growth was seen at the MTD of ⁹⁰Y-MN14 compared to only a tumor growth delay with ¹³¹I-MN14 (Figure 2). Moreover, when smaller 2-week old tumors were treated, an average of 60% tumor volume reduction, with some complete tumor regressions, was seen at the MTD of ⁹⁰Y-MN14. These anti-tumor effects were very significant compared with the relatively rapid tumor growth in untreated animals or those treated at the MTD of control MAbs. Thus, our preclinical studies demonstrated that this animal model is exquisitely suitable for experimental RAIT with anti-CEA MAbs.

The longer path length and higher energy of ⁹⁰Y compared with ¹³¹I, in addition to the fact that ⁹⁰Y is retained longer by target cells led to delivery of an increased radiation dose to tumor and thus more effective therapy at equitoxic doses. If our results with residualizing ¹³¹I (refs) can be generalized to MN-14 in MTC, we would expect that residualizing ¹³¹I would be at least equally effective to ⁹⁰Y in tumors of the size studied here, and most likely superior in the setting of micrometastatic disease or as adjuvant therapy following surgery.

### Example 4: Chemotherapy.

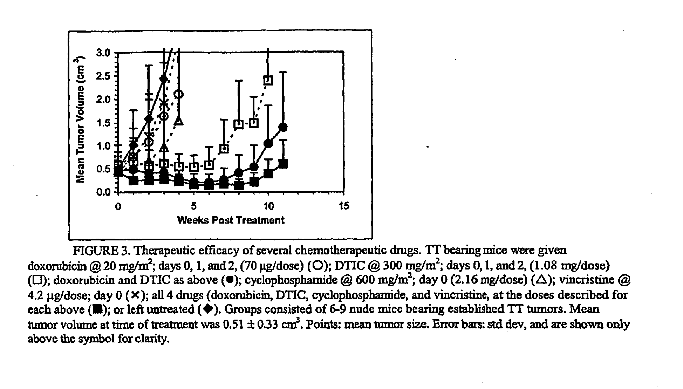

Four drugs, doxorubicin, DTIC (dacarbazine), cyclophosphamide, and vincristine, were evaluated, singly and in combination, for their effect on the growth of TT MTC xenografts in nude mice. Doses were selected based on the doses of each drug given clinically to humans on a mg/m² basis. Animals were monitored for survival, and tumor volumes and body weights were measured weekly. Figure 3 shows the tumor growth curve for animals in this study. Given individually, doxorubicin, DTIC and cyclophosphamide, but not vincristine, yielded significant growth inhibition, although the growth delay caused by DTIC was markedly longer than that of the other drugs. Approximate mean time to doubling for each group was: untreated, 1 week; doxorubicin, 2.5 weeks; DTIC, 7.5 weeks; cyclophosphamide, 3 weeks; and vincristine, 1.5 weeks. Combining doxorubicin and DTIC improved the efficacy compared to either drug alone, increasing the mean time to doubling to 10 weeks. However, the increased efficacy of doxorubicin and DTIC combination did not reach the 95% confidence level in comparison to DTIC alone. The P values for AUC comparisons were as follows: P<0.01 for doxorubicin + DTIC versus doxorubicin, and P<0.1 for doxorubicin + DTIC versus DTIC. The 4-drug regimen extended the mean time to doubling to 12 weeks; P<0.01 for comparisons to both doxorubicin and DTIC.

Log rank analysis of survival data for the individual drugs versus the untreated group indicated a significant difference only for DTIC and cyclophosphamide. Mean survival time for the untreated control group was 4 weeks compared to 11 weeks and 8 weeks for DTIC and cyclophosphamide treatment groups, respectively, and greater than 12 weeks for the drug combinations. Toxicity, as measured by body weight loss, was within the acceptable range for all study groups. Maximum weight loss was observed 1 week after treatment in the mice treated with all 4 drugs, ranging from 3-12% loss of body weight.

### Example 5. Combining Radioimmunotherapy and Chemotherapy for Treatment of MTC

### RAIT Plus 4-drug Combination.

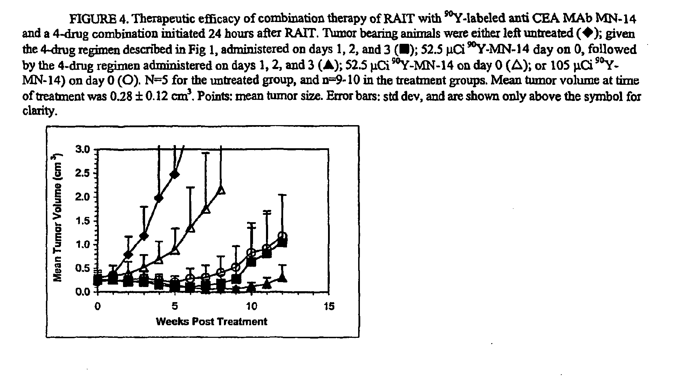

The effect of combining RAIT with ⁹⁰Y-anti CEA MAb MN-14 and the 4-drug combination was evaluated by comparing the growth of TT in untreated mice to those treated with the 4-drug regimen described above (doxorubicin, DTIC, cyclophosphamide, and vincristine), 100% of the maximum tolerated dose (MTD) of RAIT (105 µCi), 50% of the MTD of RAIT, and 50% of the MTD of RAIT combined with the 4 drugs. Figure 4 shows the growth curves of TT tumors in mice given the various treatment regimens. All four of the treatment groups yielded significant improvement in efficacy compared to the untreated animals. Whereas the approximate mean time to doubling in the untreated animals was 1.5 weeks, chemotherapy with the 4 drugs extended the mean doubling time to 10 weeks and RAIT alone yielded 4-week and 8-week doubling times at 50% and 100% of the MTD, respectively. As expected, both the 100% RAIT group and the 4-drug therapy regimen were significantly better than the 50% RAIT group. Most importantly, combining 50% RAIT and the 4-drug regimen yielded improved results, compared to either therapy alone, further extending the mean doubling time to approximately 12.5 weeks. For the comparison of the combined treatment to the 4-drug regimen, P<0.02, and for the comparison to 100% RAIT, P<0.01.

Mean weight loss 1 week post treatment (nadir) was 9% for the 100% RAIT and the 4-drug regimens, but 15% for combined 50% RAIT plus 4-drug treatment. In addition, in the combined therapy group, one animal died three weeks post treatment and a second animal had a weight loss greater than 20%. Thus, this treatment exceeded the maximum tolerated dose.

### RAIT Plus Chemotherapy with 2-drug regimens.

The effect of combining RAIT with ⁹⁰Y-anti CEA MAb MN-14 and chemotherapy with a 2-drug combination, consisting of doxorubicin and DTIC, was also evaluated in this MTC xenograft model. Approximate doubling times for the groups were: untreated, 1.5 weeks; doxorubicin plus DTIC, 8 weeks; the MTD of RAIT, 10 weeks; and the MTD of RAIT combined with 25-75% of the 2-drug regimen, greater than 12 weeks. Thus, RAIT alone was more effective than the 2-drug regimen and, most significantly, combining RAIT and the 2-drug regimen yielded improved results compared to either therapy alone. For the comparison of the combined treatment to the 2-drug regimen, P<0.005, for the comparison to RAIT alone, P<0.02.

Mean weight loss 1-2 weeks post treatment (nadir) was 2-8% for all groups, except the 100% RAIT plus 75% 2-drug chemotherapy group, where a 13% loss was observed at 2 weeks. In addition, in this combined therapy group, two animals died 3-4 weeks post treatment and one experienced a weight loss greater than 20%. Thus, addition of the 75% dose level of doxorubicin and DTIC to 100% RAIT treatment exceeded the MTD, whereas 50% of this 2-drug combination can be tolerated in combination with 100% RAIT.

### RAIT plus Doxorubicin.

Because previous publications have reported the combination of RAIT with doxorubicin in this model (Stein et al., Clin Cancer Res., 5:3199s (1999); Behr et al., Cancer Res. 57:5309 (1997)), a direct comparison was made to the RAIT plus doxorubicin regimen. A direct comparison was also made to RAIT plus the 4-drug regimen. All treatments yielded significant efficacy compared to the untreated animals. The mean doubling time for the RAIT plus doxorubicin group was 12 weeks. In this study combining the full MTD of RAIT with either 50% of doxorubicin and DTIC or the 4-drug regimen extended the mean doubting time to greater than 15 weeks, with no statistically significant difference between these two groups. A substantial number of objective responses were observed in these studies. Following treatment with RAIT plus doxorubicin there were 3 complete responses, 2 partial responses, and 5 animals with stable disease for at least 4 weeks, out of a total of 10 mice. The RAIT plus 2-drug protocol increased the objective responses to 10 complete responses and 2 partial responses of 12 animals, and the RAIT plus 4-drug treatment protocol led to 7 complete responses and 2 partial responses out of 9 mice.

### RAIT plus DTIC.

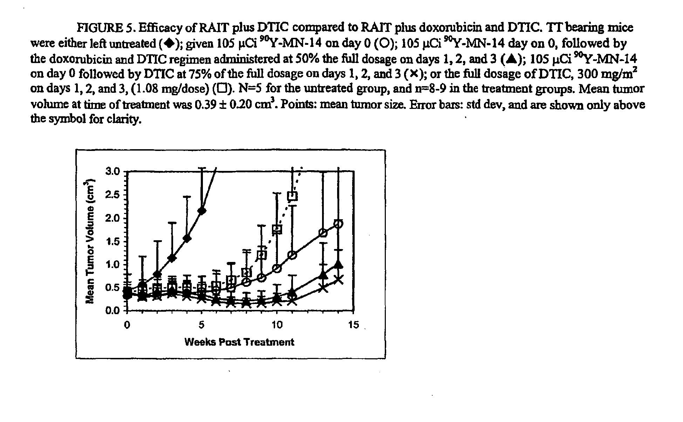

Because DTIC was the most effective chemotherapeutic agent when administered alone, the efficacy of RAIT plus DTIC was evaluated in comparison to that of RAIT plus doxorubicin and DTIC. Omitting doxorubicin from the treatment protocol will be important for clinical application in order to avoid the added toxicity of this drug, especially the known cardiac toxicity. As shown in Figure 5, the two study groups which received the chemotherapy in combination with RAIT, either doxorubicin and DTIC or DTIC only, are approximately equal to each other, and both are more effective than the single modality treatments. P values for AUC comparisons were as follows: P<0.01 for RAIT + DTIC versus DTIC, and P<0.05 for RAIT + DTIC versus RAIT. The mean doubling time for the RAIT plus DTIC, and RAIT plus doxorubicin and DTIC groups were 15.5 weeks and 14 weeks, respectively, compared to 7.5 weeks and 9 weeks for DTIC and RAIT alone, respectively. Thus, the combined modality treatment of RAIT plus DTIC extended the mean time to doubling by 100% over the DTIC chemotherapy. No significant difference was observed by either AUC or log rank analyses between the RAIT plus DTIC, and RAIT plus doxorubicin and DTIC groups.

### Example 6: Studies with Naked Anti-CEA Alone

### Therapy with naked hMN-14

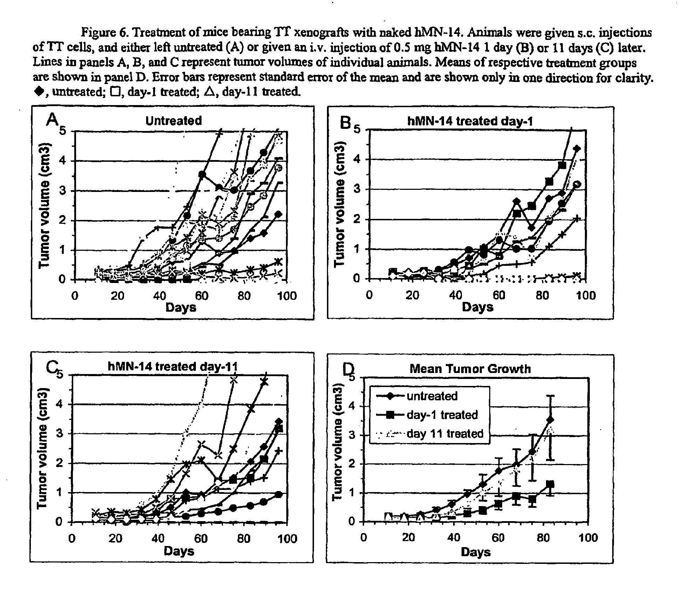

To study the effect of unlabeled hMN-14 on the growth of TT tumors in nude mice, a single injection of hMN-14 was administered i.v, either one day or eleven days post tumor cell injection. Figure 6 shows the tumor growth curves of animals treated with 0.5 mg hMN-14/mouse compared to untreated controls. The untreated group contained 16 animals; the two treatment groups contained 10 animals each. A significant growth delay was observed between the untreated group and the group treated on day-1 post tumor injection. Significant differences in the mean tumor sizes (p<0.05) were observed from day-32 through day-93. Between day-32 and day-60 there was a 64-70% inhibition of tumor size in the MN-14 treated group compared to the untreated animals. There were no significant differences between the mean tumor sizes in the day-11 group and untreated animals. Significant delay in tumor growth was also seen by t-test analysis of the area under the growth curves. P<0.05 for the untreated group compared to the group treated one day following tumor injection, but not for the group treated eleven days following tumor injection.

### Specificity of treatment

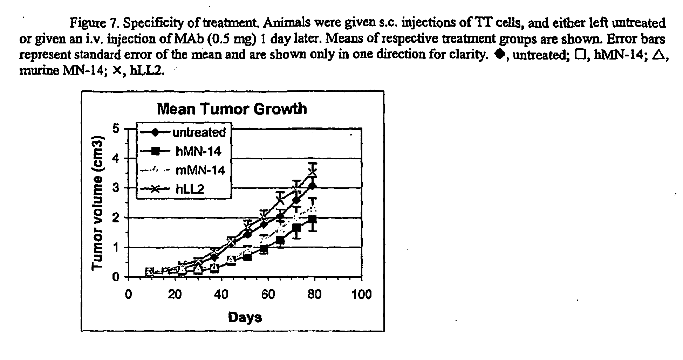

Figure 7 summarizes the results of a study on the specificity of the anti-tumor response. The effect of unlabeled hMN-14 on the growth of TT tumors in nude mice was compared to that of a negative control humanized MAb, hLL2 (anti-CD22), and the murine MN-14. MAbs (0.5 mg/mouse) were administered (i.v.) one day after TT cells, then three additional weekly doses of 0.5 mg/mouse were given. Groups of 15 animals were studied. The growth inhibition observed in the first study from treatment with 0.5 mg hMN-14 was confirmed in this study. Significant differences in mean tumor sizes (p<0.05) between the hMN-14 and the untreated group were observed starting at day-23. At day-37 the mean tumor volume in the group treated with hMN-14 was 42.7% of the untreated control animals. Treatment with murine MN-14 yielded results similar to the hMN-14. Treatment with hLL2 did not slow tumor growth; instead there was a small (not significant) increase in growth rate. For example, at day-37 87% of the tumors treated with hMN-14 were less than 0.5 cm³, compared to 40% of the untreated and 29% of the hLL2 treated group. T-test analysis of the area under the growth curves demonstrated significant differences (p<0.05) between the untreated group and the groups treated with either hMN-14 or murine MN-14, but not the group treated with hLL2. In addition, the hMN-14 group was significantly different from the hLL2 group but not the murine MN-14 treated animals.

### Effect of Dose

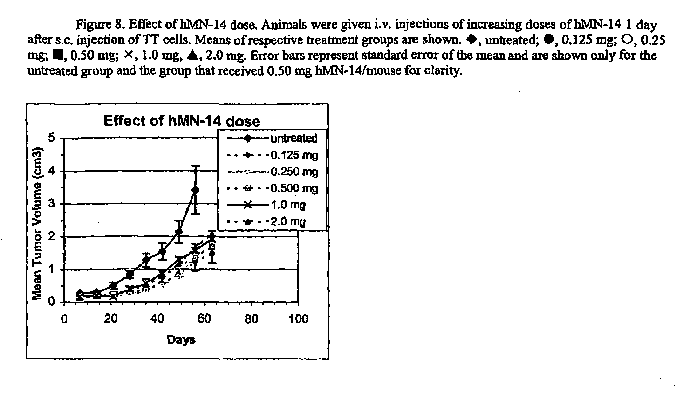

To study the effect of dose of unlabeled hMN-14 on the growth of TT tumors in nude mice, increasing doses of hMN-14 were evaluated. Antibody doses were administered 1 day after TT cells, then weekly until the termination of the study. Weekly doses ranged from 0.125 mg to 2.0 mg hMN-14/mouse in groups of six mice. Significant differences in mean tumor sizes and area under the growth curves between the untreated group and all treatment groups were observed (Figure 8). For example, between day-21 and day-49 mean tumor volume in the 2 lowest hMN-14 treatment groups were 27-40% of the size of tumors in the untreated animals. Treatment with the lower doses, 0.125 mg and 0.25 mg, appeared to be more effective than treatment with the higher doses, although the difference did not reach statistical significance.

### Timing

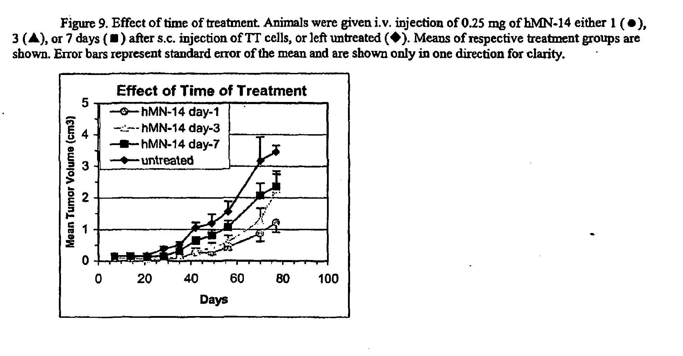

The effect of time between TT injection and initial dose of hMN-14 on the growth of TT tumors in nude mice was evaluated by varying the day of administration of MAb. hMN-14 (0.25 mg) was administered either 1, 3, or 7 days after TT cells, then weekly until termination of the study. Groups of 7-8 animals were studied. Results are summarized in Figure 9. Significant differences in mean tumor sizes (p < 0.05) between the untreated group and all three treatment groups were observed. However, the difference in mean tumor size between the untreated mice and the day-7 treatment group was only significant at one time point, day-28. Day-1 treated mice yielded significant differences from 21-77 days, and day-3 treated mice yielded significant differences from 21-70 days. T-test analysis of the area under the growth curves indicated significant growth inhibition for the groups treated with hMN-14 either 1 or 3 days after TT cell administration compared to untreated group. This analysis did not reach the 95% confidence limit for difference between the untreated group and the group treated on day-7 (p=0.057 at 5 weeks).

### Example 7: Combined naked anti-CEA Plus DTIC Therapy of MTC

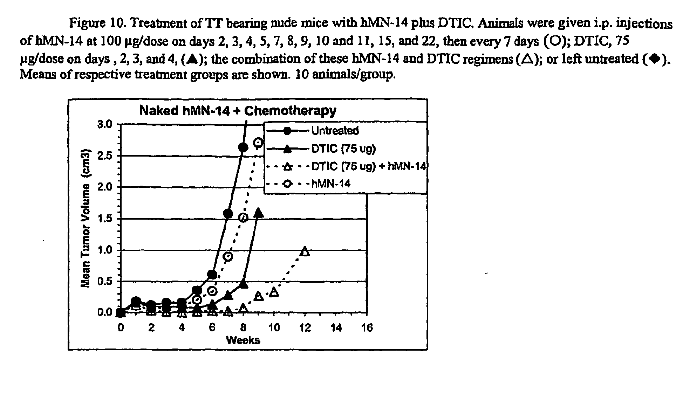

To study whether naked hMN-14 can add to the efficacy of DTIC, IT bearing nude mice were given DTIC (75 µg/dose) in combination with a course of treatment of the unlabeled MAb. DTIC was administered for 3 consecutive days at 75 µg/dose as one course, beginning 2 days after s.c. injection of TT cells. hMN-14 MAb treatment was initiated on the same day as the first dose of DTIC, at 100 µg/dose/day for 5 days in the first two weeks, then twice weekly. Significant delays in tumor growth were caused by these schedules of either MAb therapy or chemotherapy alone (Figure 10). The 75-µg dose of DTIC in combination with this schedule of hMN-14 was significantly more effective than either treatment alone (P<0.02). At 7 weeks, 8/10 mice in the 75 µg DTIC + MAb group had no palpable tumor, compared to 1/10 in the 75 µg DTIC-only group and 0/10 in the untreated and MAb-only groups. Mean tumor volumes at 7 weeks were 0.018+0.039 cm³ (75 µg DTIC+hMN-14), 0.284+0.197 cm³ (75 µg DTIC), 0.899+0.545 cm³ (hMN-14) and 1.578+0.959 cm³ (untreated).

The anti-CEA MAb MN-14 has shown unexpected anti-tumor efficacy in MTC without conjugation to a cytotoxic agent. Differences in mean tumor sizes between the hMN-14 treated and the untreated groups were observed beginning at 3 weeks and lasting at least 2 months. Treatment with isotype matched negative control MAbs did not slow tumor growth. This is the first evidence of tumor suppression with a "naked" anti-CEA MAb. However, combined therapy of the naked anti-CEA MAb with DTIC augments the anti-tumor effects of antibody or chemotherapy alone, without increased toxicity. The superiority of the combined modality treatment argues for the integration of CEA-MAb therapy into chemotherapeutic regimens for MTC management. The mechanism of tumor cell killing by MN-14 is not known and likely involves several mechanisms. The studies proposed here will provide the basis for understanding these observations and for justification of future clinical trials.

## Claims

1. A composition comprising at least one naked Class III anti-CEA monoclonal antibody (MAb) or fragment thereof and at least one therapeutic agent.

2. The composition of claim 1, wherein said Class III anti-CEA MAb or fragment thereof is a chimeric, humanized or human MAb that retains the Class III anti-CEA binding specificity of a murine Class III anti-CEA MAb.

3. The composition of claim 1, wherein the therapeutic agent is a toxin or a chemotherapeutic agent.

4. The composition according to claim 3, wherein the chemotherapeutic agent is selected from the group consisting of vincristine, doxorubicin, DTIC, cyclophosphamide, CPT-11, oxaliplatin, taxane, gemcitabine, a COX-2 inhibitor and 5-fluorouracil.

5. The composition of claim 1, wherein said Class III anti-CEA monoclonal antibody or fragment thereof is a MN-14 antibody or fragment thereof.

6. The composition of claim 5, wherein said MN-14 monoclonal antibody or fragment thereof comprises the complementarity-determining regions (CDRs) of a murine MN-14 monoclonal antibody, wherein the CDRs of the light chain variable region of said MN-14 antibody comprises CDR1 comprising the amino acid sequence KASQDVGTSVA; CDR2 comprising the amino acid sequence WTSTRHT; and CDR3 comprising the amino acid sequence QQYSLYRS; and the CDRs of the heavy chain variable region of said Class III anti-CEA antibody comprises CDR1 comprising TYWMS; CDR2 comprising EIHPDSSTINYAPSLKD; and CDR3 comprising LYFGFPWFAY.

7. The composition of claim 5, wherein said MN-14 monoclonal antibody reacts with CEA and is unreactive with normal cross-reactive antigen (NCA) and meconium antigen (MA).

8. The composition of claim 7, wherein said MN-14 monoclonal antibody or fragment thereof is a humanized MN-14 antibody or fragment thereof.

9. The composition of claim 7, wherein said MN-14 monoclonal antibody or fragment thereof is a chimeric MN-14 antibody or fragment thereof.

10. The composition of claim 8, wherein the framework regions (FRs) of the light and heavy chain variable regions of said humanized MN-14 antibody or fragment thereof comprise at least one amino acid substituted from the corresponding FRs of a murine MN-14 monoclonal antibody.

11. The composition of claim 10, wherein said humanized MN-14 antibody or fragment thereof comprises at least one amino acid from said corresponding FR of said murine MN-14 antibody, selected from the group consisting of amino acid residue 24, 28, 30, 48, 49, 74 and 94 of the murine heavy chain variable region of Figure 14A-C, preferably the heavy chain variable region is humanized heavy chain variable region KLHuVhAIGA of Figure 14A-C or hMN14 of Figure 15B or Figure 16B.

12. The composition of claim 10, wherein said humanized MN-14 antibody or fragment thereof comprises at least one amino acid from said corresponding FR of said murine MN-14 light chain variable region.

13. The composition of claim 8, wherein said humanized MN-14 antibody or fragment thereof comprises the light chain variable region as set forth in Figure 13A or Figure 15A designated as hMN14 or Figure 16A, and the heavy chain variable region set forth in Figure 14A-C designated as KLHuVhAIGA or Figure 15B designated as hMN14 or Figure 16B.

14. The composition of claim 9, wherein said chimeric MN-14 antibody or fragment thereof comprises the light chain variable region as set forth in Figure 13A designated as murine MN-14 Vκ and the heavy chain variable region set forth in Figure 14A-C designated as murine MN-14 VH.

15. The composition of any of claims 1-14, wherein said fragment is selected from the group consisting of F(ab')2, Fab', Fab, Fv and scFv.

16. The composition of any of claims 1-14, wherein said therapeutic agent is selected from the group consisting of a cytotoxic agent, a drug, a radionuclide, an immunomodulator, a photoactive therapeutic agent, a hormone, or a combination thereof, optionally formulated in a pharmaceutically acceptable vehicle.

17. The composition of any of claims 1-14, wherein said therapeutic agent is a naked antibody or an immunoconjugate, wherein said naked antibody or an antibody portion of said immunoconjugate comprises a humanized, chimeric, human or murine monoclonal antibody or fragment thereof selected from the group consisting of a monoclonal antibody or fragment thereof reactive with EGP-1, EGP-2, MUC-1, MUC-2, MUC-3, MUC-4, PAM-4, KC4, TAG-72, EGFR, HER2/neu, BrE3, Le-Y, A3, A33, Ep-CAM, AFP, Tn, Thomson-Friedenreich antigens, VEGF, Ga 733, or a combination thereof.

18. The composition of claim 17, wherein said fragment is selected from the group consisting of F(ab')2, Fab', Fab, Fv and scFv.

19. The composition of any of claims 1-14, wherein said therapeutic agent is not DTIC.

20. A naked Class III anti-CEA monoclonal antibody or fragment thereof for use in a method for treating CEA expressing non-medullary thyroid carcinoma comprising administering to a subject, either concurrently or sequentially, a therapeutically effective amount of the Class III anti-CEA monoclonal antibody or fragment thereof and at least one therapeutic agent, and optionally formulated in a pharmaceutically acceptable vehicle.

21. The naked Class III anti-CEA monoclonal antibody or fragment thereof of claim 20 for use as in claim 20, wherein said Class III anti-CEA MAb or fragment thereof is humanized, wherein said humanized MAb retains substantially the Class III anti-CEA binding specificity of a murine Class III anti-CEA MAb.

22. The naked Class III anti-CEA monoclonal antibody or fragment thereof of claim 20 for use as in claim 20, wherein said Class III anti-CEA MAb or fragment thereof is a chimeric MAb, and wherein said chimeric MAb retains substantially the Class III anti-CEA binding specificity of murine Class III anti-CEA MAb.

23. The naked Class III anti-CEA monoclonal antibody or fragment thereof of claim 20 for use as in claim 20, wherein said Class III anti-CEA monoclonal antibody or fragment thereof is a MN-14 antibody or fragment thereof.

24. The naked Class III anti-CEA monoclonal antibody or fragment thereof of claim 20 for use as in claim 20, wherein said MN-14 monoclonal antibody or fragment thereof comprises the complementarity-determining regions (CDRs) of a murine MN-14 monoclonal antibody, wherein the CDRs of the light chain variable region of said MN-14 antibody comprises CDR1 comprising the amino acid sequence KASQDVGTSVA; CDR2 comprising the amino acid sequence WTSTRHT; and CDR3 comprising the amino acid sequence QQYSLYRS; and the CDRs of the heavy chain variable region of said Class III anti-CEA antibody comprises CDR1 comprising TYWMS; CDR2 comprising EIHPDSSTINYAPSLKD; and CDR3 comprising LYFGFPWFAY.

25. The naked Class III anti-CEA monoclonal antibody or fragment thereof of claim 24 for use as in claim 20, wherein said MN-14 monoclonal antibody reacts with CEA and is unreactive with normal cross-reactive antigen (NCA) and meconium antigen (MA).

26. The naked Class III anti-CEA monoclonal antibody or fragment thereof of claim 25 for use as in claim 20, wherein said MN-14 monoclonal antibody or fragment thereof is a humanized MN-14 antibody or fragment thereof.

27. The naked Class III anti-CEA monoclonal antibody or fragment thereof of claim 25 for use as in claim 20, wherein said MN-14 monoclonal antibody or fragment thereof is a chimeric MN-14 antibody or fragment thereof.

28. The naked Class III anti-CEA monoclonal antibody or fragment thereof of claim 26 for use as in claim 20, wherein the framework regions (FRs) of the light and heavy chain variable regions of said humanized MN-14 antibody or fragment thereof comprise at least one amino acid substituted from the corresponding FRs of a murine MN-14 monoclonal antibody.

29. The naked Class III anti-CEA monoclonal antibody or fragment thereof of claim 28 for use as in claim 20, wherein said humanized MN-14 antibody or fragment thereof comprising at least one amino acid from said corresponding FR of said murine MN-14 antibody, selected from the group consisting of amino acid residue 24, 28, 30, 48, 49, 74 and 94 of the murine heavy chain variable region of Figure 14A-C, preferably the heavy chain variable region is humanized heavy chain variable region KLHuVhAIGA of Figure 14A-C or hMN14 of Figure 15B or Figure 16B.

30. The naked Class III anti-CEA monoclonal antibody or fragment thereof of claim 28 for use as in claim 20, wherein said humanized MN-14 antibody or fragment thereof comprises at least one amino acid from said corresponding FR of said murine MN14 light chain variable region.

31. The naked Class III anti-CEA monoclonal antibody or fragment thereof of claim 28 for use as in claim 20, wherein said humanized MN-14 antibody or fragment thereof comprises the light chain variable region as set forth in Figure 13A or 15A designated as hMN14 or 16A and the heavy chain variable region set forth in Figure 14A-C designated as KLHuVhAIGA or 15B designated as hMN14 or 16B.

32. The naked Class III anti-CEA monoclonal antibody or fragment thereof of any of claims 20-31 for use as in claim 20, wherein said fragment is selected from the group consisting of F(ab')2, Fab', Fab, Fv and scFv.

33. The naked Class III anti-CEA monoclonal antibody or fragment thereof of any of claims 20-31 for use as in claim 20, wherein said therapeutic agent is selected from the group consisting of humanized, chimeric, human or murine monoclonal antibody or fragment thereof selected from the group consisting of a Class II anti-CEA monoclonal antibody, and a fragment thereof, and is administered either concurrently or sequentially in a therapeutically effective amount.

34. The naked Class III anti-CEA monoclonal antibody or fragment thereof of any of claims 20-31 for use as in claim 20, wherein said therapeutic agent is selected from the group consisting of a cytotoxic agent, a drug, a radionuclide, an immunomodulator, a photoactive therapeutic agent, a hormone, or a combination thereof, optionally formulated in a pharmaceutically acceptable vehicle.

35. The naked Class III anti-CEA monoclonal antibody or fragment thereof of any of claims 20-32 for use as in claim 20, wherein said therapeutic agent is a naked antibody or an immunoconjugate, wherein the naked antibody and the antibody moiety of the immunoconjugate is selected from the group consisting of a humanized, chimeric, human or murine monoclonal antibody or fragment thereof reactive with EGP-1, EGP-2, MUC-1, MUC-2, MUC-3, MUC-4, PAM-4, KC4, TAG-72, EGFR, HER2/neu, BrE3, Le-Y, A3, A33, Ep-CAM, AFP, Tn, Thomson-Friedenreich antigens, VEGF, Ga 733, and a combination thereof, and is administered to said subject either concurrently or sequentially in a therapeutically effective amount.

36. The naked Class III anti-CEA monoclonal antibody or fragment thereof of any of claims 20-31 for use as in claim 20, wherein said therapeutic agent is not DTIC.

37. The naked Class III anti-CEA monoclonal antibody or fragment thereof of claim 34 for use as in claim 20, wherein said cytotoxic agent is a drug or a toxin.

38. The naked Class III anti-CEA monoclonal antibody or fragment thereof of claim 37 for use as in claim 20, wherein said drug possesses the pharmaceutical property selected from the group consisting of antimitotic, alkylating, antimetabolite, antiangiogenic, apoptotic, alkaloid, COX-2, and antibiotic agents and combinations thereof.

39. The naked Class III anti-CEA monoclonal antibody or fragment thereof of claim 37 for use as in claim 20, wherein said drug is selected from the group consisting of nitrogen mustards, ethylenimine derivatives, alkyl sulfonates, nitrosoureas, triazenes, folic acid analogs, anthracyclines, taxanes, COX-2 inhibitors, pyrimidine analogs, purine analogs, antimetabolites, antibiotics, enzymes, epipodophyllotoxins, platinum coordination complexes, vinca alkaloids, substituted ureas, methyl hydrazine derivatives, adrenocortical suppressants, antagonists, endostatin, taxols, camptothecins, doxorubicins and their analogs, and a combination thereof.

40. The naked Class III anti-CEA monoclonal antibody or fragment thereof of claim 37 for use as in claim 20, wherein said toxin is a microbial, plant or animal toxin selected from the group consisting of ricin, abrin, alpha toxin, saporin, ribonuclease (RNase), DNase I, Staphylococcal enterotoxin-A, pokeweed antiviral protein, gelonin, diphtheria toxin, Pseudomonas exotoxin, and Pseudomonas endotoxin.

41. The naked Class III anti-CEA monoclonal antibody or fragment thereof of claim 34 for use as in claim 20, wherein said immunomodulator is selected from the group consisting of a cytokine, a stem cell growth factor, a lymphotoxin, a hematopoietic factor, a colony stimulating factor (CSF), an interferon (IFN), erythropoietin, thrombopoietin and a combination thereof.

42. The naked Class III anti-CEA monoclonal antibody or fragment thereof of claim 41 for use as in claim 20, wherein said lymphotoxin is tumor necrosis factor (TNF), said hematopoietic factor is an interleukin (IL), said colony stimulating factor is granulocyte-colony stimulating factor (G-CSF) or granulocyte macrophage-colony stimulating factor (GM-CSF), said interferon is interferon-α, -β or -γ, and said stem cell growth factor is designated "S1 factor".

43. The naked Class III anti-CEA monoclonal antibody or fragment thereof of claim 41 for use as in claim 20, wherein said immunomodulator comprises IL-1, IL-2, IL-3, IL-6, IL-10, IL-12, IL-18, interferon-γ, TNF-α or a combination thereof.

44. The naked Class III anti-CEA monoclonal antibody or fragment thereof of claim 34 for use as in claim 20, wherein said radionuclide has an energy between 20 and 10,000 keV.

45. The naked Class III anti-CEA monoclonal antibody or fragment thereof of claim 44 for use as in claim 20, wherein said radionuclide is selected from the group consisting of ¹²⁵I, ¹³¹I, ⁹⁰Y, ⁸⁸Y, ²²⁵Ac, ¹⁷⁷Lu, ¹⁸⁸Re, ¹⁸⁶Re, and combinations thereof.

46. The naked Class III anti-CEA monoclonal antibody or fragment thereof of claim 34 for use as in claim 20, wherein said photoactive therapeutic agent is a chromogen or dye.

47. The naked Class III anti-CEA monoclonal antibody or fragment thereof of claim 38 for use as in claim 20, wherein said alkylating agent is dacarbazine.

48. The naked Class III anti-CEA monoclonal antibody or fragment thereof of claim 47 for use as in claim 20, wherein said MN-14 antibody or fragment thereof is administered in a dosage of 100 to 600 milligrams protein per dose per injection.

49. The naked Class III anti-CEA monoclonal antibody or fragment thereof of claim 48 for use as in claim 20, wherein said MN-14 antibody or fragment thereof is administered in a dosage of 300 milligrams protein per dose per injection.

50. A naked Class III anti-CEA monoclonal antibody or fragment thereof for use in treating medullary thyroid carcinoma comprising administering to a subject, either concurrently or sequentially, a therapeutically effective amount of the Class III anti-CEA monoclonal antibody or fragment thereof and at least one therapeutic agent, and optionally formulated in a pharmaceutically acceptable vehicle.

51. The naked Class III anti-CEA monoclonal antibody or fragment thereof of claim 50 for use as in claim 50, wherein said Class III anti-CEA MAb or fragment thereof is humanized, wherein said humanized MAb retains substantially the Class III anti-CEA binding specificity of a murine Class III anti-CEA MAb.

52. The naked Class III anti-CEA monoclonal antibody or fragment thereof of claim 50 for use as in claim 50, wherein said Class III anti-CEA MAb or fragment thereof is a chimeric MAb, and wherein said chimeric MAb retains substantially the Class III anti-CEA binding specificity of murine Class III anti-CEA MAb.

53. The naked Class III anti-CEA monoclonal antibody or fragment thereof of claim 50 for use as in claim 50, wherein said Class III anti-CEA monoclonal antibody or fragment thereof is a MN-14 antibody or fragment thereof.

54. The naked Class III anti-CEA monoclonal antibody or fragment thereof of claim 53 for use as in claim 50, wherein said MN-14 monoclonal antibody or fragment thereof comprises the complementarity-determining regions (CDRs) of a murine MN-14 monoclonal antibody, wherein the CDRs of the light chain variable region of said MN-14 antibody comprises CDR1 comprising the amino acid sequence KASQDVGTSVA; CDR2 comprising the amino acid sequence WTSTRHT; and CDR3 comprising the amino acid sequence QQYSLYRS; and the CDRs of the heavy chain variable region of said Class III anti-CEA antibody comprises CDR1 comprising TYWMS; CDR2 comprising EIHPDSSTINYAPSLKD; and CDR3 comprising LYFGFPWFAY.

55. The naked Class III anti-CEA monoclonal antibody or fragment thereof of claim 54 for use as in claim 50, wherein said MN-14 monoclonal antibody reacts with CEA and is unreactive with normal cross-reactive antigen (NCA) and meconium antigen (MA).

56. The naked Class III anti-CEA monoclonal antibody or fragment thereof of claim 55 for use as in claim 50, wherein said MN-14 monoclonal antibody or fragment thereof is a humanized MN-14 antibody or fragment thereof.

57. The naked Class III anti-CEA monoclonal antibody or fragment thereof of claim 55 for use as in claim 50, wherein said MN-14 monoclonal antibody or fragment thereof is a chimeric MN-14 antibody or fragment thereof.

58. The naked Class III anti-CEA monoclonal antibody or fragment thereof of claim 56 for use as in claim 50, wherein the framework regions (FRs) of the light and heavy chain variable regions of said humanized MN-14 antibody or fragment thereof comprise at least one amino acid substituted from the corresponding FRs of a murine MN-14 monoclonal antibody.

59. The naked Class III anti-CEA monoclonal antibody or fragment thereof of claim 58 for use as in claim 50, wherein said humanized MN-14 antibody or fragment thereof comprising at least one amino acid from said corresponding FR of said murine MN-14 antibody, selected from the group consisting of amino acid residue 24, 28, 30, 48, 49, 74 and 94 of the murine heavy chain variable region of Figure 14A-C, preferably the heavy chain variable region is humanized heavy chain variable region KLHuVhAIGA of Figure 14A-C or hMN-14 of Figure 15B or Figure 16B.

60. The naked Class III anti-CEA monoclonal antibody or fragment thereof of claim 58 for use as in claim 50, wherein said humanized MN-14 antibody or fragment thereof comprises at least one amino acid from said corresponding FR of said murine MN14 light chain variable region.

61. The naked Class III anti-CEA monoclonal antibody or fragment thereof of claim 58 for use as in claim 50, wherein said humanized MN-14 antibody or fragment thereof comprises the light chain variable region as set forth in Figure 13A or 15A designated as hMN14 or 16A and the heavy chain variable region set forth in Figure 14A-C or 15B designated as hMN 14 or 16B.

62. The naked Class III anti-CEA monoclonal antibody or fragment thereof of any of claims 50-61 for use as in claim 50, wherein said fragment is selected from the group consisting of F(ab')2, Fab', Fab, Fv and scFv.

63. The naked Class III anti-CEA monoclonal antibody or fragment thereof of any of claims 50-61 for use as in claim 50, wherein said therapeutic agent is selected from the group consisting of humanized, chimeric, human or murine monoclonal antibody or fragment thereof selected from the group consisting of a Class II anti-CEA monoclonal antibody, and a fragment thereof, and is administered either concurrently or sequentially in a therapeutically effective amount.

64. The naked Class III anti-CEA monoclonal antibody or fragment thereof of any of claims 50-61 for use as in claim 50, wherein said therapeutic agent is selected from the group consisting of a cytotoxic agent, a drug, a radionuclide, an immunomodulator, a photoactive therapeutic agent, a hormone, or a combination thereof, optionally formulated in a pharmaceutically acceptable vehicle.

65. The naked Class III anti-CEA monoclonal antibody or fragment thereof of any of claims 50-62 for use as in claim 50, wherein said therapeutic agent is a naked antibody or an immunoconjugate, wherein the naked antibody and the antibody moiety of the immunoconjugate is selected from the group consisting of a humanized, chimeric, human or murine monoclonal antibody or fragment thereof reactive with EGP-1, EGP-2, MUC-1, MUC-2, MUC-3, MUC-4, PAM-4, KC4, TAG-72, EGFR, HER2/neu, BrE3, Le-Y, A3, A33, Ep-CAM, AFP, Tn, Thomson-Friedenreich antigens, VEGF, Ga 733, and a combination thereof, and is administered to said subject either concurrently or sequentially in a therapeutically effective amount.

66. The naked Class III anti-CEA monoclonal antibody or fragment thereof of any of claims 50-61 for use as in claim 50, wherein said therapeutic agent is not DTIC.

67. The naked Class III anti-CEA monoclonal antibody or fragment thereof of claim 64 for use as in claim 50, wherein said cytotoxic agent is a drug or a toxin.

68. The naked Class III anti-CEA monoclonal antibody or fragment thereof of claim 64 for use as in claim 50, wherein said drug possesses the pharmaceutical property selected from the group consisting of antimitotic, alkylating, antimetabolite, antiangiogenic, apoptotic, alkaloid, COX-2, and antibiotic agents and combinations thereof.

69. The naked Class III anti-CEA monoclonal antibody or fragment thereof of claim 67 for use as in claim 50, wherein said drug is selected from the group consisting of nitrogen mustards, ethylenimine derivatives, alkyl sulfonates, nitrosoureas, triazenes, folic acid analogs, anthracyclines, taxanes, COX-2 inhibitors, pyrimidine analogs, purine analogs, antimetabolites, antibiotics, enzymes, epipodophyllotoxins, platinum coordination complexes, vinca alkaloids, substituted ureas, methyl hydrazine derivatives, adrenocortical suppressants, antagonists, endostatin, taxols, camptothecins, doxorubicins and their analogs, and a combination thereof.

70. The naked Class III anti-CEA monoclonal antibody or fragment thereof of claim 67 for use as in claim 50, wherein said microbial, plant or animal toxin is selected from the group consisting of ricin, abrin, alpha toxin, saporin, ribonuclease (RNase), DNase I, Staphylococcal enterotoxin-A, pokeweed antiviral protein, gelonin, diphtheria toxin, Pseudomonas exotoxin, and Pseudomonas endotoxin.

71. The naked Class III anti-CEA monoclonal antibody or fragment thereof of claim 64 for use as in claim 50, wherein said immunomodulator is selected from the group consisting of a cytokine, a stem cell growth factor, a lymphotoxin, a hematopoietic factor, a colony stimulating factor (CSF), an interferon (IFN), erythropoietin, thrombopoietin and a combination thereof.

72. The naked Class III anti-CEA monoclonal antibody or fragment thereof of claim 71 for use as in claim 50, wherein said lymphotoxin is tumor necrosis factor (TNF), said hematopoietic factor is an interleukin (IL), said colony stimulating factor is granulocyte-colony stimulating factor (G-CSF) or granulocyte macrophage-colony stimulating factor (GM-CSF), said interferon is interferon-α, -β or -γ, and said stem cell growth factor is designated "S1 factor".

73. The naked Class III anti-CEA monoclonal antibody or fragment thereof of claim 64 for use as in claim 50, wherein said immunomodulator comprises IL-1, IL-2, IL-3, IL-6, IL-10, IL-12, IL-18, interferon-γ, TNF-α or a combination thereof.

74. The naked Class III anti-CEA monoclonal antibody or fragment thereof of claim 64 for use as in claim 50, wherein said radionuclide has an energy between 20 and 10,000 keV.

75. The naked Class III anti-CEA monoclonal antibody or fragment thereof of claim 74 for use as in claim 50, wherein said radionuclide is selected from the group consisting of ¹²⁵I, ¹³¹I, ⁹⁰Y, ⁸⁸Y, ²²⁵Ac, ¹⁷⁷Lu, ¹⁸⁸Re, ¹⁸⁶Re, and combinations thereof.

76. The naked Class III anti-CEA monoclonal antibody or fragment thereof of claim 64 for use as in claim 50, wherein said photoactive therapeutic agent is a chromogen or dye.

77. The naked Class III anti-CEA monoclonal antibody or fragment thereof of claim 68 for use as in claim 50, wherein said alkylating agent is dacarbazine.

78. The naked Class III anti-CEA monoclonal antibody or fragment thereof of claim 77 for use as in claim 50, wherein said MN-14 antibody or fragment thereof is administered in a dosage of 100 to 600 milligrams protein per dose per injection.

79. The naked Class III anti-CEA monoclonal antibody or fragment thereof of claim 78 for use as in claim 50, wherein said MN-14 antibody or fragment thereof is administered in a dosage of 300 milligrams protein per dose per injection.

## Patentansprüche

1. Zusammensetzung umfassend wenigstens einen nackten Klasse III-anti-CEA monoklonalen Antikörper (MAb) oder Fragment davon und wenigstens ein therapeutisches Agens.

2. Zusammensetzung nach Anspruch 1, wobei der Klasse III-anti-CEA MAb oder Fragment davon ein chimärer, humanisierter oder humaner MAb ist, der die Klasse III-anti-CEA-Bindungspezifität eines murinen Klasse III-anti-CEA MAb beibehält.

3. Zusammensetzung nach Anspruch 1, wobei das therapeutische Agens ein Toxin oder ein chemotherapeutisches Agens ist.

4. Zusammensetzung nach Anspruch 3, wobei das chemotherapeutische Agens ausgewählt ist aus der Gruppe bestehend aus Vincristin, Doxorubicin, DTIC, Cyclophosphamid, CPT-11, Oxaliplatin, Taxan, Gemcitabin, einem COX-2-Inhibitor und 5-Fluorouracil.

5. Zusammensetzung nach Anspruch 1, wobei der Klasse III-anti-CEA monoklonale Antikörper oder Fragment davon ein MN-14 Antikörper oder Fragment davon ist.

6. Zusammensetzung nach Anspruch 5, wobei der MN-14 monklonale Antikörper oder Fragment davon die komplementaritätsbestimmenden Regionen (CDRs) eines murinen MN-14 monoklonalen Antikörpers umfasst, wobei die CDRs der variablen Region der leichten Kette des MN-14 Antikörpers CDR1 umfassend die Aminosäuresequenz KASQDVGTSVA: CDR2 umfassend die Aminosäuresequenz WTSTRHT; und CDR 3 umfassend die Aminosäuresequenz QQYSLYRS umfasst; und die CDRs der variablen Region der schweren Kette des Klasse III-anti-CEA Antikörpers CDR1 umfassend TYWMS; CDR2 umfassend EIHPDSSTINYAPSLKD; und CDR3 umfassend LYFGFPWFAY umfasst.

7. Zusammensetzung nach Anspruch 5, wobei der MN-14 monoklonale Antikörper mit CEA reagiert und nicht reagiert mit normalem kreuzreaktivem Antigen (NCA) und Meconium-Antigen (MA).

8. Zusammensetzung nach Anspruch 7, wobei der MN-14 monoklonale Antikörper oder Fragment davon ein humanisiert MN-14 Antikörper oder Fragment davon ist.

9. Zusammensetzung nach Anspruch 7, wobei der MN-14 monoklonale Antikörper oder Fragment davon ein chimärer MN-14 Antikörper oder Fragment davon ist.

10. Zusammensetzung nach Anspruch 8, wobei die Gerüstregionen (FRs) der variablen Regionen der leichten und schweren Kette des humanisierten MN-14 Antikörpers oder Fragmentes davon wenigstens eine Aminosäure umfassen, die gegenüber den entsprechenden FRs eines murinen MN-14 monoklonalen Antikörpers ausgetauscht ist.

11. Zusammensetzung nach Anspruch 10, wobei der humanisierte MN-14 Antikörper oder Fragment davon wenigstens eine Aminosäure der korrespondierenden FR des murinen MN-14 Antikörpers umfasst, die ausgewählt ist aus der Gruppe bestehend aus Aminosäureresten 24, 28, 30, 48, 49, 74 und 94 der variablen Region der murinen schweren Kette von Figur 14A-C, bevorzugterweise ist die variable Region der schweren Kette die variable Region der humanisierten schweren Kette KLHuVhAIGA von Figur 14A-C oder hMN14 von Figur 15B oder Figur 16B.

12. Zusammensetzung nach Anspruch 10, wobei der humanisierte MN-14 Antikörper oder Fragment davon wenigstens eine Aminosäure von der korrespondierenden FR der variablen Region der leichten Kette des murinen MN-14 umfasst.

13. Zusammensetzung nach Anspruch 8, wobei der humanisierte MN-14 Antikörper oder Fragment davon die variable Region der leichten Kette wie in Figur 13A oder Figur 15A, bezeichnet als hMN14, oder Figur 16A angegeben und die variable Region der schweren Kette wie in Figur 14A-C, bezeichnet als KLHuVhAIGA, oder Figur 15B, bezeichnet als hMN14, oder Figur 16B angegeben umfasst.

14. Zusammensetzung nach Anspruch 9, wobei der chimäre MN-14 Antikörper oder Fragment davon die variable Region der leichten Kette wie in Figur 13A, als murine MN-14 Vk bezeichnet, angegeben, und die variable Region der schweren Kette wie in Figur 14A-C, als murine MN-14 VH bezeichnet, angegeben umfasst.

15. Zusammensetzung nach einem der Ansprüche 1-14, wobei das Fragment ausgewählt ist aus der Gruppe bestehend aus F(ab')2, Fab', Fab, Fv und scFv.

16. Zusammensetzung nach einem der Ansprüche 1-14, wobei das therapeutische Agens ausgewählt ist aus der Gruppe bestehend aus einem cytotoxischen Agens, einem Wirkstoff, einem Radionuklid, einem Immunmodulator, einem photoaktiven therapeutischen Agens, einem Hormon oder einer Kombination davon, optional formuliert in einem pharmazeutisch akzeptablen Vehikel.

17. Zusammensetzung nach einem der Ansprüche 1-14, wobei das therapeutische Agens ein nackter Antikörper oder ein Immunkonjugat ist, wobei der nackte Antikörper oder ein Antikörperteil des Immunkonjugat einen humanisierten, chimären, humanen oder murinen monoklonalen Antikörper oder Fragment davon umfasst, der/das ausgewählt ist aus der Gruppe bestehend aus einem monoklonale Antikörper oder Fragment davon, der/das reagiert mit EGP-1, EGP-2, MUC-1, MUC-2, MUC-3, MUC-4, PAM-4, KC4, TAG-72, EGFR, HER2/neu, BrE3, Le-Y, A3, A33, Ep-CAM, AFP, Tn, Thomson-Friedenreich-Antigene, VEGF, Ga 733 oder einer Kombination davon.

18. Zusammensetzung nach Anspruch 17, wobei das Fragment ausgewählt ist aus der Gruppe bestehend aus F(ab')2, Fab', Fab, Fv und scFv.

19. Zusammensetzung nach einem der Ansprüche 1-14, wobei das therapeutische Agens nicht DTIC ist.

20. Ein nackter Klasse III-anti-CEA monoklonaler Antikörper oder Fragment davon zur Verwendung in einem Verfahren zur Behandlung von CEA-exprimierendem nichtmedullärem Schilddrüsenkrebs umfassend Verabreichung an ein Lebewesen, entweder gleichzeitig oder sequentiell, einer therapeutisch wirksamen Menge des Klasse III-anti-CEA monoklonalen Antikörpers oder Fragmentes davon und wenigstens eines therapeutischen Agens, und optional formuliert in einem pharmazeutisch akzeptablen Vehikel.

21. Nackter Klasse III-anti-CEA monoklonaler Antikörper oder Fragment davon nach Anspruch 20, zur Verwendung wie in Anspruch 20, wobei der Klasse III-anti-CEA MAb oder Fragment davon humanisiert ist, wobei der humanisierte MAb im Wesentlichen die Klasse III-anti-CEA-Bindungsspezifität eines murinen Klasse III-anti-CEA MAb beibehält.

22. Nackter Klasse III-anti-CEA monoklonaler Antikörper oder Fragment davon nach Anspruch 20, zur Verwendung wie in Anspruch 20, wobei der Klasse III-anti-CEA MAb oder Fragment davon ein chimärer MAb ist, wobei der chimäre MAb im Wesentlichen die Klasse III-anti-CEA-Bindungsspezifität eines murinen Klasse III-anti-CEA MAb beibehält.

23. Nackter Klasse III-anti-CEA monoklonaler Antikörper oder Fragment davon nach Anspruch 20, zur Verwendung wie in Anspruch 20, wobei der Klasse III-anti-CEA monoklonale Antikörper oder Fragment davon ein MN-14 Antikörper oder Fragment davon ist.

24. Nackter Klasse III-anti-CEA monoklonaler Antikörper oder Fragment davon nach Anspruch 20, zur Verwendung wie in Anspruch 20, wobei der MN-14 monoklonale Antikörper oder Fragment davon die komplementaritätsbestimmenden Regionen (CDRs) eines murinen MN-14 monoklonalen Antikörpers umfasst, wobei die CDRs der variablen Region der leichten Kette des MN-14 Antikörpers CDR 1 umfassend die Aminosäuresequenz KASQDVGTSVA; CDR2 umfassend die Aminosäuresequenz WTSTRHT; und CDR3 umfassend die Aminosäuren QQYSLYRS umfasst; und die CDRs der variablen Region der schweren Kette des Klasse III-anti-CEA Antikörpers CDR1 umfassend TYWMS; CDR2 umfassend EIHPDSSTINYAPSLKD; und CDR3 umfassend LYFGFPWFAY umfasst.

25. Nackter Klasse III-anti-CEA monoklonaler Antikörper oder Fragment davon nach Anspruch 24, zur Verwendung wie in Anspruch 20, wobei der MN-14 monoklonale Antikörper mit CEA reagiert und nicht reagiert mit normalem kreuzreaktivem Antigen (NCA) und Meconium-Antigen (MA).

26. Nackter Klasse III-anti-CEA monoklonaler Antikörper oder Fragment davon nach Anspruch 25, zur Verwendung wie in Anspruch 20, wobei der MN-14 monoklonale Antikörper oder Fragment davon ein humanisierter MN-14 Antikörper oder Fragment davon ist.

27. Nackter Klasse III-anti-CEA monoklonaler Antikörper oder Fragment davon nach Anspruch 25, zur Verwendung wie in Anspruch 20, wobei der MN-14 monoklonale Antikörper oder Fragment davon ein chimärer MN-14 Antikörper oder Fragment davon ist.

28. Nackter Klasse III-anti-CEA monoklonaler Antikörper oder Fragment davon nach Anspruch 26, zur Verwendung wie in Anspruch 20, wobei die Gerüstregionen (FRs) der variablen Regionen der leichten und schweren Kette des humanisierten MN-14 Antikörpers oder Fragmentes davon wenigstens eine Aminosäure umfassen, die substiuiert ist gegenüber den korrespondierenden FRs eines murinen MN-14 monoklonalen Antikörpers.

29. Nackter Klasse III-anti-CEA monoklonaler Antikörper oder Fragment davon nach Anspruch 28, zur Verwendung wie in Anspruch 20, wobei der humanisierte MN-14 Antikörper oder Fragment davon wenigstens eine Aminosäure von der korrespondierenden FR des murinen MN-14 Antikörpers umfasst, die ausgewählt ist aus der Gruppe bestehend aus Aminosäureresten 24, 28, 30, 48, 49, 74 und 94 der variablen Region der murinen schweren Kette von Figur 14A-C, bevorzugterweise ist die variable Region der schweren Kette die variable Region der humanisierten schweren Kette KLHuVhAIGA von Figur 14A-C oder hMN14 von Figur 15B oder von Figur 16B.

30. Nackter Klasse III-anti-CEA monoklonaler Antikörper oder Fragment davon nach Anspruch 28, zur Verwendung wie in Anspruch 20, wobei der humanisierte MN-14 Antikörper oder Fragment davon wenigstens eine Aminosäure von der korrespondierenden FR der variablen Region der leichten Kette des murinen MN-14 umfasst.

31. Nackter Klasse III-anti-CEA monoklonaler Antikörper oder Fragment davon nach Anspruch 28, zur Verwendung wie in Anspruch 20, wobei der humanisierte MN-14 Antikörper oder Fragment davon die variable Region der leichten Kette wie angegeben in Figur 13A oder 15A, bezeichnet als hMN14, oder 16A, und die variable Region der schweren Kette wie angegeben in Figur 14A-C, bezeichnet als KLHuVhAIGA, oder 15B, bezeichnet als hMN14, oder 16B, umfasst.

32. Nackter Klasse III-anti-CEA monoklonaler Antikörper oder Fragment davon nach einem der Ansprüche 20-31, zur Verwendung wie in Anspruch 20, wobei das Fragment ausgewählt ist aus der Gruppe bestehend aus F(ab')2, Fab', Fab, Fv und scFv.

33. Nackter Klasse III-anti-CEA monoklonaler Antikörper oder Fragment davon nach einem der Ansprüche 20-31, zur Verwendung wie in Anspruch 20, wobei das therapeutische Agens ausgewählt ist aus der Gruppe bestehend aus humanisiertem, chimärem, menschlichem oder murinem monoklonalen Antikörper oder Fragment davon, ausgewählt aus der Gruppe bestehend aus einem Klasse II-anti-CEA monoklonalen Antikörper, und einem Fragment davon, und entweder gleichzeitig oder sequentiell in einer therapeutisch wirksamen Menge verabreicht wird.

34. Nackter Klasse III-anti-CEA monoklonaler Antikörper oder Fragment davon nach einem der Ansprüche 20-31, zur Verwendung wie in Anspruch 20, wobei das therapeutische Agens ausgewählt ist aus der Gruppe bestehend aus einem cytotoxischen Agens, einem Wirkstoff, einem Radionuklid, einem Immunmodulator, einem photoaktiven therapeutischen Agens, einem Hormon oder einer Kombination davon, optional formuliert in einem pharmazeutisch akzeptablen Vehikel.

35. Nackter Klasse III-anti-CEA monoklonaler Antikörper oder Fragment davon nach einem der Ansprüche 20-32, zur Verwendung wie in Anspruch 20, wobei das therapeutische Agens ein nackter Antikörper oder ein Immunkonjugat ist, wobei der nackte Antikörper und der Antikörperteil des Immunkonjugates ausgewählt ist aus der Gruppe bestehend aus einem humanisierten, chimären, humanen oder murinen monoklonalen Antikörper oder Fragment davon, der/das reaktiv ist mit EGP-1, EGP-2, MUC-1, MUC-2, MUC-3, MUC-4, PAM-4, KC4, TAG-72, EGFR, HER2/neu, BrE3, Le-Y, A3, A33, Ep-CAM, AFP, Tn, Thomson-Friedenreich-Antigenen, VEGF, Ga 733 und einer Kombination davon, und dem Lebewesen entweder gleichzeitig oder sequentiell in einer therapeutisch wirksamen Menge verabreicht wird.

36. Nackter Klasse III-anti-CEA monoklonaler Antikörper oder Fragment davon nach einem der Ansprüche 20-31, zur Verwendung wie in Anspruch 20, wobei das therapeutische Agens nicht DTIC ist.

37. Nackter Klasse III-anti-CEA monoklonaler Antikörper oder Fragment davon nach Anspruch 34, zur Verwendung wie in Anspruch 20, wobei das cytotoxische Agens ein Wirkstoff oder ein Toxin ist.

38. Nackter Klasse III-anti-CEA monoklonaler Antikörper oder Fragment davon nach Anspruch 37, zur Verwendung wie in Anspruch 20, wobei der Wirkstoff die pharmazeutische Eigenschaft besitzt, die ausgewählt ist aus der Gruppe bestehend aus antimitotischen, alkylierenden, antimetabolischen, antiangiogenen, apoptotischen, alkaloiden, COX-2- und antibiotischen Agenzien und Kombinationen davon.

39. Nackter Klasse III-anti-CEA monoklonaler Antikörper oder Fragment davon nach Anspruch 37, zur Verwendung wie in Anspruch 20, wobei der Wirkstoff ausgewählt ist aus der Gruppe bestehend aus Stickstoffsenfgasen, Ethylenimin-Derivaten, Alkylsulfonaten, Nitrosoharnstoffen, Triazenen, Folsäureanalogen, Anthracyclinen, Taxanen, COX-2 Inhibitoren, Pyrimidin-Analogen, Purin-Analogen, Antimetaboliten, Antibiotika, Enzymen, Epipodophyllotoxinen, Platinkoordinationskomplexen, Vincaalkaloiden, substituierten Harnstoffen, Methylhydrazinderivaten, adrenocorticalen Suppressoren, Antagonisten, Endostatin, Taxolen, Camptothecin, Doxorubicine und ihren Analogen und einer Kombinationen davon.

40. Nackter Klasse III-anti-CEA monoklonaler Antikörper oder Fragment davon nach Anspruch 37, zur Verwendung wie in Anspruch 20, wobei das Toxin ein mikrobielles, Planzen- oder tierisches Toxin ist, das ausgewählt ist aus der Gruppe bestehend aus Ricin, Abrin, Alphatoxin, Saporin, Ribonuclease (RNase), DNase I, Staphylokoken- Enterotoxin-A, pokeweed antivirales Protein, Gelonin, Diphterie-Toxin, Pseudomonas-Exotoxin und Pseudomonas-Endotoxin.

41. Nackter Klasse III-anti-CEA monoklonaler Antikörper oder Fragment davon nach Anspruch 34, zur Verwendung wie in Anspruch 20, wobei der Immunmodulator ausgewählt ist aus der Gruppe bestehend aus einem Cytokin, einem Stammzellwachstumsfaktor, einem Lymphotoxin, einem hämatopoetischen Faktor, einem Kolonie-stimulierenden Faktor (CSF), einem Interferon (IFN), Erythropoetin, Thrombopoetin und einer Kombination davon.

42. Nackter Klasse III-anti-CEA monoklonaler Antikörper oder Fragment davon nach Anspruch 41, zur Verwendung wie in Anspruch 20, wobei das Lymphotoxin Tumornekrosefaktor (TNF), der hämatopoetische Faktor ein Interleukin (IL), der Koloniestimulierende Faktor Granulocyten-Kolonie-stimulierender Faktor (G-CSF) oder Granulozyten-Makrophagen-Kolonie-stimulierender Faktor (GM-CSF), das Interferon Interferon-α, -β, oder -γ und der Stammzellenwachstumsfaktor der "S1-factor" bezeichnete ist.

43. Nackter Klasse III-anti-CEA monoklonaler Antikörper oder Fragment davon nach Anspruch 41, zur Verwendung wie in Anspruch 20, wobei der Immunmodulator IL-1, IL-2, IL-3, IL-6, IL-10, IL-12, IL18, Inferon-γ, TNF- α oder eine Kombination davon umfasst.

44. Nackter Klasse III-anti-CEA monoklonaler Antikörper oder Fragment davon nach Anspruch 34, zur Verwendung wie in Anspruch 20, wobei das Radionuklid eine Energie zwischen 20 und 10 000 keV aufweist.

45. Nackter Klasse III-anti-CEA monoklonaler Antikörper oder Fragment davon nach Anspruch 44, zur Verwendung wie in Anspruch 20, wobei das Radionuklid ausgewählt ist aus der Gruppe bestehend aus ¹²⁵I, ¹³¹I, ⁹⁰Y, ⁸⁸Y, ²²⁵Ac, ¹⁷⁷Lu, ¹⁸⁸Re, ¹⁸⁶Re und Kombinationen davon.

46. Nackter Klasse III-anti-CEA monoklonaler Antikörper oder Fragment davon nach Anspruch 34, zur Verwendung wie in Anspruch 20, wobei das photoaktive therapeutische Agens ein Chromogen oder Farbstoff ist.

47. Nackter Klasse III-anti-CEA monoklonaler Antikörper oder Fragment davon nach Anspruch 38, zur Verwendung wie in Anspruch 20, wobei das alkylierende Agens Dacarbazin ist.

48. Nackter Klasse III-anti-CEA monoklonaler Antikörper oder Fragment davon nach Anspruch 47, zur Verwendung wie in Anspruch 20, wobei der MN-14 Antikörper oder Fragment davon in einer Dosierung von 100 bis 600 Milligramm Protein pro Dosis pro Injektion verabreicht wird.

49. Nackter Klasse III-anti-CEA monoklonaler Antikörper oder Fragment davon nach Anspruch 48, zur Verwendung wie in Anspruch 20, wobei der MN-14 Antikörper oder Fragment davon in einer Dosierung von 300 Milligramm Protein pro Dosis pro Injektion verabreicht wird.

50. Nackter Klasse III-anti-CEA monoklonaler Antikörper oder Fragment davon zur Verwendung bei der Behandlung von medullärem Schilddrüsenkrebs, umfassend Verabreichen an ein Lebewesen, entweder gleichzeitig oder sequentiell, einer therapeutisch wirksamen Menge des Klasse III-anti-CEA monoklonalen Antikörpers oder Fragmentes davon und wenigstens eines therapeutischen Agens, und optional formuliert in einem pharmazeutisch akzeptablen Vehikel.

51. Nackter Klasse III-anti-CEA, monoklonaler Antikörper oder Fragment davon nach Anspruch 50, zur Verwendung wie in Anspruch 50, wobei der Klasse III-anti-CEA MAb oder Fragment davon humanisiert ist, wobei der humanisierte MAb im Wesentlichen die Klasse III-anti-CEA-Bindungsspezifität eines murinen Klasse III-anti-CEA MAb beibehält.

52. Nackter Klasse III-anti-CEA monoklonaler Antikörper oder Fragment davon nach Anspruch 50, zur Verwendung wie in Anspruch 50, wobei der Klasse III-anti-CEA MAb oder Fragment davon ein chimärer MAb ist und wobei der chimäre MAb im Wesentlichen die Klasse III-anti-CEA-Bindungsspezifität von murinem Klasse III-anti-CEA MAb beibehält.

53. Nackter Klasse III-anti-CEA monoklonaler Antikörper oder Fragment davon nach Anspruch 50, zur Verwendung wie in Anspruch 50, wobei der Klasse III-anti-CEA monoklonale Antikörper oder Fragment davon ein MN-14 Antikörper oder Fragment davon ist.

54. Nackter Klasse III-anti-CEA monoklonaler Antikörper oder Fragment davon nach Anspruch 53, zur Verwendung wie in Anspruch 50, wobei der MN-14 monoklonale Antikörper oder Fragment davon die komplementaritätbestimmenden Regionen (CDRs) eines murinen MN-14 monoklonalen Antikörpers umfasst, wobei die CDRs der variablen Region der leichten Kette des MN-14 Antikörpers CDR1 umfassend die Aminosäuresequenz KASQDVGTSVA; CDR2 umfassend die Aminosäuresequenz WTSTRHT; und CDR3 umfassend die Aminosäuresequenz QQYSLYRS umfasst; und die CDRs der variablen Region der schweren Kette des Klasse III-anti-CEA Antikörpers CDR1 umfassend TYWMS; CDR2 umfassend EIHPDSSTINYAPSLKD; und CDR3 umfassend LYFGFPWFAY umfasst.

55. Nackter Klasse III-anti-CEA monoklonaler Antikörper oder Fragment davon nach Anspruch 54, zur Verwendung wie in Anspruch 50, wobei der MN-14 monoklonale Antikörper mit CEA reagiert und nicht reagiert mit normalem kreuzreaktivem Antigen (NCA) und Meconium-Antigen (MA).

56. Nackter Klasse III-anti-CEA monoklonaler Antikörper oder Fragment davon nach Anspruch 55, zur Verwendung wie in Anspruch 50, wobei der MN-14 monoklonale Antikörper oder Fragment davon ein humanisierter MN-14 Antikörper oder Fragment davon ist.

57. Nackter Klasse III-anti-CEA monoklonaler Antikörper oder Fragment davon nach Anspruch 55, zur Verwendung wie in Anspruch 50, wobei der MN-14 monoklonale Antikörper oder Fragment davon ein chimärer MN-14 Antikörper oder Fragment davon ist.

58. Nackter Klasse III-anti-CEA monoklonaler Antikörper oder Fragment davon nach Anspruch 56, zur Verwendung wie in Anspruch 50, wobei die Gerüstregionen (FRs) der variablen Regionen der leichten und der schweren Kette des humanisierten MN-14 Antikörpers oder Fragmentes davon wenigstens eine Aminosäure umfassen, die substituiert ist gegenüber den korrespondierenden FRs eines murinen MN-14 monoklonalen Antikörpers.

59. Nackter Klasse III-anti-CEA monoklonaler Antikörper oder Fragment davon nach Anspruch 58, zur Verwendung wie in Anspruch 50, wobei der humanisierte MN-14 Antikörper oder Fragment davon wenigstens eine Aminosäure von der korrespondierenden FR des murinen MN-14 Antikörpers umfasst, die ausgewählt ist aus der Gruppe bestehend aus Aminosäurerest 24, 28, 30, 48, 49, 74 und 94 der variablen Region der murinen schweren Kette die Figur 14A-C, bevorzugterweise ist die variable Region der schweren Kette die variable Region der humanisierten schweren Kette KLHuVhAIGA von Figur 14A-C oder hMN14 von Figur 15B oder Figur 16B.

60. Nackter Klasse III-anti-CEA monoklonaler Antikörper oder Fragment davon nach Anspruch 58, zur Verwendung wie in Anspruch 50, wobei der humanisierte MN-14 Antikörper oder Fragment davon wenigstens eine Aminosäure von der korrespondierenden FR der variablen Region der leichten Kette des murinen MN-14 umfasst.

61. Nackter Klasse III-anti-CEA monoklonaler Antikörper oder Fragment davon nach Anspruch 58, zur Verwendung wie in Anspruch 50, wobei der humanisierte MN-14 Antikörper oder Fragment davon die variable Region der leichten Kette wie in Figur 13A oder 15A, bezeichnet als hMN14, oder 16A angegeben und die variable Region der schweren Kette wie in Figur 14A-C oder 15B, bezeichnet als hMN14, oder 16B angegeben umfasst.

62. Nackter Klasse III-anti-CEA monoklonaler Antikörper oder Fragment davon nach einem der Ansprüche 50-61, zur Verwendung wie in Anspruch 50, wobei das Fragment ausgewählt ist aus der Gruppe bestehend aus F(ab')2, Fab', Fab, Fv und scFv.

63. Nackter Klasse III-anti-CEA monoklonaler Antikörper oder Fragment davon nach einem der Ansprüche 50-61, zur Verwendung wie in Anspruch 50, wobei das therapeutische Agens ausgewählt ist aus der Gruppe bestehend aus humanisiertem, chimärem, humanem oder murinem monoklonalen Antikörper oder Fragment davon, der/das ausgewählt ist aus der Gruppe bestehend aus einem Klasse II-anti-CEA monoklonalen Antikörper, und einem Fragment davon, und gleichzeitig oder sequentiell in einer therapeutisch wirksamen Menge verabreicht wird.

64. Nackter Klasse III-anti-CEA monoklonaler Antikörper oder Fragment davon nach einem der Ansprüche 50-61, zur Verwendung wie in Anspruch 50, wobei das therapeutische Agens ausgewählt ist aus der Gruppe bestehend aus einem cytotoxischen Agens, einem Wirkstoff, einem Radionuklid, einem Immunmodulator, einem photoaktiven therapeutischen Agens, einem Hormon oder einer Kombination davon, optional formuliert in einem pharmazeutische akzeptablen Vehikel.

65. Nackter Klasse III-anti-CEA monoklonaler Antikörper oder Fragment davon nach einem der Ansprüche 50-62, zur Verwendung wie in Anspruch 50, wobei das therapeutische Agens ein nackter Antikörper oder ein Immunkonjugat ist, wobei der nackte Antikörper oder der Antikörperteil des Immunkonjugates ausgewählt ist aus der Gruppe bestehend aus einem humanisierten, chimären, humanen oder murinen monoklonalen Antikörper oder Fragment davon, der/das reaktiv ist mit EGP-1, EGP-2, MUC-1, MUC-2, MUC-3, MUC-4, PAM-4; KC4; TAG-72, EGFR, HER2/neu, BrE3, Le-Y, A3, A33, Ep-CAM, AFP, Tn, Thomson-Friedenreich-Antigenen, VEGF, Ga 733 und einer Kombination davon, und dem Lebewesen entweder gleichzeitig oder sequentiell in einer therapeutisch wirksamen Menge verabreicht wird.

66. Nackter Klasse III-anti-CEA monoklonaler Antikörper oder Fragment davon nach einem der Ansprüche 50-61, zur Verwendung wie in Anspruch 50, wobei das therapeutische Agens nicht DTIC ist.

67. Nackter Klasse III-anti-CEA monoklonaler Antikörper oder Fragment davon nach Anspruch 64, zur Verwendung wie in Anspruch 50, wobei das cytotoxische Agens ein Wirkstoff oder ein Toxin ist.

68. Nackter Klasse III-anti-CEA monoklonaler Antikörper oder Fragment davon nach Anspruch 64, zur Verwendung wie in Anspruch 50, wobei der Wirkstoff die pharmazeutische Aktivität aufweist, die ausgewählt ist aus der Gruppe bestehend aus antimitotischen, alkylierenden, antimetabolischen, antiangiogenen, apoptotischen, alkaloiden, COX-2- und antibiotischen Agenzien und Kombinationen davon.

69. Nackter Klasse III-anti-CEA monoklonaler Antikörper oder Fragment davon nach Anspruch 67, zur Verwendung wie in Anspruch 50, wobei der Wirkstoff ausgewählt ist aus der Gruppe bestehend aus Stickstoffsenfgasen, Ethylenimin-Derivaten, Alkylsulfonaten, Nitrosoharnstoffen, Triazenen, Folsäureanalogen, Anthracyclinen, Taxanen, COX-2 Inhibitoren, Pyrimidin-Analogen, Purin-Analogen, Antimetaboliten, Antibiotika, Enzymen, Epipodophyllotoxinen, Platinkoordinantionskomplexen, Vincaalkaloiden, substituierten Harnstoffen, Methylhydrazinderivaten, adrenocorticalen Suppressoren, Antagonisten, Endostatin, Taxolen, Camptothecinen, Doxorubicinen und ihren Analogen und Kombinationen davon.

70. Nackter Klasse III-anti-CEA monoklonaler Antikörper oder Fragment davon nach Anspruch 67, zur Verwendung wie in Anspruch 50, wobei das mikrobielle, Pflanzen- oder tierische Toxin ausgewählt ist aus der Gruppe bestehend aus Ricin, Abrin, Alphatoxin, Saporin, Ribonuklease (RNase), DNase I, Staphylokokken-Enterotoxin-A, pokeweed antiviralem Protein, Gelonin, Diphtherie-Toxin, Pseudomonas-Exotoxin und Pseudomonas-Endotoxin.

71. Nackter Klasse III-anti-CEA monoklonaler Antikörper oder Fragment davon nach Anspruch 64, zur Verwendung wie in Anspruch 50, wobei der Immunmodulator ausgewählt ist aus der Gruppe bestehend aus einem Cytokin, einem Stammzellenwachstumsfaktor, einem Lymphotoxin, einem hämatopoetischen Faktor, einem Kolonie-stimmulierenden Faktor (CSF), einem Interferon (IFN), Erythropoetin, Thrombopoetin und einer Kombination davon.

72. Nackter Klasse III-anti-CEA monoklonaler Antikörper oder Fragment davon nach Anspruch 71, zur Verwendung wie in Anspruch 50, wobei das Lymphotoxin Tumornekrosefaktor (TNF), der hämatopoetische Faktor ein Interleukin (IL), der Koloniestimulierende Faktor Granulozyten-Kolonie-stimulierender Faktor (G-CSF) oder Granulozyten-Makrophagen-Kolonie-stimulierender Faktor (GM-CSF), das Interferon Interferon -α, -β, oder -γ und der Stammzellenwachstumsfaktor der als "S1 factor" bezeichnete ist.

73. Nackter Klasse III-anti-CEA monoklonaler Antikörper oder Fragment davon nach Anspruch 64, zur Verwendung wie in Anspruch 50, wobei der Immunmodulator umfasst IL-1, IL-2, IL-3, IL-6, IL-10, IL-12, IL-18, Interferon -γ, TNF -α oder eine Kombination davon.

74. Nackter Klasse III-anti-CEA monoklonaler Antikörper oder Fragment davon nach Anspruch 64, zur Verwendung wie in Anspruch 50, wobei das Radionuklid eine Energie zwischen 20 und 10 000 keV aufweist.

75. Nackter Klasse III-anti-CEA monoklonaler Antikörper oder Fragment davon nach Anspruch 74, zur Verwendung wie in Anspruch 50, wobei das Radionuklid ausgewählt ist aus der Gruppe bestehend aus, ¹²⁵I, ¹³¹I, ⁹⁰Y, ⁸⁸Y, ²²⁵Ac, ¹⁷⁷Lu, ¹⁸⁸Re, ¹⁸⁶Re und Kombinationen davon.

76. Nackter Klasse III-anti-CEA monoklonaler Antikörper oder Fragment davon nach Anspruch 64, zur Verwendung wie in Anspruch 50, wobei das photoaktive therapeutische Agens ein Chromogen oder Farbstoff ist.

77. Nackter Klasse III-anti-CEA monoklonaler Antikörper oder Fragment davon nach Anspruch 68, zur Verwendung wie in Anspruch 50, wobei das alkylierende Agens Dacarbazin ist.

78. Nackter Klasse III-anti-CEA monoklonaler Antikörper oder Fragment davon nach Anspruch 77, zur Verwendung wie in Anspruch 50, wobei der MN-14 Antikörper oder Fragment davon in einer Dosierung von 100 bis 600 Milligramm Protein pro Dosis pro Injektion verabreicht wird.

79. Nackter Klasse III-anti-CEA monoklonaler Antikörper oder Fragment davon nach Anspruch 78, zur Verwendung wie in Anspruch 50, wobei der MN-14 Antikörper oder Fragment davon in einer Dosierung von 300 Milligramm Protein pro Dosis pro Injektion verabreicht wird.

## Revendications

1. Composition comprenant au moins un anticorps monoclonal (AcM) nu anti-CEA de classe III ou un fragment de celui-ci et au moins un agent thérapeutique.

2. Composition selon la revendication 1, dans laquelle ledit AcM anti-CEA de classe III ou un fragment de celui-ci est un AcM chimérique, humanisé ou humain, lequel conserve la spécificité de liaison anti-CEA de classe III d'un AcM anti-CEA de classe III murin.

3. Composition selon la revendication 1, dans laquelle l'agent thérapeutique est une toxine ou un agent chimio-thérapeutique.

4. Composition selon la revendication 3, dans laquelle l'agent chimio-thérapeutique est choisi dans le groupe constitué par les vincristine, doxorubicine, DTIC, cyclophosphamide, CPT-11, oxaliplatine, taxane, gemcitabine, un inhibiteur de la COX-2 et le 5-fluoro-uracile.

5. Composition selon la revendication 1, dans laquelle ledit anticorps monoclonal anti-CEA de classe III ou un fragment de celui-ci est un anticorps MN-14 ou un fragment de celui-ci.

6. Composition selon la revendication 5, dans laquelle ledit anticorps monoclonal MN-14 ou un fragment de celui-ci comprend les régions déterminant la complémentarité (CDR) d'un anticorps monoclonal MN-14 murin, dans lequel les CDR de la région variable de chaîne légère dudit anticorps MN-14 comprennent : un CDR1 comprenant la séquence d'acides aminés de KASQDVGTSVA ; un CDR2 comprenant la séquence d'acides aminés de WTSTRHT ; et un CDR3 comprenant la séquence d'acides aminés de QQYSLYRS ; et les CDR de la région variable de chaîne lourde dudit anticorps anti-CEA de classe III comprennent : un CDR1 comprenant une TYWMS ; un CDR2 comprenant une EIHPDSSTINYAPSLKD ; et un CDR3 comprenant une LYFGFPWFAY.

7. Composition selon la revendication 5, dans laquelle ledit anticorps monoclonal MN-14 réagit avec un CEA et est non réactif avec un antigène normal à réaction croisée (NCA) et un antigène méconial (MA).

8. Composition selon la revendication 7, dans laquelle ledit anticorps monoclonal MN-14 ou un fragment de celui-ci est un anticorps MN-14 humanisé ou un fragment de celui-ci.

9. Composition selon la revendication 7, dans laquelle ledit anticorps monoclonal MN-14 ou un fragment de celui-ci est un anticorps MN-14 chimérique ou un fragment de celui-ci.

10. Composition selon la revendication 8, dans laquelle les régions du cadre (FR) des régions variables de chaînes légère et lourde dudit anticorps MN-14 humanisé ou un fragment de celui-ci comprennent au moins un acide aminé substitué à partir des FR correspondantes d'un anticorps monoclonal MN-14 murin.

11. Composition selon la revendication 10, dans laquelle ledit anticorps MN-14 humanisé ou un fragment de celui-ci comprenant au moins un acide aminé provenant de ladite FR correspondante dudit anticorps MN-14 murin, choisi dans le groupe constitué par les résidus d'acides aminés 24, 28, 30, 48, 49, 74 et 94 de la région variable de chaîne lourde murine de la Figure 14A-C, de préférence la région variable de chaîne lourde est une région variable de chaîne lourde humanisée KLHuVhAIGA de la Figure 14A-C ou hMN14 de la Figure 15B ou Figure 16B.

12. Composition selon la revendication 10, dans laquelle ledit anticorps MN-14 humanisé ou un fragment de celui-ci comprend au moins un acide aminé provenant de ladite FR correspondante de ladite région variable de chaîne légère du MN-14 murin.

13. Composition selon la revendication 8, dans laquelle ledit anticorps MN-14 humanisé ou un fragment de celui-ci comprend la région variable de chaîne légère, telle qu'indiquée à la Figure 13A ou Figure 15A, désignée comme hMN14, ou Figure 16A, et la région variable de chaîne lourde indiquée à la Figure 14A-C, désignée comme KLHuVhAIGA, ou Figure 15B, désignée comme hMN14, ou Figure 16B.

14. Composition selon la revendication 9, dans laquelle ledit anticorps MN-14 chimérique ou un fragment de celui-ci comprend la région variable de chaîne légère, telle qu'indiquée à la Figure 13A, désignée comme Vκ MN-14 murine, et la région variable de chaîne lourde indiquée à la Figure 14A-C désignée comme VH MN-14 murine.

15. Composition selon l'une quelconque des revendications 1 à 14, dans laquelle ledit fragment est choisi dans le groupe constitué par les F(ab')2, Fab', Fab, Fv et Fvsc.

16. Composition selon l'une quelconque des revendications 1 à 14, dans laquelle ledit agent thérapeutique est choisi dans le groupe constitué par un agent cytotoxique, une substance médicamenteuse, un radionucléide, un immuno-modulateur, un agent thérapeutique photoactif, une hormone ou une combinaison de ceux-ci, formulé en option dans un véhicule acceptable d'un point de vue pharmaceutique.

17. Composition selon l'une quelconque des revendications 1 à 14, dans laquelle ledit agent thérapeutique est un anticorps nu ou un immuno-conjugué, dans laquelle ledit anticorps nu ou une partie de l'anticorps dudit immuno-conjugué comprend un anticorps monoclonal humanisé, chimérique, humain ou murin ou un fragment de celui-ci, choisi dans le groupe constitué par un anticorps monoclonal ou un fragment de celui-ci étant réactif avec les EGP-1, EGP-2, MUC-1, MUC-2, MUC-3, MUC-4, PAM-4, KC4, TAG-72, EGFR, HER2/neu, BrE3, Le-Y, A3, A33, Ep-CAM, AFP, Tn, antigènes de Thomson-Friedenreich, VEGF, Ga 733 ou une combinaison de ceux-ci.

18. Composition selon la revendication 17, dans laquelle ledit fragment est choisi dans le groupe constitué par les F(ab')2, Fab', Fab, Fv et Fvsc.

19. Composition selon l'une quelconque des revendications 1 à 14, dans laquelle ledit agent thérapeutique n'est pas un DTIC.

20. Anticorps monoclonal nu anti-CEA de classe III ou fragment de celui-ci destiné à être utilisé dans un procédé pour traiter un carcinome thyroïdien non médullaire exprimant un CEA, comprenant l'administration à un sujet, soit de manière concourante soit séquentiellement, d'une quantité thérapeutiquement efficace de l'anticorps monoclonal anti-CEA de classe III ou un fragment de celui-ci et d'au moins un agent thérapeutique, et étant formulé en option dans un véhicule acceptable d'un point de vue pharmaceutique.

21. Anticorps monoclonal nu anti-CEA de classe III ou fragment de celui-ci, selon la revendication 20, destiné à être utilisé selon la revendication 20, dans lequel ledit AcM anti-CEA de classe III ou un fragment de celui-ci est humanisé, dans lequel ledit AcM humanisé conserve substantiellement la spécificité de liaison anti-CEA de classe III d'un AcM anti-CEA de classe III murin.

22. Anticorps monoclonal nu anti-CEA de classe III ou fragment de celui-ci, selon la revendication 20, destiné à être utilisé selon la revendication 20, dans lequel ledit AcM anti-CEA de classe III ou un fragment de celui-ci est un AcM chimérique, et dans lequel ledit AcM chimérique conserve substantiellement la spécificité de liaison anti-CEA de classe III d'un AcM anti-CEA de classe III murin.

23. Anticorps monoclonal nu anti-CEA de classe III ou fragment de celui-ci, selon la revendication 20, destiné à être utilisé selon la revendication 20, dans lequel ledit anticorps monoclonal anti-CEA de classe III ou un fragment de celui-ci est un anticorps MN-14 ou un fragment de celui-ci.

24. Anticorps monoclonal nu anti-CEA de classe III ou fragment de celui-ci, selon la revendication 20, destiné à être utilisé selon la revendication 20, dans lequel ledit anticorps monoclonal MN-14 ou un fragment de celui-ci comprend les régions déterminant la complémentarité (CDR) d'un anticorps monoclonal MN-14 murin, dans lequel les CDR de la région variable de chaîne légère dudit anticorps MN-14 comprennent : un CDR1 comprenant la séquence d'acides aminés de KASQDVGTSVA ; un CDR2 comprenant la séquence d'acides aminés de WTSTRHT ; et un CDR3 comprenant la séquence d'acides aminés de QQYSLYRS ; et les CDR de la région variable de chaîne lourde dudit anticorps anti-CEA de classe III comprennent : un CDR1 comprenant une TYWMS ; un CDR2 comprenant une EIHPDSSTINYAPSLKD ; et un CDR3 comprenant une LYFGFPWFAY.

25. Anticorps monoclonal nu anti-CEA de classe III ou fragment de celui-ci, selon la revendication 24, destiné à être utilisé selon la revendication 20, dans lequel ledit anticorps monoclonal MN-14 réagit avec un CEA et est non réactif avec un antigène normal à réaction croisée (NCA) et un antigène méconial (MA).

26. Anticorps monoclonal nu anti-CEA de classe III ou fragment de celui-ci, selon la revendication 25, destiné à être utilisé selon la revendication 20, dans lequel ledit anticorps monoclonal MN-14 ou un fragment de celui-ci un anticorps MN-14 humanisé ou un fragment de celui-ci.

27. Anticorps monoclonal nu anti-CEA de classe III ou fragment de celui-ci, selon la revendication 25, destiné à être utilisé selon la revendication 20, dans lequel ledit anticorps monoclonal MN-14 ou un fragment de celui-ci est un anticorps MN-14 chimérique ou un fragment de celui-ci.

28. Anticorps monoclonal nu anti-CEA de classe III ou fragment de celui-ci, selon la revendication 26, destiné à être utilisé selon la revendication 20, dans lequel les régions du cadre (FR) des régions variables de chaînes légère et lourde dudit anticorps MN-14 humanisé ou un fragment de celui-ci comprennent au moins un acide aminé substitué à partir des FR correspondantes d'un anticorps monoclonal MN-14 murin.

29. Anticorps monoclonal nu anti-CEA de classe III ou fragment de celui-ci, selon la revendication 28, destiné à être utilisé selon la revendication 20, dans lequel ledit anticorps MN-14 humanisé ou un fragment de celui-ci comprend au moins un acide aminé provenant de ladite FR correspondante dudit anticorps MN-14 murin, choisi dans le groupe constitué par les résidus d'acides aminés 24, 28, 30, 48, 49, 74 et 94 de la région variable de chaîne lourde murine de la Figure 14A-C, de préférence la région variable de chaîne lourde est une région variable de chaîne lourde humanisée KLHuVhAIGA de la Figure 14A-C ou hMN14 de la Figure 15B ou Figure 16B.

30. Anticorps monoclonal nu anti-CEA de classe III ou fragment de celui-ci, selon la revendication 28, destiné à être utilisé selon la revendication 20, dans lequel ledit anticorps MN-14 humanisé ou un fragment de celui-ci comprend au moins un acide aminé provenant de ladite FR correspondante de ladite région variable de chaîne légère du MN-14 murin.

31. Anticorps monoclonal nu anti-CEA de classe III ou fragment de celui-ci, selon la revendication 28, destiné à être utilisé selon la revendication 20, dans lequel ledit anticorps MN-14 humanisé ou un fragment de celui-ci comprend la région variable de chaîne légère, telle qu'indiquée à la Figure 13A ou la 15A, désignée comme hMN14, ou la 16A, et la région variable de chaîne lourde indiquée à la Figure 14A-C, désignée comme KLHuVhAIGA, ou la 15B, désignée comme hMN14, ou la 16B.

32. Anticorps monoclonal nu anti-CEA de classe III ou fragment de celui-ci, selon l'une quelconque des revendications 20 à 31, destiné à être utilisé selon la revendication 20, dans lequel ledit fragment est choisi dans le groupe constitué par les F(ab')2, Fab', Fab, Fv et Fvsc.

33. Anticorps monoclonal nu anti-CEA de classe III ou fragment de celui-ci, selon l'une quelconque des revendications 20 à 31, destiné à être utilisé selon la revendication 20, dans lequel ledit agent thérapeutique est choisi dans le groupe constitué par un anticorps monoclonal humanisé, chimérique, humain ou murin ou bien un fragment de celui-ci choisi dans le groupe constitué par un anticorps monoclonal anti-CEA de classe II et un fragment de celui-ci, et est administré soit de manière concourante soit séquentiellement en une quantité thérapeutiquement efficace.

34. Anticorps monoclonal nu anti-CEA de classe III ou fragment de celui-ci, selon l'une quelconque des revendications 20 à 31, destiné à être utilisé selon la revendication 20, dans lequel ledit agent thérapeutique est choisi dans le groupe constitué par un agent cytotoxique, une substance médicamenteuse, un radionucléide, un immuno-modulateur, un agent thérapeutique photoactif, une hormone ou une combinaison de ceux-ci, formulé en option dans un véhicule acceptable d'un point de vue pharmaceutique.

35. Anticorps monoclonal nu anti-CEA de classe III ou fragment de celui-ci, selon l'une quelconque des revendications 20 à 32, destiné à être utilisé selon la revendication 20, dans lequel ledit agent thérapeutique est un anticorps nu ou un immuno-conjugué, dans lequel l'anticorps nu et la fraction de l'anticorps de l'immuno-conjugué sont choisis dans le groupe constitué par un anticorps monoclonal humanisé, chimérique, humain ou murin ou un fragment de celui-ci étant réactif avec les EGP-1, EGP-2, MUC-1, MUC-2, MUC-3, MUC-4, PAM-4, KC4, TAG-72, EGFR, HER2/neu, BrE3, Le-Y, A3, A33, Ep-CAM, AFP, Tn, antigènes de Thomson-Friedenreich, VEGF, Ga 733 et une combinaison de ceux-ci, et est administré audit sujet, soit de manière concourante soit séquentiellement, en une quantité thérapeutiquement efficace.

36. Anticorps monoclonal nu anti-CEA de classe III ou fragment de celui-ci, selon l'une quelconque des revendications 20 à 31, destiné à être utilisé selon la revendication 20, dans lequel ledit agent thérapeutique n'est pas un DTIC.

37. Anticorps monoclonal nu anti-CEA de classe III ou fragment de celui-ci, selon la revendication 34, destiné à être utilisé selon la revendication 20, dans lequel ledit agent cytotoxique est une substance médicamenteuse ou une toxine.

38. Anticorps monoclonal nu anti-CEA de classe III ou fragment de celui-ci, selon la revendication 37, destiné à être utilisé selon la revendication 20, dans lequel ladite substance médicamenteuse possède la propriété pharmaceutique choisie dans le groupe constitué par les agents antimitotiques, alkylants, anti-métabolites, anti-angiogènes, apoptotiques, alcaloïdes, de COX-2 et antibiotiques ainsi que des combinaisons de ceux-ci.

39. Anticorps monoclonal nu anti-CEA de classe III ou fragment de celui-ci, selon la revendication 37, destiné à être utilisé selon la revendication 20, dans lequel ladite substance médicamenteuse est choisie dans le groupe constitué par des : moutardes azotées, dérivés de l'éthylèneimine, sulfonates d'alkyle, nitroso-urées, triazènes, analogues de l'acide folique, anthracyclines, taxanes, inhibiteurs de la COX-2, analogues d'une pyrimidine, analogues d'une purine, antimétabolites, antibiotiques, enzymes, épipodophyllotoxines, complexes de coordination du platine, alcaloïdes de la pervenche, urées substituées, dérivés de la méthyl-hydrazine, suppresseurs cortico-surrénaliens, antagonistes, endostatines, taxols, camptothécines, doxorubicines et leurs analogues, ainsi qu'une combinaison de ceux-ci.

40. Anticorps monoclonal nu anti-CEA de classe III ou fragment de celui-ci, selon la revendication 37, destiné à être utilisé selon la revendication 20, dans lequel ladite toxine est une toxine microbienne, végétale ou animale, choisie dans le groupe constitué par la ricine, l'abrine, l'alpha toxine, la saporine, une ribonucléase (RNAse), la DNAse I, l'entérotoxine A staphylococcique, une protéine antivirale de la phytolaque, la gélonine, la toxine de la diphtérie, une exotoxine de Pseudomonas et une endotoxine de Pseudomonas.

41. Anticorps monoclonal nu anti-CEA de classe III ou fragment de celui-ci, selon la revendication 34, destiné à être utilisé selon la revendication 20, dans lequel ledit immuno-modulateur est choisi dans le groupe constitué par une cytokine, un facteur de croissance des cellules souches, une lymphotoxine, un facteur hématopoïétique, un facteur de stimulation des colonies (CSF), un interféron (IFN), l'érythropoïétine, la thrombopoïétine et une combinaison de ceux-ci.

42. Anticorps monoclonal nu anti-CEA de classe III ou fragment de celui-ci, selon la revendication 41, destiné à être utilisé selon la revendication 20, dans lequel ladite lymphotoxine est le facteur de nécrose tumoral (TNF), ledit facteur hématopoïétique est une interleukine (IL), ledit facteur de stimulation des colonies est le facteur de stimulation des colonies de granulocytes (G-CSF) ou le facteur de stimulation des colonies de granulocytes-macrophages (GM-CSF), ledit interféron est l'interféron α, le β ou le γ et ledit facteur de croissance des cellules souches est désigné comme le "facteur S1".

43. Anticorps monoclonal nu anti-CEA de classe III ou fragment de celui-ci, selon la revendication 41, destiné à être utilisé selon la revendication 20, dans lequel ledit immuno-modulateur comprend les IL-1, IL-2, IL-3, IL-6, IL-10, IL-12, IL-18, l'interféron γ, le TNF-α ou une combinaison de ceux-ci.

44. Anticorps monoclonal nu anti-CEA de classe III ou fragment de celui-ci, selon la revendication 34, destiné à être utilisé selon la revendication 20, dans lequel ledit radionucléide a une énergie comprise entre 20 et 10 000 keV.

45. Anticorps monoclonal nu anti-CEA de classe III ou fragment de celui-ci, selon la revendication 44, destiné à être utilisé selon la revendication 20, dans lequel ledit radionucléide est choisi dans le groupe constitué par les ¹²⁵I, ¹³¹I, ⁹⁰Y, ⁸⁸Y, ²²⁵Ac, ¹⁷⁷Lu, ¹⁸⁸Re, ¹⁸⁶Re et des combinaisons de ceux-ci.

46. Anticorps monoclonal nu anti-CEA de classe III ou fragment de celui-ci, selon la revendication 34, destiné à être utilisé selon la revendication 20, dans lequel ledit agent thérapeutique photoactif est un chromogène ou un colorant.

47. Anticorps monoclonal nu anti-CEA de classe III ou fragment de celui-ci, selon la revendication 38, destiné à être utilisé selon la revendication 20, dans lequel ledit agent alkylant est la dacarbazine.

48. Anticorps monoclonal nu anti-CEA de classe III ou fragment de celui-ci, selon la revendication 47, destiné à être utilisé selon la revendication 20, dans lequel ledit anticorps MN-14 ou un fragment de celui-ci est administré avec un dosage de 100 à 600 milligrammes de protéine par dose par injection.

49. Anticorps monoclonal nu anti-CEA de classe III ou fragment de celui-ci, selon la revendication 48, destiné à être utilisé selon la revendication 20, dans lequel ledit anticorps MN-14 ou un fragment de celui-ci est administré avec un dosage de 300 milligrammes de protéine par dose par injection.

50. Anticorps monoclonal nu anti-CEA de classe III ou fragment de celui-ci destiné à être utilisé dans le traitement d'un carcinome thyroïdien médullaire, comprenant l'administration à un sujet, soit de manière concourante soit séquentiellement, d'une quantité thérapeutiquement efficace de l'anticorps monoclonal anti-CEA de classe III ou un fragment de celui-ci et d'au moins un agent thérapeutique, et étant formulé en option dans un véhicule acceptable d'un point de vue pharmaceutique.

51. Anticorps monoclonal nu anti-CEA de classe III ou fragment de celui-ci, selon la revendication 50, destiné à être utilisé selon la revendication 50, dans lequel ledit AcM anti-CEA de classe III ou un fragment de celui-ci est humanisé, dans lequel ledit AcM humanisé conserve substantiellement la spécificité de liaison anti-CEA de classe III d'un AcM anti-CEA de classe III murin.

52. Anticorps monoclonal nu anti-CEA de classe III ou fragment de celui-ci, selon la revendication 50, destiné à être utilisé selon la revendication 50, dans laquel ledit AcM anti-CEA de classe III ou un fragment de celui-ci est un AcM chimérique, et dans lequel ledit AcM chimérique conserve substantiellement la spécificité de liaison anti-CEA de classe III d'un AcM anti-CEA de classe III murin.

53. Anticorps monoclonal nu anti-CEA de classe III ou fragment de celui-ci, selon la revendication 50, destiné à être utilisé selon la revendication 50, dans lequel ledit anticorps monoclonal anti-CEA de classe III ou un fragment de celui-ci est un anticorps MN-14 ou un fragment de celui-ci.

54. Anticorps monoclonal nu anti-CEA de classe III ou fragment de celui-ci, selon la revendication 53, destiné à être utilisé selon la revendication 50, dans lequel ledit anticorps monoclonal MN-14 ou un fragment de celui-ci comprend les régions déterminant la complémentarité (CDR) d'un anticorps monoclonal MN-14 murin, dans lequel les CDR de la région variable de chaîne légère dudit anticorps MN-14 comprennent : un CDR1. comprenant la séquence d'acides aminés de KASQDVGTSVA ; un CDR2 comprenant la séquence d'acides aminés de WTSTRHT ; et un CDR3 comprenant la séquence d'acides aminés de QQYSLYRS ; et les CDR de la région variable de chaîne lourde dudit anticorps anti-CEA de classe III comprennent : un DR1 comprenant une TYWMS ; un CDR2 comprenant une EIHPDSSTINYAPSLKD ; et un CDR3 comprenant une LYFGFPWFAY.

55. Anticorps monoclonal nu anti-CEA de classe III ou fragment de celui-ci, selon la revendication 54, destiné à être utilisé selon la revendication 50, dans lequel ledit anticorps monoclonal MN-14 réagit avec un CEA et est non réactif avec un antigène normal à réaction croisée (NCA) et un antigène méconial (MA).

56. Anticorps monoclonal nu anti-CEA de classe III ou fragment de celui-ci, selon la revendication 55, destiné à être utilisé selon la revendication 50, dans lequel ledit anticorps monoclonal MN-14 ou un fragment de celui-ci est un anticorps MN-14 humanisé ou un fragment de celui-ci.

57. Anticorps monoclonal nu anti-CEA de classe III ou fragment de celui-ci, selon la revendication 55, destiné à être utilisé selon la revendication 50, dans lequel ledit anticorps monoclonal MN-14 ou un fragment de celui-ci est un anticorps MN-14 chimérique ou un fragment de celui-ci.

58. Anticorps monoclonal nu anti-CEA de classe III ou fragment de celui-ci, selon la revendication 56, destiné à être utilisé selon la revendication 50, dans lequel les régions du cadre (FR) des régions variables de chaînes légère et lourde dudit anticorps MN-14 humanisé ou un fragment de celui-ci comprennent au moins un acide aminé substitué à partir des FR correspondantes d'un anticorps monoclonal MN-14 murin.

59. Anticorps monoclonal nu anti-CEA de classe III ou fragment de celui-ci, selon la revendication 58, destiné à être utilisé selon la revendication 50, dans lequel ledit anticorps MN-14 humanisé ou un fragment de celui-ci comprend au moins un acide aminé provenant de ladite FR correspondante dudit anticorps MN-14 murin, choisi dans le groupe constitué par les résidus d'acides aminés 24, 28, 30, 48, 49, 74 et 94 de la région variable de chaîne lourde murine de la Figure 14A-C, de préférence la région variable de chaîne lourde est une région variable de chaîne lourde humanisée KLHuVhAIGA de la Figure 14A-C ou hMN14 de la Figure 15B ou Figure 16B.

60. Anticorps monoclonal nu anti-CEA de classe III ou fragment de celui-ci, selon la revendication 58, destiné à être utilisé selon la revendication 50, dans lequel ledit anticorps MN-14 humanisé ou un fragment de celui-ci comprend au moins un acide aminé provenant de ladite FR correspondante de ladite région variable de chaîne légère du MN-14 murin.

61. Anticorps monoclonal nu anti-CEA de classe III ou fragment de celui-ci, selon la revendication 58, destiné à être utilisé selon la revendication 50, dans lequel ledit anticorps MN-14 humanisé ou un fragment de celui-ci comprend la région variable de chaîne légère, telle qu'indiquée à la Figure 13A ou la 15A désignée comme hMN14, ou la 16A, et la région variable de chaîne lourde indiquée à la Figure 14A-C ou la 15B, désignée comme hMN14, ou la 16B.

62. Anticorps monoclonal nu anti-CEA de classe III ou fragment de celui-ci, selon l'une quelconque des revendications 50 à 61, destiné à être utilisé selon la revendication 50, dans lequel ledit fragment est choisi dans le groupe constitué par les F(ab')2, Fab', Fab, Fv et Fvsc.

63. Anticorps monoclonal nu anti-CEA de classe III ou fragment de celui-ci, selon l'une quelconque des revendications 50 à 61, destiné à être utilisé selon la revendication 50, dans lequel ledit agent thérapeutique est choisi dans le groupe constitué par un anticorps monoclonal humanisé, chimérique, humain ou murin ou bien un fragment de celui-ci, choisi dans le groupe constitué par un anticorps monoclonal anti-CEA de classe II et un fragment de celui-ci, et est administré soit de manière concourante soit séquentiellement en une quantité thérapeutiquement efficace.

64. Anticorps monoclonal nu anti-CEA de classe III ou fragment de celui-ci, selon l'une quelconque des revendications 50 à 61, destiné à être utilisé selon la revendication 50, dans lequel ledit agent thérapeutique est choisi dans le groupe constitué par un agent cytotoxique, une substance médicamenteuse, un radionucléide, un immuno-modulateur, un agent thérapeutique photoactif, une hormone ou une combinaison de ceux-ci, formulé en option dans un véhicule acceptable d'un point de vue pharmaceutique.

65. Anticorps monoclonal nu anti-CEA de classe III ou fragment de celui-ci, selon l'une quelconque des revendications 50 à 62, destiné à être utilisé selon la revendication 50, dans lequel ledit agent thérapeutique est un anticorps nu ou un immuno-conjugué, dans lequel l'anticorps nu et la fraction de l'anticorps de l'immuno-conjugué sont choisis dans le groupe constitué par un anticorps monoclonal humanisé, chimérique, humain ou murin ou un fragment de celui-ci étant réactif avec les EGP-1, EGP-2, MUC-1, MUC-2, MUC-3, MUC-4, PAM-4, KC4, TAG-72, EGFR, HER2/neu, BrE3, Le-Y, A3, A33, Ep-CAM, AFP, Tn, antigènes de Thomson-Friedenreich, VEGF, Ga 733 et une combinaison de ceux-ci, et est administré audit sujet, soit de manière concourante soit séquentiellement, en une quantité thérapeutiquement efficace.

66. Anticorps monoclonal nu anti-CEA de classe III ou fragment de celui-ci, selon l'une quelconque des revendications 50 à 61, destiné à être utilisé selon la revendication 50, dans lequel ledit agent thérapeutique n'est pas un DTIC.

67. Anticorps monoclonal nu anti-CEA de classe III ou fragment de celui-ci, selon la revendication 64, destiné à être utilisé selon la revendication 50, dans lequel ledit agent cytotoxique est une substance médicamenteuse ou une toxine.

68. Anticorps monoclonal nu anti-CEA de classe III ou fragment de celui-ci, selon la revendication 64, destiné à être utilisé selon la revendication 50, dans lequel ladite substance médicamenteuse possède la propriété pharmaceutique choisie dans le groupe constitué par les agents antimitotiques, alkylants, anti-métabolites, anti-angiogènes, apoptotiques, alcaloïdes, de COX-2 et antibiotiques ainsi que des combinaisons de ceux-ci.

69. Anticorps monoclonal nu anti-CEA de classe III ou fragment de celui-ci, selon la revendication 67, destiné à être utilisé selon la revendication 50, dans lequel ladite substance médicamenteuse est choisie dans le groupe constitué par des : moutardes azotées, dérivés de l'éthylèneimine, sulfonates d'alkyle, nitroso-urées, triazènes, analogues de l'acide folique, anthracyclines, taxanes, inhibiteurs de la COX-2, analogues d'une pyrimidine, analogues d'une purine, antimétabolites, antibiotiques, enzymes, épipodophyllotoxines, complexes de coordination du platine, alcaloïdes de la pervenche, urées substituées, dérivés de la méthyl-hydrazine, suppresseurs cortico-surrénaliens, antagonistes, endostatines, taxols, camptothécines, doxorubicines et leurs analogues, ainsi qu'une combinaison de ceux-ci.

70. Anticorps monoclonal nu anti-CEA de classe III ou fragment de celui-ci, selon la revendication 67, destiné à être utilisé selon la revendication 50, dans lequel ladite toxine est une toxine microbienne, végétale ou animale, choisie dans le groupe constitué par la ricine, l'abrine, l'alpha toxine, la saporine, une ribonucléase (RNAse), la DNAse I, l'entérotoxine A staphylococcique, une protéine antivirale de la phytolaque, la gélonine, la toxine de la diphtérie, une exotoxine de Pseudomonas et une endotoxine de Pseudomonas.

71. Anticorps monoclonal nu anti-CEA de classe III ou fragment de celui-ci, selon la revendication 64, destiné à être utilisé selon la revendication 50, dans lequel ledit immuno-modulateur est choisi dans le groupe constitué par une cytokine, un facteur de croissance des cellules souches, une lymphotoxine, un facteur hématopoïétique, un facteur de stimulation des colonies (CSF), un interféron (IFN), l'érythropoïétine, la thrombopoïétine et une combinaison de ceux-ci.

72. Anticorps monoclonal nu anti-CEA de classe III ou fragment de celui-ci, selon la revendication 71, destiné à être utilisé selon la revendication 50, dans lequel ladite lymphotoxine est le facteur de nécrose tumoral (TNF), ledit facteur hématopoïétique est une interleukine (IL), ledit facteur de stimulation des colonies est le facteur de stimulation des colonies de granulocytes (G-CSF) ou le facteur de stimulation des colonies de granulocytes-macrophages (GM-CSF), ledit interféron est l'interféron α, le β ou le γ et ledit facteur de croissance des cellules souches est désigné comme le "facteur S1".

73. Anticorps monoclonal nu anti-CEA de classe III ou fragment de celui-ci, selon la revendication 64, destiné à être utilisé selon la revendication 50, dans lequel ledit immuno-modulateur comprend les IL-1, IL-2, IL-3, IL-6, IL-10, IL-12, IL-18, l'interféron γ, le TNF-α ou une combinaison de ceux-ci.

74. Anticorps monoclonal nu anti-CEA de classe III ou fragment de celui-ci, selon la revendication 64, destiné à être utilisé selon la revendication 50, dans lequel ledit radionucléide a une énergie comprise entre 20 et 10 000 keV.

75. Anticorps monoclonal nu anti-CEA de classe III ou fragment de celui-ci, selon la revendication 74, destiné à être utilisé selon la revendication 50, dans lequel ledit radionucléide est choisi dans le groupe constitué par les ¹²⁵I, ¹³¹I, ⁹⁰Y, ⁸⁸Y, ²²⁵Ac, ¹⁷⁷Lu, ¹⁸⁸Re_{,} ¹⁸⁶Re et des combinaisons de ceux-ci.

76. Anticorps monoclonal nu anti-CEA de classe III ou fragment de celui-ci, selon la revendication 64, destiné à être utilisé selon la revendication 50, dans lequel ledit agent thérapeutique photoactif est un chromogène ou un colorant.

77. Anticorps monoclonal nu anti-CEA de classe III ou fragment de celui-ci, selon la revendication 68, destiné à être utilisé selon la revendication 50, dans lequel ledit agent alkylant est la dacarbazine.

78. Anticorps monoclonal nu anti-CEA de classe III ou fragment de celui-ci, selon la revendication 77, destiné à être utilisé selon la revendication 50, dans lequel ledit anticorps MN-14 ou un fragment de celui-ci est administré avec un dosage de 100 à 600 milligrammes de protéine par dose par injection.

79. Anticorps monoclonal nu anti-CEA de classe III ou fragment de celui-ci, selon la revendication 78, destiné à être utilisé selon la revendication 50, dans lequel ledit anticorps MN-14 ou un fragment de celui-ci est administré avec un dosage de 300 milligrammes de protéine par dose par injection.
